(19) Europäisches Patentamt
European Patent Office
Office européen des brevets

(11) EP 4 467 537 A1

(12) EUROPEAN PATENT APPLICATION
published in accordance with Art. 153(4) EPC

(43) Date of publication:
27.11.2024 Bulletin 2024/48

(21) Application number: 22923433.1

(22) Date of filing: 25.11.2022

(51) International Patent Classification (IPC):
*C07D 403/04* (2006.01) *C07D 403/14* (2006.01)
*C07D 487/04* (2006.01) *C07D 413/14* (2006.01)
*C07D 307/77* (2006.01) *A61K 31/343* (2006.01)
*A61K 31/444* (2006.01) *A61P 35/00* (2006.01)

(52) Cooperative Patent Classification (CPC):
A61K 31/343; A61K 31/444; A61P 35/00;
C07D 307/77; C07D 403/04; C07D 403/14;
C07D 413/14; C07D 487/04

(86) International application number:
PCT/CN2022/134330

(87) International publication number:
WO 2023/142641 (03.08.2023 Gazette 2023/31)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 25.01.2022 CN 202210085981
07.11.2022 CN 202211383830

(71) Applicant: Chengdu Easton Biopharmaceuticals Co., Ltd.
Hi-tech District
Chengdu, Sichuan 611731 (CN)

(72) Inventors:
- LIU, Chunchi
  Chengdu, Sichuan 611731 (CN)
- MA, Zhen
  Chengdu, Sichuan 611731 (CN)
- XIANG, Yongzhe
  Chengdu, Sichuan 611731 (CN)
- LI, Li
  Chengdu, Sichuan 611731 (CN)
- SONG, Zhiquan
  Chengdu, Sichuan 611731 (CN)
- CHEN, Hong
  Chengdu, Sichuan 611731 (CN)
- WANG, Ying
  Chengdu, Sichuan 611731 (CN)

(74) Representative: Petty, Catrin Helen
Venner Shipley LLP
200 Aldersgate
London EC1A 4HD (GB)

# (54) PYRIDINE DERIVATIVE, PREPARATION METHOD THEREFOR AND USE THEREOF

(57) Provided are a compound of formula I, a stereoisomer or a pharmaceutically acceptable salt thereof, a pharmaceutical composition including the compound, a preparation method for the compound and a use thereof in preparation of drugs for treating LSD1-related diseases.

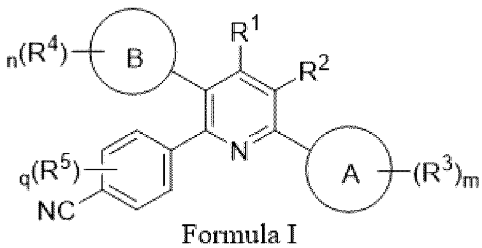

Formula I

EP 4 467 537 A1

## Description

### TECHNICAL FIELD

The present application relates to the field of medicinal chemistry, in particular to a pyridine derivative used as a lysine-specific demethylase 1 (LSD1) inhibitor, or a pharmaceutically acceptable salt thereof, a preparation method therefor and a use thereof.

### BACKGROUND

Histone lysine-specific demethylase 1 (LSD1) is the first reported histone demethylase that, with the assistance of flavin adenine dinucleotide (FAD), can specifically recognize H3K4 and H3K9 substrates and remove their single-methyl or double-methyl modifications. At the same time, the LSD1 can form transcriptional regulatory complexes with multiple factors, and under the recruitment of transcription factors, it can reach a promoter region of a designated gene, thereby regulating gene expression.

Researches have shown that the LSD1 is related to various diseases, is highly expressed in various tumor cells, and is closely related to poor tumor prognosis. Downregulation of LSD1 expression or inhibition of its activity can significantly inhibit tumor cell growth. Therefore, developing an efficient and specific LSD1 inhibitor is of great significance for the treatment of diseases such as tumors.

### SUMMARY OF THE INVENTION

The present application relates to a pyridine derivative as an LSD1 inhibitor, and particularly relates to a pyridine derivative as well as a preparation method therefor and an application thereof in medicines, in particular, a pyridine derivative as shown in formula I below and a use thereof in preparation of drugs for treating LSD1-mediated diseases, more specifically, a use in preparation of drugs suitable for tumors.

One aspect of the present application provides a pyridine derivative with a structure as shown in formula I below, and a stereoisomer or a pharmaceutically acceptable salt thereof:

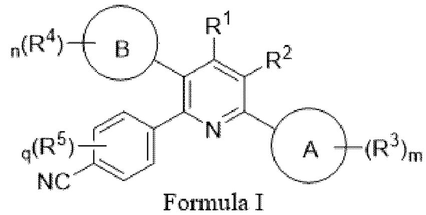

Formula I

wherein,

a ring A represents saturated cycloalkyl, saturated heterocycloalkyl, unsaturated cyclohydrocarbyl or unsaturated heterocyclohydrocarbyl;

a ring B represents monocyclic or dicyclic unsaturated hydrocarbyl, or monocyclic or dicyclic unsaturated heterocyclohydrocarbyl;

$R^1$ and $R^2$ are each independently selected from the group consisting of hydrogen, fluorine, cyano,

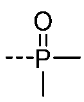 , 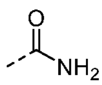 and 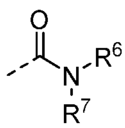 ,

and $R^1$ and $R^2$ are not hydrogen or fluorine at the same time, and when $R^1$ is hydrogen, $R^2$ is not fluorine, and when $R^1$ is fluorine, $R^2$ is not hydrogen; or $R^1$ and $R^2$ together with a carbon atom to which they are connected form a ring structure selected from

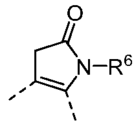

or

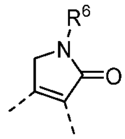

;

$R^3$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and Cs-Cs cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl, $C_1$-$C_6$ alkoxy and $C_1$-$C_6$ alkylacylamino; and preferably, when m is 2 to 5, $R^3$ may be selected from different groups;

$R^4$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl, $C_1$-$C_6$ alkoxy and $C_1$-$C_6$ alkylacylamino; and preferably, when n is 2 to 5, $R^4$ may be selected from different groups;

$R^5$ is selected from the group consisting of halogen, hydroxyl, substituted or unsubstituted amino, $C_1$-$C_6$ alkyl, $C_1$-$C_6$ alkoxy, ($C_3$-$C_8$) cycloalkyl and ($C_3$-$C_8$) cycloalkyloxy, wherein the $C_1$-$C_6$ alkyl, the $C_1$-$C_6$ alkoxy, the $C_3$-$C_8$ cycloalkyl or the$C_3$-$C_8$ cycloalkyloxy is unsubstituted or is substituted with one or more substituents selected from the group consisting of halogen, cyano, hydroxyl, amino, $C_1$-$C_6$ alkyl or $C_1$-$C_6$ alkoxy;

$R^6$ and $R^7$ are each independently selected from the group consisting of hydrogen, $C_1$-$C_6$ alkyl and $C_3$-$C_8$ cycloalkyl;

m is selected from the group consisting of 0, 1, 2, 3, 4 and 5;

n is selected from the group consisting of 0, 1, 2, 3, 4 and 5; and

q is selected from the group consisting of 0, 1, 2, 3 and 4.

In some implementations, the ring A is selected from the group consisting of $C_4$-$C_{13}$ cycloalkyl and 3- to 13-membered heterocycloalkyl, and the heterocycloalkyl contains 1-3 heteroatoms selected from the group consisting of a nitrogen atom, an oxygen atom and a sulfur atom;

preferably, the ring A is selected from the group consisting of $C_4$-$C_8$ monocycloalkyl, $C_7$-$C_{13}$ spirocycloalkyl, 3- to 10-membered monoheterocycloalkyl and 7- to 13- membered spiroheterocycloalkyl, and the heterocycloalkyl contains 1-3 heteroatoms selected from the group consisting of a nitrogen atom and an oxygen atom;

more preferably, the ring A is selected from the group consisting of $C_5$-$C_7$ monocycloalkyl, $C_8$-$C_{12}$ spirocycloalkyl, 3- to 8- membered monoheterocycloalkyl and 8- to 12- membered spiroheterocycloalkyl, and the heterocycloalkyl contains 1-3 heteroatoms selected from the group consisting of a nitrogen atom and an oxygen atom; or, the ring A is selected from the group consisting of $C_5$-$C_7$ monocycloalkyl (e.g., $C_6$ monocycloalkyl), a 3- to 8- membered monoheterocycloalkyl (e.g., 4- to 7- membered monoheterocycloalkyl) and 8- to 12- membered spiroheterocycloalkyl (e.g., 9- to 10-membered spiroheterocycloalkyl), and the heterocycloalkyl contains 1-3 heteroatoms selected from the group consisting of a nitrogen atom and an oxygen atom;

or preferably, the ring A is selected from the group consisting of piperidyl, tetrahydropyridyl, piperazinyl, azacyclobutyl, azacyclopentyl, azacyclohexyl, azacycloheptyl, homopiperazinyl, cyclohexyl, cyclobutyl, cyclopentyl, cycloheptyl, diazaspirononyl, diazaspirodecyl, oxa-azaspirononyl and oxa-azaspirodecyl.

In some implementations, the ring B is selected from the group consisting of $C_5$-$C_{10}$ cycloalkenyl, 5- to 10-membered heterocycloalkenyl, $C_5$-$C_{10}$ aryl and 5- to 10- membered heteroaryl, and the heterocycloalkenyl or the heteroaryl contain 1-3 heteroatoms selected from the group consisting of a nitrogen atom, an oxygen atom and a sulfur atom;

preferably, the ring B is selected from the group consisting of $C_3$-$C_8$ cycloalkenyl and 8- to 10-membered heterocycloalkenyl, and the heterocycloalkenyl contains 1-3 heteroatoms selected from the group consisting of a nitrogen atom, an oxygen atom and a sulfur atom;

or preferably, the ring B is selected from the group consisting of phenyl, pyridyl, dihydropyridyl, pyrazinyl, pyridazinyl, pyrimidinyl, and benzo 5- to 6- membered heterocycloalkenyl containing 1-3 heteroatoms selected from the group consisting of a nitrogen atom and an oxygen atom (e.g., indolyl, isoindolyl, isoindolinyl, dihydroindolyl, indazolyl, dihydroindazolyl, benzoxazolyl, benzoisoxazolyl, quinolyl, isoquinolyl, quinazolinyl, quinoxalinyl, benzodioxolyl, benzofuryl, benzimidazolyl, and benzodihydrooxazinyl).

In some implementations, $R^1$ and $R^2$ are each independently selected from the group consisting of hydrogen,cyano,

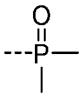 , 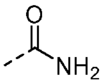 and 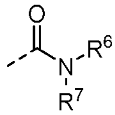 ,

or $R^1$ and $R^2$ together with a carbon atom to which they are connected form a ring structure selected from

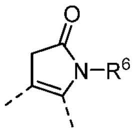 or 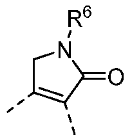 ;

and $R^6$ and $R^7$ are each independently selected from the group consisting of hydrogen, $C_1$-$C_6$ alkyl and $C_3$-$C_6$ cycloalkyl; preferably, R’ is selected from the group consisting of hydrogen, cyano,

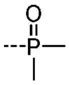 , 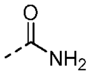

and

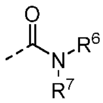

(e.g., hydrogen, cyano or

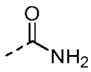 ),

$R^2$ is selected from the group consisting of hydrogen, cyano,

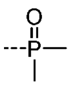 , 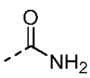 or 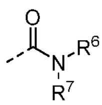

(e.g., hydrogen, cyano,

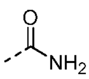 , 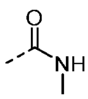 , 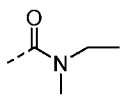 ,

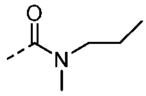, 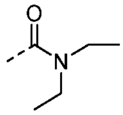, 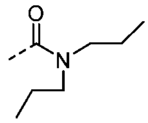, 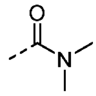, 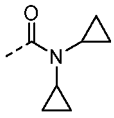 and 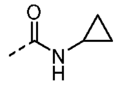),

or $R^1$ and $R^2$ together with a carbon atom to which they are connected form a ring structure selected from

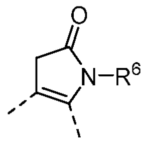

or

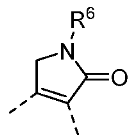 (e.g., 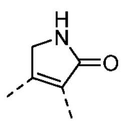);

and $R^6$ and $R^7$ are each independently selected from the group consisting of hydrogen, $C_1$-$C_5$ alkyl (e.g., $C_1$-$C_4$ alkyl, and $C_1$-$C_3$ alkyl) and $C_3$-$C_5$ cycloalkyl (e.g., $C_3$-$C_4$ cycloalkyl and cyclopropyl).

In some implementations, m is 0, 1, 2, 3, 4 or 5, and $R^3$ is selected from the group consisting of halogen (including fluorine, chlorine, bromine or iodine), oxo, hydroxyl, substituted or unsubstituted amino, $C_1$-$C_6$ alkyl and $C_1$-$C_6$ alkoxy, wherein the $C_1$-$C_6$ alkyl or the $C_1$-$C_6$ alkoxy is unsubstituted or is substituted with one or more substituents selected from the group consisting of halogen, cyano, hydroxyl and amino, and preferably, when m is 2 to 5, a plurality of $R^3$ may be selected from different groups;

or in some implementations, m is 0, 1, 2 or 3 (preferably 0, 1 or 2), and $R^3$ is selected from the group consisting of fluorine, chlorine, bromine, oxo, hydroxyl, amino and $C_1$-$C_6$ alkyl (e.g., $C_1$-$C_3$ alkyl), wherein the $C_1$-$C_6$ alkyl (e.g., $C_1$-$C_3$ alkyl) is unsubstituted or is substituted with amino; and preferably, when m is 2, two $R^3$ may be the same or different.

In some implementations, n is selected from the group consisting of 0, 1, 2, 3, 4 and 5, and $R^4$ is selected from the group consisting of halogen (including fluorine, chlorine, bromine or iodine), oxo, hydroxyl, $C_1$-$C_6$ alkyl, $C_1$-$C_6$ alkoxy, $C_3$-$C_6$ cycloalkyl and $C_3$-$C_6$ cycloalkyloxy, wherein the $C_1$-$C_6$ alkyl, the $C_1$-$C_6$ alkoxy, the $C_3$-$C_6$ cycloalkyl or the $C_3$-$C_6$ cycloalkyloxy is unsubstituted or is substituted with one or more substituents selected from the group consisting of halogen, hydroxyl and amino; and preferably, when n is 2 to 5, a plurality of $R^4$ may be selected from different groups;

or in some implementations, n is selected from the group consisting of 0, 1, 2, 3 and 4, and $R^4$ is selected from the group consisting of fluorine, chlorine, bromine, oxo, hydroxyl, $C_1$-$C_6$ alkyl and $C_1$-$C_6$ alkoxy, wherein the $C_1$-$C_6$ alkyl or the $C_1$-$C_6$ alkoxy is unsubstituted or is substituted with one or more substituents selected from halogen or hydroxyl; and preferably, when n is 2 to 4, a plurality of $R^4$ may be selected from different groups.

In some implementations, q is selected from the group consisting of 0, 1 and 2, and $R^5$ is selected from the group consisting of halogen, hydroxyl, amino, $C_1$-$C_6$ alkyl and $C_1$-$C_6$ alkoxy, wherein the $C_1$-$C_6$ alkyl or the $C_1$-$C_6$ alkoxy is unsubstituted or is substituted with one or more substituents selected from the group consisting of halogen and hydroxyl;

or in some implementations, q is 1 or 2, $R^5$ is selected from halogen, and preferably, $R^5$ is selected from the group consisting of fluorine, chlorine and bromine.

Preferably, in the compound of formula I, and the stereoisomer or the pharmaceutically acceptable salt thereof,

the ring A is selected from the group consisting of 5- to 8- membered saturated monocycloalkyl, 4-to 10- membered saturated monocyclic heterocycloalkyl and spiroheterocycloalkyl, wherein the heterocycloalkyl contains 1-3 nitrogen atoms as heteroatoms;

the ring B is selected from the group consisting of 5- to 8- membered unsaturated monocyclohydrocarbyl, 5- to 14-membered unsaturated monocyclic heterocyclohydrocarbyl and dicyclic heterocyclohydrocarbyl, wherein the heterocyclohydrocarbyl contains 0-4 nitrogen atoms and 0-2 oxygen atoms as heteroatoms and contains at least one selected from the group consisting of nitrogen and oxygen;

$R^1$ and $R^2$ are each independently selected from the group consisting of hydrogen, fluorine, cyano,

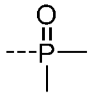 , 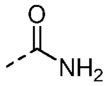 and 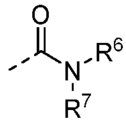 ,

$R^1$ and $R^2$ are not hydrogen or fluorine at the same time, when R’ is hydrogen, $R^2$ is not fluorine, and when $R^1$ is fluorine, $R^2$ is not hydrogen; or $R^1$ and $R^2$ together with a carbon atom to which they are connected form a ring structure selected from

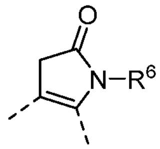

or

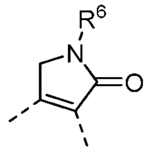

;

$R^3$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and Cs-Cs cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy;

$R^4$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and ($C_3$-$C_8$) cycloalkyloxy, wherein the ($C_1$-$C_6$) alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy;

$R^5$ is selected from the group consisting of halogen, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy;

$R^6$ and $R^7$ are each independently selected from the group consisting of hydrogen, $C_1$-$C_6$ alkyl and $C_3$-$C_8$ cycloalkyl;

m is selected from the group consisting of 0, 1, 2, 3, 4 and 5;

n is selected from the group consisting of 0, 1, 2, 3, 4 and 5; and

q is selected from the group consisting of 0, 1, 2, 3 and 4.

Preferably, in the compound of formula I, and the stereoisomer or the pharmaceutically acceptable salt thereof,

the ring A is selected from the group consisting of 4- to 10- membered saturated monocyclic heterocycloalkyl and spiroheterocycloalkyl, wherein the heterocycloalkyl contains 1-3 nitrogen atoms as heteroatoms, preferably 1-2 nitrogen atoms as the heteroatoms; or, the ring A is selected from the group consisting of piperidyl, piperazinyl, azacyclobutyl, azacyclopentyl, homopiperazinyl, diazaspirononyl, diazaspirodecyl and oxa-azaspirodecyl;

the ring B is selected from the group consisting of 6-membered unsaturated monocyclohydrocarbyl, and 6- to 10-membered unsaturated dicyclic heterocyclohydrocarbyl, wherein the heterocyclohydrocarbyl contains 0-4 nitrogen atoms and 0-2 oxygen atoms as heteroatoms and contains at least one selected from the group consisting of nitrogen and oxygen; preferably, the ring B is selected from the group consisting of 6- to 10- membered aryl and heterocycloaryl, wherein the heterocycloaryl contains 1-2 nitrogen atoms and 0-2 oxygen atoms as heteroatoms and contains at least one selected from the group consisting of nitrogen and oxygen; and more preferably, the ring B is selected from the group consisting of phenyl, pyridyl, indolyl, dihydroindolyl, indazolyl, benzoisoxazolyl, benzoxazolyl, benzodioxolyl and benzodihydrooxazinyl;

$R^1$ and $R^2$ are each independently selected from the group consisting of hydrogen, fluorine, cyano,

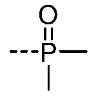, 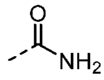 and 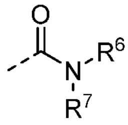,

$R^1$ and $R^2$ are not hydrogen or fluorine at the same time, when $R^1$ is hydrogen, $R^2$ is not fluorine, and when $R^1$ is fluorine, $R^2$ is not hydrogen; or $R^1$ and $R^2$ together with a carbon atom to which they are connected form a ring structure selected from

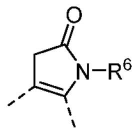 or 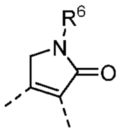;

preferably, $R^1$ and $R^2$ are each independently selected from the group consisting of hydrogen, fluorine, cyano,

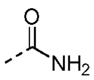, 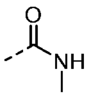, 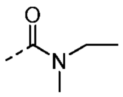, 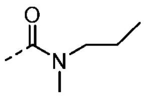, 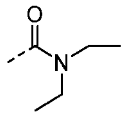, 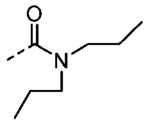, 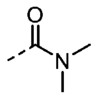, 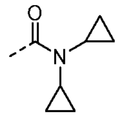

and

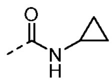

(e.g., $R^1$ and $R^2$ are each independently selected from the group consisting of hydrogen, fluorine, cyano,

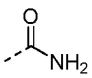, 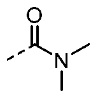 and 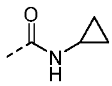),

$R^1$ and $R^2$ are not hydrogen or fluorine at the same time, when $R^1$ is hydrogen, $R^2$ is not fluorine, and when $R^1$ is fluorine, $R^2$ is not hydrogen; or $R^1$ and $R^2$ together with a carbon atom to which they are connected form a ring structure selected from

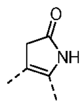

or

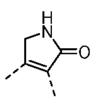;

$R^3$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy; preferably, $R^3$ is selected from the group consisting of halogen, cyano, oxo, hydroxyl, substituted or unsubstituted amino, $C_1$-$C_3$ alkyl, $C_1$-$C_3$ alkoxy, $C_3$-$C_5$ cycloalkyl and $C_3$-$C_5$ cycloalkyloxy, and the $C_1$-$C_3$ alkyl, the $C_1$-$C_3$ alkoxy, the $C_3$-$C_5$ cycloalkyl or the $C_3$-$C_5$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy; and preferably, $R^3$ is selected from the group consisting of fluorine, chlorine, oxo, hydroxyl, amino and $C_1$-$C_3$ alkyl (e.g., methyl), wherein the $C_1$-$C_3$ alkyl (e.g.,

methyl) is unsubstituted or is substituted with amino;

$R^4$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy; preferably, the $C_1$-$C_6$ alkyl, the $C_1$-$C_6$ alkoxy, the $C_3$-$C_8$ cycloalkyl or the $C_3$-$C_8$ cycloalkyloxy is $C_1$-$C_3$ alkyl, $C_1$-$C_3$ alkoxy, $C_3$-$C_5$ cycloalkyl or $C_3$-$C_5$ cycloalkyloxy, and is unsubstituted or is substituted with one or more substituents selected from the group consisting of halogen, cyano, hydroxyl, amino, $C_1$-$C_6$ alkyl or $C_1$-$C_6$ alkoxy; and preferably, $R^4$ is selected from the group consisting of fluorine, chlorine, oxo, methyl, ethyl, propyl, methoxy, ethoxy, propoxy,

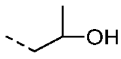,

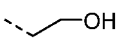, 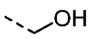, 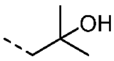, 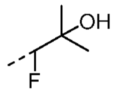 and 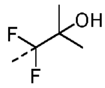

(e.g., $R^4$ is selected from the group consisting of fluorine, chlorine, oxo, methyl, methoxy,

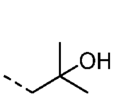 and 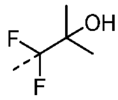);

$R^5$ is selected from the group consisting of halogen, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy; and preferably, $R^5$ is selected from the group consisting of fluorine, chlorine, bromine, methyl, methoxy, trifluoromethyl and trifluoromethoxy, for example, $R^5$ is selected from the group consisting of fluorine, chlorine and bromine;

$R^6$ and $R^7$ are each independently selected from the group consisting of hydrogen, $C_1$-$C_6$ alkyl and $C_3$-$C_8$ cycloalkyl, and preferably, $R^6$ and $R^7$ are each independently selected from the group consisting of hydrogen, $C_1$-$C_6$ alkyl and $C_3$-$C_6$ cycloalkyl;

m is selected from the group consisting of 0, 1, 2, 3, 4 and 5; and preferably, m is selected from the group consisting of 0, 1, 2 and 3;

n is selected from the group consisting of 0, 1, 2, 3, 4 and 5; and preferably, n is selected from the group consisting of 0, 1, 2, 3 and 4; and

q is selected from the group consisting of 0, 1, 2, 3 and 4; and preferably, q is 1 or 2.

In some implementations, in the compound of formula I, and the stereoisomer or the pharmaceutically acceptable salt thereof,

the ring A is selected from the group consisting of piperidyl, piperazinyl, azacyclobutyl, azacyclopentyl, homopiperazinyl, diazaspirononyl, diazaspirodecyl and oxa-azaspirodecyl;

the ring B is selected from the group consisting of phenyl, pyridyl, indolyl, dihydroindolyl, indazolyl, benzoisoxazolyl, benzoxazolyl, benzodioxolyl and benzodihydrooxazinyl;

$R^1$ and $R^2$ are each independently selected from the group consisting of hydrogen, cyano,

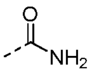,

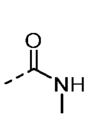, 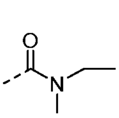, 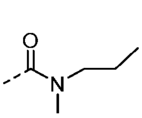, 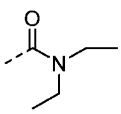, 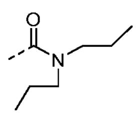, 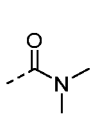, 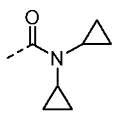 and 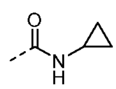;

or $R^1$ and $R^2$ together with a carbon atom to which they are connected form a ring structure selected from

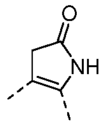 or 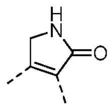 ;

$R^3$ is selected from the group consisting of fluorine, chlorine, bromine, oxo, hydroxyl, amino and $C_1$-$C_3$ alkyl, wherein the $C_1$-$C_3$ alkyl is unsubstituted or is substituted with amino;
$R^4$ is selected from the group consisting of fluorine, chlorine, bromine, oxo, hydroxyl, $C_1$-$C_6$ alkyl and $C_1$-$C_6$ alkoxy, wherein the $C_1$-$C_6$ alkyl is unsubstituted or is substituted with one or more substituents selected from the group consisting of halogen and hydroxyl;
$R^5$ is selected from the group consisting of fluorine, chlorine and bromine;
m is selected from the group consisting of 0, 1, 2 and 3;
n is selected from the group consisting of 0, 1, 2, 3 and 4; and
q is 1 or 2.

Preferably, the compound of formula I, and the stereoisomer or the pharmaceutically acceptable salt thereof further have a structure shown in formula II,

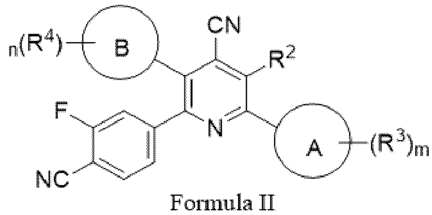

Formula II

wherein,

the ring A is selected from the group consisting of cyclohexyl, piperidyl, piperazinyl, azacycloheptyl and homopiperazinyl; and preferably, the piperidinyl, the piperazinyl, the azacycloheptyl or the homopiperazinyl is connected to a pyridine ring through an N atom;
the ring B is selected from the group consisting of phenyl, pyridyl, indolyl, dihydroindolyl, and indazolyl;
$R^2$ is selected from the group consisting of hydrogen and fluorine;
$R^3$ is selected from the group consisting of fluorine, chlorine, oxo, hydroxyl and amino;
$R^4$ is selected from the group consisting of fluorine, chlorine, oxo, methyl, methoxy,

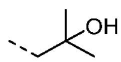

and

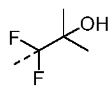 ;

m is selected from the group consisting of 0, 1, 2 and 3; and
n is selected from the group consisting of 0, 1, 2, 3 and 4.

Or, in some implementations, in the compound of formula II, and the stereoisomer or the pharmaceutically acceptable salt thereof,

the ring A is selected from the group consisting of cyclohexyl, piperidyl, piperazinyl, azacyclobutyl, azacycloheptyl, homopiperazinyl, diazaspirononyl and diazaspriodecyl; and preferably, the piperidinyl, the piperazinyl, the azacyclobutyl, the azacycloheptyl, the homopiperazinyl, the diazaspirononyl or the diazaspriodecyl is connected to a pyridine ring through an N atom;
the ring B is selected from the group consisting of phenyl, pyridyl, indolyl, dihydroindolyl, indazolyl, benzoisoxazolyl, benzoxazolyl and benzodioxolyl;
$R^2$ is selected from the group consisting of hydrogen, cyano,

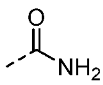 and 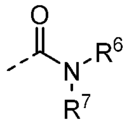 ,

and $R^6$ and $R^7$ are each independently selected from the group consisting of hydrogen, $C_1$-$C_5$ alkyl and $C_3$-$C_5$ cycloalkyl;

$R^3$ is selected from the group consisting of fluorine, chlorine, bromine, oxo, hydroxyl, amino and $C_1$-$C_6$ alkyl, wherein the $C_1$-$C_6$ alkyl is unsubstituted or is substituted with amino;

$R^4$ is selected from the group consisting of fluorine, chlorine, bromine, oxo, $C_1$-$C_6$ alkyl and $C_1$-$C_6$ alkoxy, wherein the $C_1$-$C_6$ alkyl is unsubstituted or is substituted with one or more substituents selected from the group consisting of halogen and hydroxyl;

m is selected from the group consisting of 0, 1, 2 and 3; and

n is selected from the group consisting of 0, 1, 2, 3 and 4.

More preferably, a heterocyclic derivative with a structure shown in formula I, and a stereoisomer or a pharmaceutically acceptable salt thereof are selected from the group consisting of following compounds, and stereoisomers or pharmaceutically acceptable salts thereof:

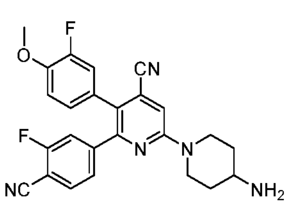 , 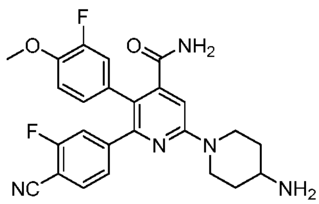 , 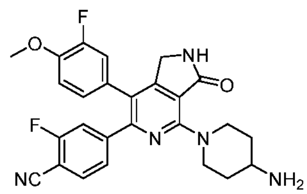 ,

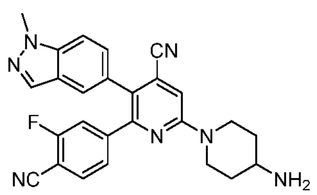 , 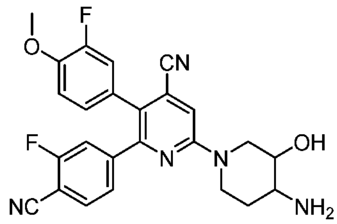 , 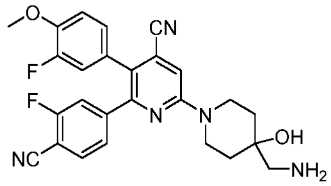 ,

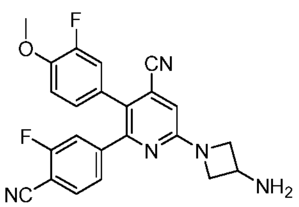 , 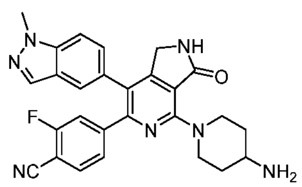 , 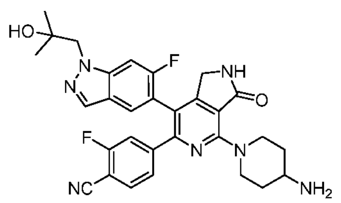 ,

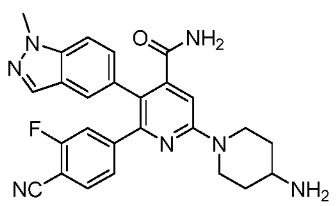 , 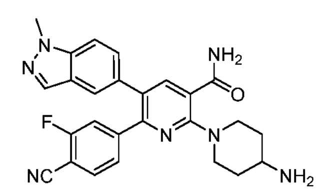 , 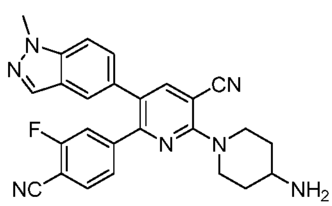 ,

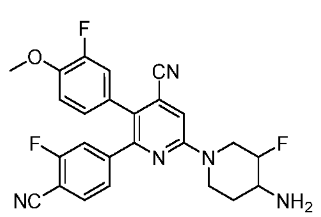, 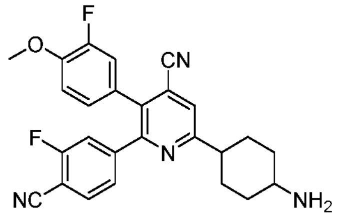, 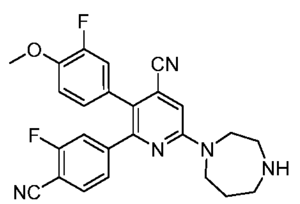,

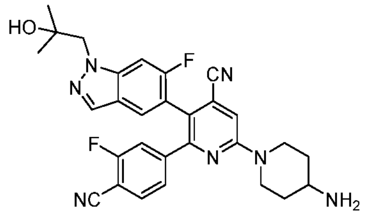, 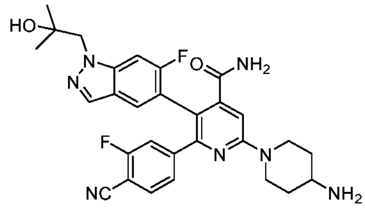, 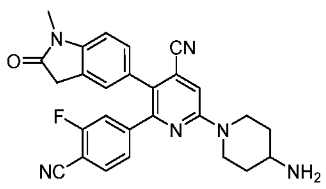,

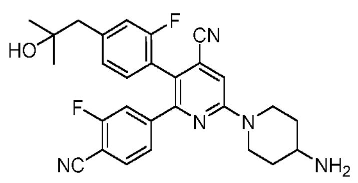, 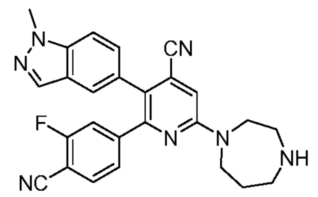, 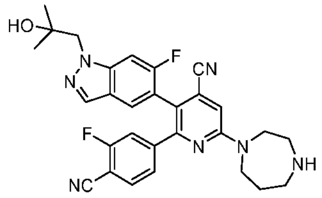,

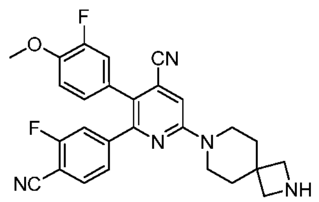, 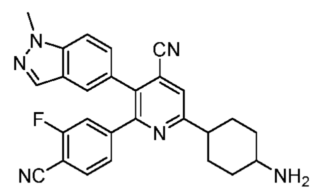, 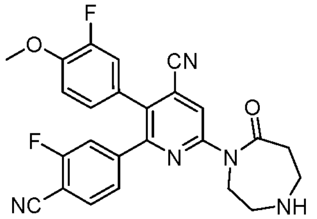,

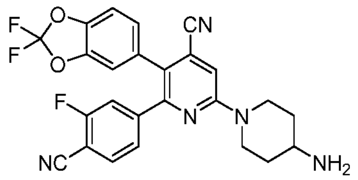, 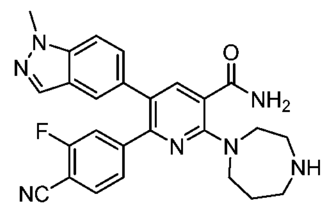, 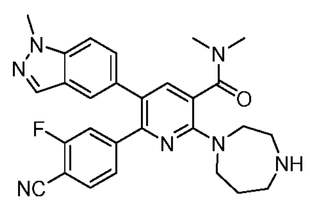,

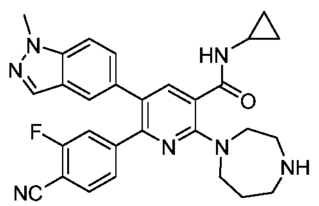, 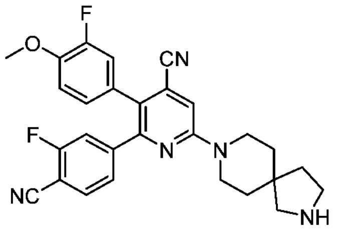, 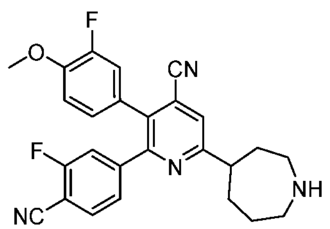,

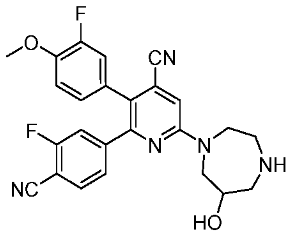,

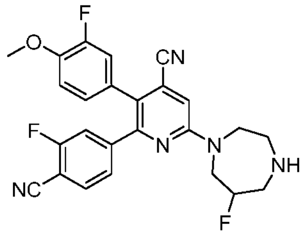,

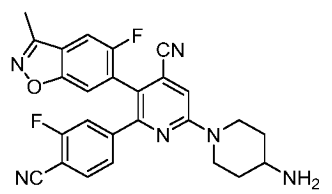,

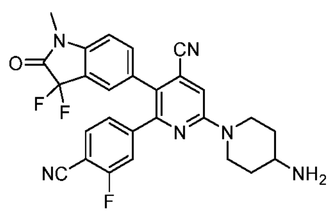,

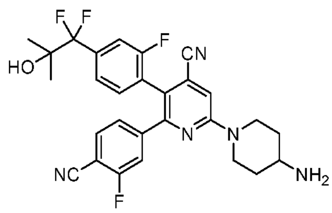,

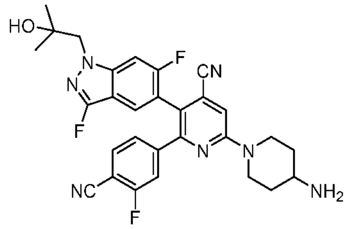,

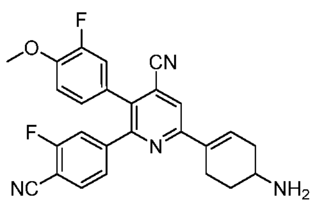,

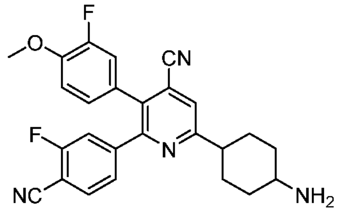,

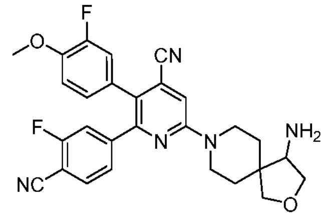,

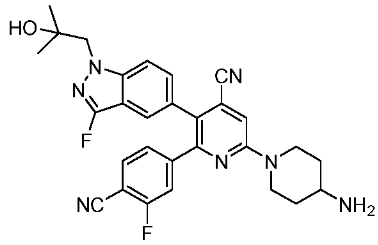,

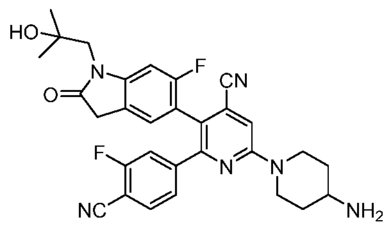,

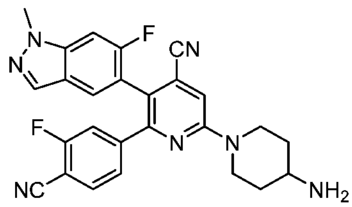,

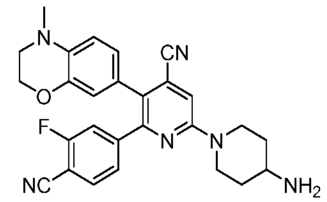,

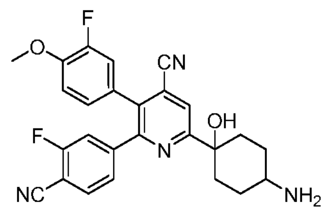,

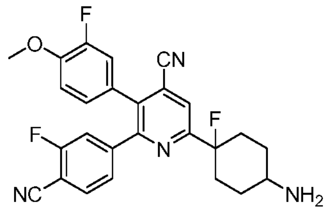,

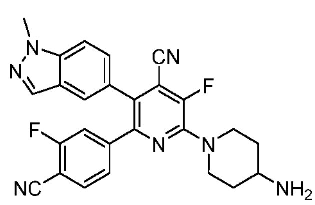,

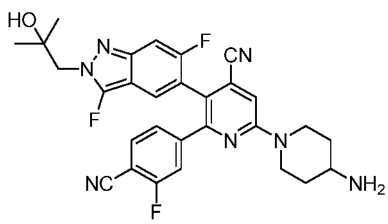,

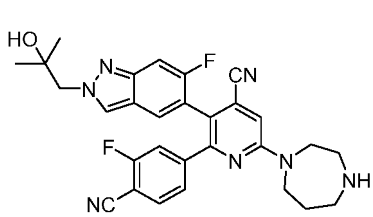

and

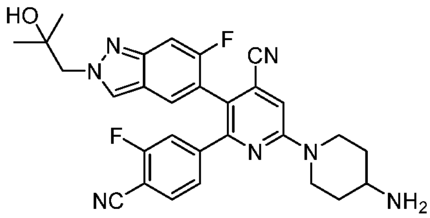

The present application also covers solutions obtained through any combination, deletion, or exchange of the above implementations and preferred solutions.

Another aspect of the present application provides a preparation method for preparing the pyridine derivative with the structure shown in formula I above, and the method includes the following steps: method I:

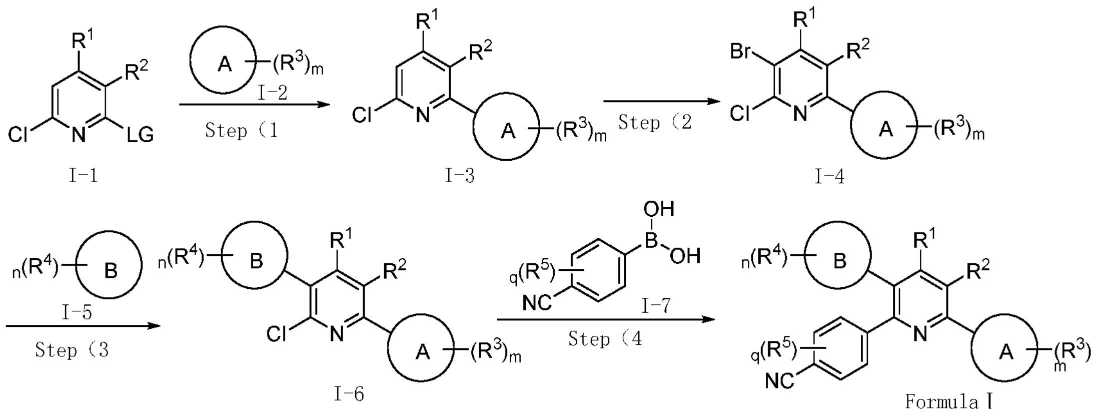


wherein LG represents a leaving group, and the leaving group includes but is not limited to a halogen atom, methylsulfonyloxy, and p-tosyloxy. $R^1$, $R^2$, $R^3$, $R^4$, $R^5$, a ring A, a ring B, m, n and q are defined as in the pyridine derivative with the structure shown in formula I above.

(1) A compound 1-1 reacts with a compound I-2 to obtain a compound I-3

The reaction is preferably carried out in a suitable organic solvent. The organic solvent may be selected from the group consisting of tetrahydrofuran, N,N-dimethylformamide, *N,N*-dimethylacetamide, 1,4-dioxane and any combination thereof, preferably *N,N*-dimethylformamide. The reaction is preferably carried out with the presence of a suitable base and/or carried out with the presence of a transition metal catalyst and a ligand. The base may be selected from the group consisting of triethylamine, pyridine, 4-dimethylaminopyridine, diisopropylethylamine, potassium carbonate, cesium carbonate, sodium carbonate and potassium tert-butoxide, preferably potassium carbonate. The transition metal catalyst may be selected from the group consisting of $Pd(dppf)Cl_2$, $Pd(OAc)_2$, $Pd_2(dba)_3$, $Pd(PPh_3)_4$ and RuPhos-Pd-$G_3$. The reaction is preferably carried out under a suitable temperature which is preferably 50°C to 80°C. The reaction is preferably carried out for suitable time such as 2 to 8 hours.

(2) The compound I-3 is subjected to a substitution reaction to obtain a compound I-4

The reaction is preferably carried out in a suitable organic solvent. The organic solvent may be selected from the group consisting of acetonitrile, toluene, chloroform, tetrahydrofuran and any combination thereof, preferably acetonitrile. The reaction is preferably carried out with the presence of a suitable bromo reagent. The bromo reagent may be selected from the group consisting of N-bromosuccinimide, liquid bromine and pyridine bromine onium salt, preferably N-bromosuccinimide. The reaction is preferably carried out under a suitable temperature which is preferably 20°C to 50°C. The reaction is preferably carried out for suitable time such as 2 to 6 hours.

(3) The compound I-4 is subjected to a coupling reaction with boric acid or borate of a compound I-5 to obtain a compound I-6

The reaction is preferably carried out in a suitable organic solvent. The organic solvent may be selected from the group consisting of tetrahydrofuran, 1,4-dioxane, toluene, acetonitrile, ethanol, water and any combination thereof, preferably a combination of 1,4-dioxane and water. The reaction is preferably carried out with the presence of a suitable catalyst. The catalyst may be selected from the group consisting of $Pd(dppf)Cl_2$, $Pd(OAc)_2$, $Pd_2(dba)_3$ and $Pd(PPh_3)_4$, preferably Pd(dppf)Clz. The reaction is preferably carried out with the presence of a suitable base. The base may be selected from the group consisting of triethylamine, pyridine, 4-dimethylaminopyridine, diisopropylethylamine, potassium carbonate, cesium carbonate and sodium carbonate, preferably potassium carbonate. The reaction is preferably carried out under a suitable temperature which is preferably 80°C to 120°C. The reaction is

preferably carried out for suitable time such as 8 to 12 hours.

(4) The compound I-6 is subjected to a coupling reaction with a compound I-7 to obtain the compound with the structure shown in formula I

The reaction is preferably carried out in a suitable organic solvent. The organic solvent may be selected from the group consisting of tetrahydrofuran, 1,4-dioxane, toluene, acetonitrile, ethanol, water and any combination thereof, preferably a combination of 1,4-dioxane and water. The reaction is preferably carried out with the presence of a suitable catalyst. The catalyst may be selected from the group consisting of $Pd(dppf)Cl_2$, $Pd(OAc)_2$, $Pd_2(dba)_3$ and $Pd(PPh_3)_4$, preferably $Pd(dppf)Cl_2$. The reaction is preferably carried out with the presence of a suitable base. The base may be selected from the group consisting of triethylamine, pyridine, 4-dimethylaminopyridine, diisopropylethylamine, potassium carbonate, cesium carbonate and sodium carbonate, preferably potassium carbonate. The reaction is preferably carried out under a suitable temperature which is preferably 80°C to 120°C. The reaction is preferably carried out for suitable time such as 8 to 12 hours.

Method II:

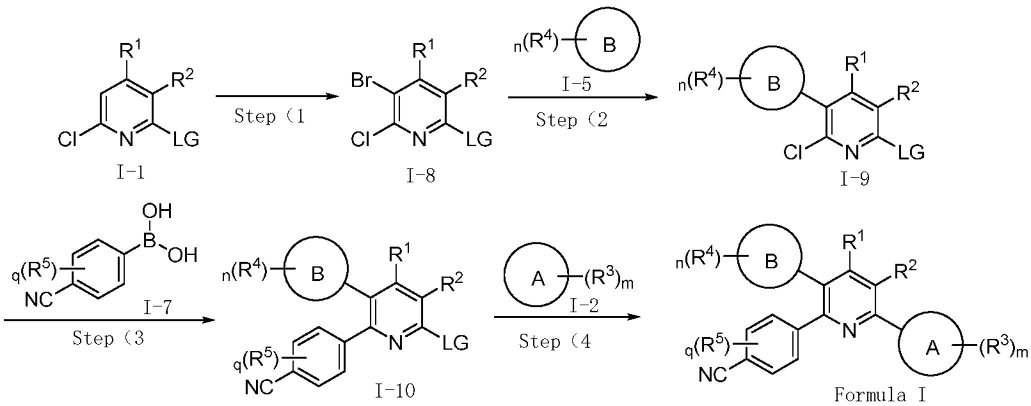


wherein LG represents a leaving group, and the leaving group includes but is not limited to a halogen atom, methylsulfonyloxy, and p-tosyloxy. $R^1$, $R^2$, $R^3$, $R^4$, $R^5$, a ring A, a ring B, m, n and q are defined as in the pyridine derivative with the structure shown in formula I above.

(1) A compound I-1 is subjected to a substitution reaction to obtain a compound I-8

The reaction is preferably carried out in a suitable organic solvent. The organic solvent may be selected from the group consisting of acetonitrile, toluene, chloroform, tetrahydrofuran and any combination thereof, preferably acetonitrile. The reaction is preferably carried out with the presence of a suitable bromo reagent. The bromo reagent may be selected from the group consisting of N-bromosuccinimide, liquid bromine and pyridine bromine onium salt, preferably N-bromosuccinimide. The reaction is preferably carried out under a suitable temperature which is preferably 20°C to 50°C. The reaction is preferably carried out for suitable time such as 2 to 6 hours.

(2) The compound I-8 is subjected to a coupling reaction with boric acid or borate of a compound I-5 to obtain a compound I-9

The reaction is preferably carried out in a suitable organic solvent. The organic solvent may be selected from the group consisting of tetrahydrofuran, 1,4-dioxane, toluene, acetonitrile, ethanol, water and any combination thereof, preferably a combination of 1,4-dioxane and water. The reaction is preferably carried out with the presence of a suitable catalyst. The catalyst may be selected from the group consisting of $Pd(dppf)Cl_2$, $Pd(OAc)_2$, $Pd_2(dba)_3$ and $Pd(PPh_3)_4$, preferably $Pd(dppf)Cl_2$. The reaction is preferably carried out with the presence of a suitable base. The base may be selected from the group consisting of triethylamine, pyridine, 4-dimethylaminopyridine, diisopropylethylamine, potassium carbonate, cesium carbonate and sodium carbonate, preferably potassium carbonate. The reaction is preferably carried out under a suitable temperature which is preferably 80°C to 120°C. The reaction is preferably carried out for suitable time such as 8 to 12 hours.

(3) The compound I-9 is subjected to a coupling reaction with a compound I-7 to obtain a compound I-10

The reaction is preferably carried out in a suitable organic solvent. The organic solvent may be selected from the group consisting of tetrahydrofuran, 1,4-dioxane, toluene, acetonitrile, ethanol, water and any combination thereof, preferably a combination of 1,4-dioxane and water. The reaction is preferably carried out with the presence of a suitable catalyst. The catalyst may be selected from the group consisting of $Pd(dppf)Cl_2$, $Pd(OAc)_2$, $Pd_2(dba)_3$ and

$Pd(PPh_3)_4$, preferably Pd(dppf)Clz. The reaction is preferably carried out with the presence of a suitable base. The base may be selected from the group consisting of triethylamine, pyridine, 4-dimethylaminopyridine, diisopropylethylamine, potassium carbonate, cesium carbonate and sodium carbonate, preferably potassium carbonate. The reaction is preferably carried out under a suitable temperature which is preferably 80°C to 120°C. The reaction is preferably carried out for suitable time such as 8 to 12 hours.

(4) The compound I-10 reacts with a compound I-2 to obtain the compound with the structure shown in formula I

The reaction is preferably carried out in a suitable organic solvent. The organic solvent may be selected from the group consisting of tetrahydrofuran, *N,N*-dimethylformamide, N,N-dimethylacetamide, 1,4-dioxane and any combination thereof, preferably *N,N*-dimethylformamide. The reaction is preferably carried out with the presence of a suitable base and/or carried out with the presence of a transition metal catalyst and a ligand. The base may be selected from the group consisting of triethylamine, pyridine, 4-dimethylaminopyridine, diisopropylethylamine, potassium carbonate, cesium carbonate, sodium carbonate and potassium tert-butoxide, preferably potassium carbonate. The transition metal catalyst may be selected from the group consisting of $Pd(dppf)Cl_2$, $Pd(OAc)_2$, $Pd_2(dba)_3$, $Pd(PPh_3)_4$ and RuPhos-Pd-Gs. The reaction is preferably carried out under a suitable temperature which is preferably 50°C to 80°C. The reaction is preferably carried out for suitable time such as 2 to 8 hours.

Method III:

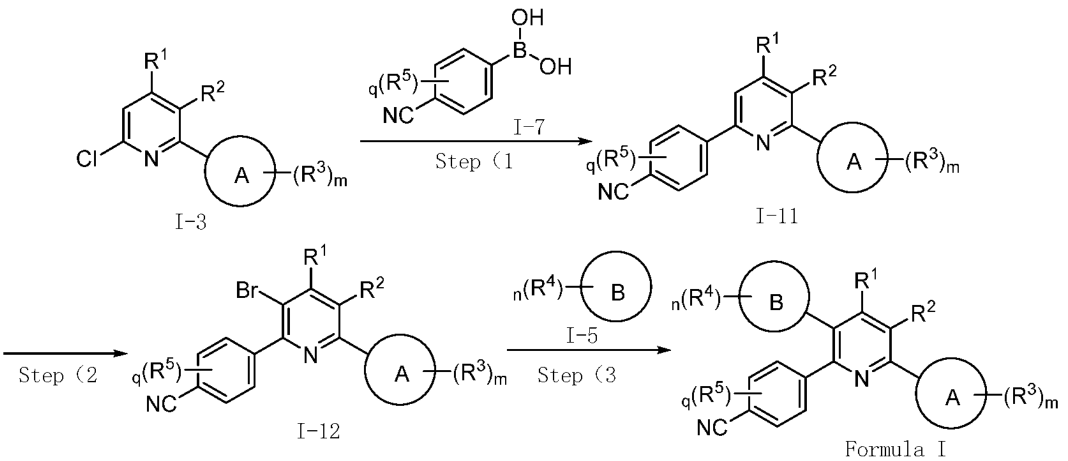


wherein LG represents a leaving group, and the leaving group includes but is not limited to a halogen atom, methylsulfonyloxy, and p-tosyloxy. $R^1$, $R^2$, $R^3$, $R^4$, $R^5$, a ring A, a ring B, m, n and q are defined as in the pyridine derivative with the structure shown in formula I above.

(1) A compound I-3 is subjected to a coupling reaction with a compound I-7 to obtain a compound I-11

The reaction is preferably carried out in a suitable organic solvent. The organic solvent may be selected from the group consisting of tetrahydrofuran, 1,4-dioxane, toluene, acetonitrile, ethanol, water and any combination thereof, preferably a combination of 1,4-dioxane and water. The reaction is preferably carried out with the presence of a suitable catalyst. The catalyst may be selected from the group consisting of $Pd(dppf)Cl_2$, $Pd(OAc)_2$, $Pd_2(dba)_3$ and $Pd(PPh_3)_4$, preferably Pd(dppf)Clz. The reaction is preferably carried out with the presence of a suitable base. The base may be selected from the group consisting of triethylamine, pyridine, 4-dimethylaminopyridine, diisopropylethylamine, potassium carbonate, cesium carbonate and sodium carbonate, preferably potassium carbonate. The reaction is preferably carried out under a suitable temperature which is preferably 80°C to 120°C. The reaction is preferably carried out for suitable time such as 8 to 12 hours.

(2) The compound I-11 is subjected to a substitution reaction to obtain a compound I-12

The reaction is preferably carried out in a suitable organic solvent. The organic solvent may be selected from the group consisting of acetonitrile, toluene, chloroform, tetrahydrofuran and any combination thereof, preferably acetonitrile. The reaction is preferably carried out with the presence of a suitable bromo reagent. The bromo reagent may be selected from the group consisting of N-bromosuccinimide, liquid bromine and pyridine bromine onium salt, preferably N-bromosuccinimide. The reaction is preferably carried out under a suitable temperature which is preferably 20°C to 50°C. The reaction is preferably carried out for suitable time such as 2 to 6 hours.

(3) The compound I-12 is subjected to a coupling reaction with boric acid or borate of a compound I-5 to obtain the

compound of formula I

The reaction is preferably carried out in a suitable organic solvent. The organic solvent may be selected from the group consisting of tetrahydrofuran, 1,4-dioxane, toluene, acetonitrile, ethanol, water and any combination thereof, preferably a combination of 1,4-dioxane and water. The reaction is preferably carried out with the presence of a suitable catalyst. The catalyst may be selected from Pd(dppf)$Cl_2$, $Pd(OAc)_2$, $Pd_2(dba)_3$, $Pd(PPh_3)_4$, preferably Pd(dppf)$Cl_2$. The reaction is preferably carried out with the presence of a suitable base. The base may be selected from the group consisting of triethylamine, pyridine, 4-dimethylaminopyridine, diisopropylethylamine, potassium carbonate, cesium carbonate and sodium carbonate, preferably potassium carbonate. The reaction is preferably carried out under a suitable temperature which is preferably 80°C to 120°C. The reaction is preferably carried out for suitable time such as 8 to 12 hours.

The specific conditions for the above reaction steps are well-known in the art and are not specifically limited in the present application. As the teachings of the present application are combined with common knowledge in the art, those skilled in the art can select and replace each substituent in the general formula to prepare different compounds, all of which are within the scope of protection of the present application.

The present application further relates to a pharmaceutical composition, including the above compound of formula I, and the stereoisomer or the pharmaceutically acceptable salt thereof as well as a pharmaceutically acceptable excipient.

The present application further relates to a use of the above compound of formula I, and the stereoisomer or the pharmaceutically acceptable salt thereof, or the above pharmaceutical composition in preparation of drugs for preventing or treating a lysine-specific demethylase 1-related disease. Or, the present application further relates to a compound of formula I, and a stereoisomer or a pharmaceutically acceptable salt thereof, or the above pharmaceutical composition used for preventing or treating an LSD1-related disease. Or, the present application further relates to a method for preventing or treating an LSD1-related disease, including administrating to a subject in need the above compound of formula I, and the stereoisomer or the pharmaceutically acceptable salt thereof, or the above pharmaceutical composition.

In some embodiments, the LSD1-related disease is a tumor or a cancer, such as acute myeloid leukemia (AML), chronic myeloid leukemia (CML), acute lymphocytic leukemia (ALL), chronic lymphocytic leukemia (CLL), small cell lung cancer (SCLC), non-small cell lung cancer (NSCLC), lymphoma, malignant sarcoma, breast cancer, cervical cancer, colon cancer, lung cancer, oral cancer, brain cancer, stomach cancer, liver cancer, colorectal cancer, pancreatic cancer, skin cancer, prostate cancer, bone cancer, kidney cancer, ovarian cancer, bladder cancer, fallopian tube tumor, peritoneal tumor, melanoma, glioma, neuroblastoma, mastoid malignant tumor, head and neck tumor or myeloma.

The present application further relates to a use of the above compound of formula I, and the stereoisomer or the pharmaceutically acceptable salt thereof, or the above pharmaceutical composition in preparation of an LSD1 inhibitor. Or, the present application further relates to the above compound of formula I, and the stereoisomer or the pharmaceutically acceptable salt thereof, or the above pharmaceutical composition for use as an LSD1 inhibitor. Or, the present application further relates to a method for inhibiting LSD1, including administrating to a subject in need the above compound of formula I, and the stereoisomer or the pharmaceutically acceptable salt thereof, or the above pharmaceutical composition.

The present application discovers a type of brand-new-structure LSD1 inhibitor having a structure as shown in formula I, and the inhibitor has good inhibiting activity.

## BRIEF DESCRIPTION OF THE DRAWINGS

FIG. 1 shows a change situation of a tumor volume in mice of a test compound group and a solvent group, wherein Cpd represents a compound.

FIG. 2 shows a tumor weight change situation in a test compound group and a solvent group.

FIG. 3 shows a weight change rate of mice of a test compound group and a solvent group.

FIG. 4 shows a change situation of a tumor volume in mice of a test compound group and a solvent group.

FIG. 5 shows a weight change rate of mice of a test compound group and a solvent group.

## DETAILED DESCRIPTION

In order to make the aspects and technical solutions of the present application clearer, the following will further elaborate on the present application in conjunction with specific examples. It should be understood that these examples are merely used for illustrating the present application, instead of limiting the scope of the present application. Moreover, specific experimental methods not mentioned in the following examples are all carried out according to conventional experimental methods.

**Definition and Explanation**

Unless otherwise stated, the terms used in the present application have the following meanings. A specific term should not be considered uncertain or unclear without a specific definition, but should be understood according to the common meaning in the art.

When covalent bonds in certain structural units or groups in the present application are not connected to specific atoms, it indicates that the covalent bonds may be connected to any atom in the structural units or groups, as long as they do not violate the rules of valent bond connection.

Herein, unless otherwise stated, "alkoxy" refers to -O-alkyl.

Herein, unless otherwise stated, "halogen" refers to fluorine, chlorine, bromine or iodine.

Herein, unless otherwise stated, "cycloalkyloxy" refers to -O-cycloalkyl.

Herein, unless otherwise stated, "saturated cycloalkyl" covers bridged rings in monocyclic and dicyclic forms, spiro in a dicyclic form, etc., and refers to fully saturated alicyclic hydrocarbon. For example, cycloalkyl herein may be $C_3$-$C_{10}$ monocyclic cycloalkyl, $C_5$-$C_{15}$ spiro cycloalkyl or $C_4$-$C_{12}$ bridged-ring cycloalkyl. Non-limiting instances of cycloalkyl include but are not limited to cyclopropyl, cyclobutyl, cyclopentyl, cyclohexyl, cycloheptyl, cyclooctyl, spiropentyl, spirohexyl, spiroheptyl, spiroheptyl, spirooctyl, spirononyl, spirodecyl, spiroundecyl, etc.

Herein, unless otherwise stated, "saturated heterocycloalkyl" refers to cycloalkyl in which one or more (such as 1-6, 1-5, 1-3, 1 or 2) carbon atoms are substituted with heteroatoms selected from the group consisting of N, O and S.

Herein, unless otherwise stated, "cyclohydrocarbyl" refers to hydrocarbyl with carbon atoms forming a ring, including saturated cyclohydrocarbyl and unsaturated cyclohydrocarbyl (such as aryl). Herein, unsaturated cyclohydrocarbyl is also sometimes referred to as unsaturated cycloalkyl. Herein, cyclohydrocarbyl may be a bridged ring in a monocyclic or dicyclic form, spiro in a dicyclic form, and a fused ring in a dicyclic form, such as $C_3$-$C_{10}$ monocyclic cyclohydrocarbyl, $C_5$-$C_{15}$ spiro cyclohydrocarbyl and $C_4$-$C_{12}$ bridged-ring cyclohydrocarbyl.

Herein, unless otherwise stated, "substituted or unsubstituted amino" covers unsubstituted amino or amino substituted with groups selected from the group consisting of $C_1$-$C_6$ alkyl, $C_1$-$C_6$ alkoxy, $C_3$-$C_8$ cycloalkyl and halogen. herein, unless otherwise stated, the used term "$C_m$-$C_n$" refers to that a part modified by this term has m-n carbon atoms (n is greater than m and both are integers). For example, $C_1$-$C_6$ indicates that a part modified by it has 1-6 carbon atoms, such as 1 carbon atom, 2 carbon atoms, 3 carbon atoms, 4 carbon atoms, 5 carbon atoms, or 6 carbon atoms.

The term "subject" is equivalent to "patient" and "individual" and represents humans or non-human animals (mammals, such as primates and rodents). "Mammals" include humans and livestock (such as laboratory mammals and household pets, such as cats, dogs, pigs, sheep, cows, sheep, goats, horses and rabbits), as well as non-domesticated mammals, such as wild mammals.

The term "treatment" means administering the compound or preparation described in the present application to alleviate or eliminate diseases or one or more symptoms related to the diseases, and includes inhibiting the progression of the diseases or symptoms, and alleviating the diseases or symptoms.

The term "pharmaceutically acceptable" refers to that a carrier, a vehicle, a diluent, an excipient, and/or a formed salt are/is typically chemically or physically compatible with other compositions that make up a drug formulation, and physiologically compatible with a receptor, without excessive toxicity, irritation, allergic reaction, or other issues or complications, commensurate with a reasonable benefit/risk ratio.

In the present application, the terms "including", "comprising", "containing" and their equivalents shall be understood as open and non-exclusive, meaning "including but not limited to", which means that in addition to the listed elements, components, and steps, other unspecified elements, components, and steps may also be covered. Herein, unless the context otherwise specifies clearly, singular terms cover plural referents, and vice versa. Similarly, unless the context otherwise indicates clearly, the word "or" is intended to include "and".

Unless otherwise stated, herein, parameter values representing the quantity, physicochemical properties, or reaction conditions of compositions should be understood to be modified by the term "about" in all cases. When the term "about" is used to describe the present application, the term "about" indicates the presence of error values, such as variations within a range of $\pm$5%, such as $\pm$1% or $\pm$0.1%, of a certain specific value.

In the present application, when chemical names and structural formulas are inconsistent, the one shown in the structural formulas shall prevail, unless it can be inferred from the context that the chemical names rather than the structural formulas are correct.

Abbreviations herein have the following meanings:

| Abbreviation | Meaning | Abbreviation | Meaning |
|---|---|---|---|
| DMSO-$d_6$ | Hexadeuterodimethyl sulfoxide | q | Quartet |
| TMS | Tetramethyl silane | dd | Doublet of doublets |

(continued)

| Abbreviation | Meaning | Abbreviation | Meaning |
|---|---|---|---|
| $^1H$ NMR | Proton nuclear magnetic resonance | m | Multiplet |
| MS | Mass spectrometry | br | Broad peak |
| s | Singlet | *J* | Coupling constant |
| d | Doublet | Hz | Hertz |
| t | Triplet | NBS | *N*-bromosuccinimide |

Structures of compounds are determined through mass spectrometry (MS) or proton nuclear magnetic resonance ($^1H$ NMR). Proton nuclear magnetic resonance ($^1H$ NMR) displacement (δ) is given with a unit of parts per million (ppm). The determination of proton nuclear magnetic resonance ($^1H$ NMR) adopts a BrukerAVANCE-400 NMR spectrometer, a determination solvent is deuterodimethyl sulfoxide (DMSO-$d_6$), an internal standard is tetramethyl silane (TMS), and chemical displacement is given with a unit of $10^{-6}$ (ppm). The determination of mass spectrometry (MS) is performed using a FINNIGAN LCQAd (ESI) mass spectrometer (Manufacturer: Therm, Model: Finnigan LCQ advantage MAX). Thin layer silica gel uses Yantai Huanghai HSGF254 or Qingdao GF254 silica gel plates. Column chromatography generally uses silica gel of 200-300 meshes from Yantai Huanghai silica gel as a carrier.

Unless otherwise stated in the present application, all reactions mentioned in the present application are carried out under a nitrogen atmosphere. The term "nitrogen atmosphere" in the present application refers to, for example, connecting a reaction flask to a 1 L volume nitrogen balloon.

The term "hydrogen atmosphere" in the present application refers to, for example, connecting a reaction flask to a 1 L volume hydrogen balloon.

Unless otherwise stated in the present application, solutions mentioned in reactions of the present application are aqueous solutions.

The term "room temperature" in the present application refers to a temperature between 10°C and 25°C.

For the purpose of description and disclosure, all patents, patent applications, and other established publications are hereby expressly incorporated by reference. These publications are provided only because their publication occurred earlier than the filing date of the present application. All statements regarding the dates of these documents or expression of the contents of these documents is based on information available to the applicant and does not constitute any recognition of the correctness of the dates or contents of these documents. Moreover, in any country, any reference to these publications herein does not constitute recognition that the publication becomes part of common knowledge in the art.

The present application will be explained in detail below through examples, but those skilled in the art can understand that the scope of protection of the present application is not limited to this. Those skilled in the art can make various modifications, changes, combinations, etc. to the implementations and examples of the present application without departing from the spirit or scope of the present application, and the adjusted solution obtained from this is also within the scope of protection of the present application.

**Example** 1 Preparation of 6-(4-atninopiperid-1-yl)-2-(4-cyano-3-fluorophenyl)-3-(3-fluoro-4-methoxyphenyl)isonicotinonitrile (1)

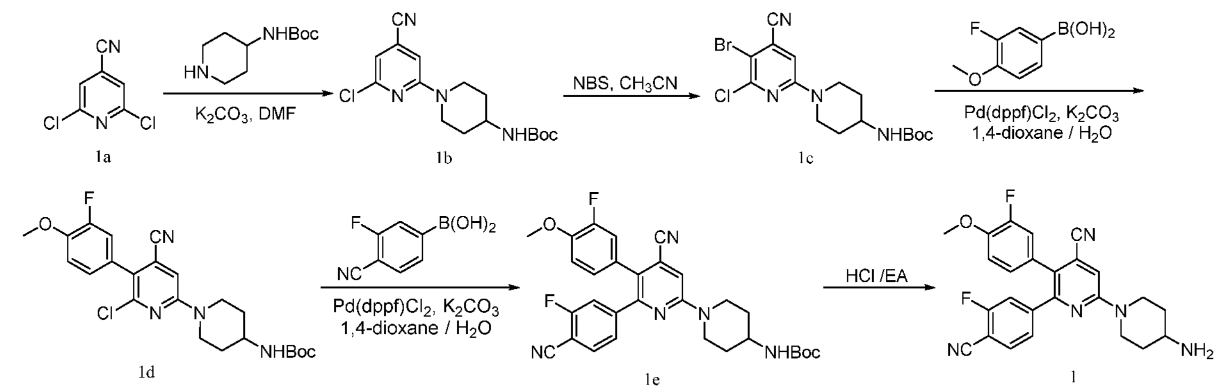

1a 1b 1c 1d 1e 1

First step: preparation of tert-butyl (1-(6-chloro-4-cyanopyrid-2-yl)piperid-4-yl)carbamate (1b)

A compound 1a (550 mg, 3.18 mmol), 4-tert-butoxycarbonyl aminopiperidine (643 mg, 3.21 mmol), and $K_2CO_3$ (658 mg, 4.76 mmol) were dissolved in *N,N*-dimethylformamide (10 mL), and this system was heated to 80°C and stirred to react overnight. A reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 856 mg of a title compound, a yield being 80%.

Second step: preparation of tert-butyl (1-(5-bromo-6-chloro-4-cyanopyrid-2-yl)piperid-4-yl)carbamate (1c)

The compound 1b (538 mg, 1.60 mmol) was dissolved in acetonitrile (15 mL), NBS (288 mg, 1.62 mmol) was slowly added into a system under an ice bath, and a reaction system was placed at the room temperature and stirred to react for 2 hours. The reaction system was subjected to vacuum concentration, and a crude product was purified using silica gel column chromatography to obtain 542 mg of a title compound, a yield being 82%.

Third step: preparation of tert-butyl [1-(6-chloro-4-cyano-5-(3-fluoro-4-methoxyphenyl)pyrid-2-yl)piperid-4-yl]carbamate (1d)

The compound 1c (231 mg, 0.56 mmol), 3-fluoro-4-methoxyphenylboronic acid (97 mg, 0.57 mmol) and $K_2CO_3$ (116 mg, 0.84 mmol) were dissolved in 1,4-dioxane (5 mL) and water (1 mL), replacement with nitrogen was carried out, Pd(dppf)$Cl_2$ (44 mg, 0.06 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 126 mg of a title compound, a yield being 49%.

Fourth step: preparation of tert-butyl (1-(4-cyano-6-(4-cyano-3-fluorophenyl)-5-(3-fluoro-4-methoxyphenyl)pyrid-2-yl) piperid-4-yl)carbamate (1e)

The compound 1d (47 mg, 0.10 mmol), 3-fluoro-4-cyanophenylboronic acid (25 mg, 0.15 mmol) and $K_2CO_3$ (41 mg, 0.30 mmol) were dissolved in 1,4-dioxane (1 mL) and water (0.2 mL), replacement with nitrogen was carried out, Pd(dppf)$Cl_2$ (7 mg, 0.01 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 27 mg of a title compound, a yield being 49%.

Fifth step: preparation of 6-(4-atninopiperid-1-yl)-2-(4-cyano-3-fluorophenyl)-3-(3-fluoro-4-methoxyphenyl)isonicotinonitrile (1)

The compound 1e (27 mg, 0.05 mmol) was dissolved in ethyl acetate (1 mL), then a hydrochloric acid-ethyl acetate solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 16 mg of a title compound, a yield being 73%.

LC-MS (ESI) m/z $(M+H)^+$: 446.2
$^1$H NMR (400 MHz, DMSO-$d_6$) δ 7.83-7.77 (m, 1H), 7.53 (s, 1H), 7.37 (dd, J = 10.8, 1.2 Hz, 1H), 7.21-7.10 (m, 3H), 6.98-6.93 (m, 1H), 4.34-4.25 (m, 2H), 3.84 (s, 3H), 3.08-2.98 (m, 2H), 2.89-2.79 (m, 1H), 1.82-1.73 (m, 2H), 1.25-1.14 (m, 2H).

**Example** 2 Preparation of 6-(4-atninopiperid-1-yl)-2-(4-cyano-3-fluorophenyl)-3-(3-fluoro-4-methoxyphenyl)isonicotinamide (2)

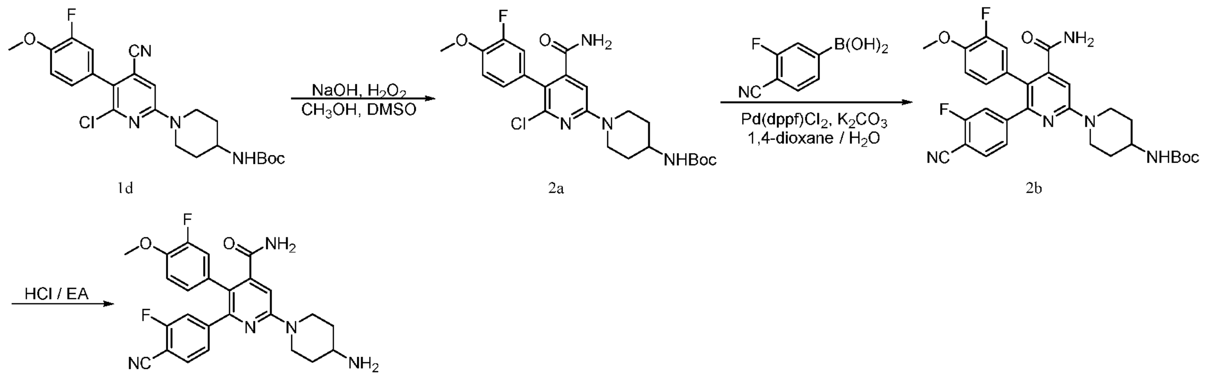


1d

2a

2b

2

First step: preparation of tert-butyl [1-(4-carbamoyl-6-chloro-5-(3-fluoro-4-methoxyphenyl)pyrid-2-yl)piperid-4-yl]carbamate (2a)

A compound 1d (125 mg, 0.27 mmol) was dissolved in a methanol solution (1 mL), then dimethyl sulfoxide (1 mL) and NaOH (32 mg, 0.80 mmol) were added in sequence, $H_2O_2$ (153 mg, 1.35 mmol, 30% aqueous solution) was slowly added dropwise into a reaction system under stirring, and the reaction system was placed at 50°C and stirred to react for 2 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 90 mg of a title compound, a yield being 69%.

Second step: preparation of tert-butyl (1-(4-carbatnoyl-6-(4-cyano-3-fluorophenyl)-5-(3-fluoro-4-methoxyphenyl)pyrid-2-yl)piperid-4-yl)carba mate (2b)

The compound 2a (55 mg, 0.11 mmol), 3-fluoro-4-cyanophenylboronic acid (28 mg, 0.17 mmol) and $K_2CO_3$ (46 mg, 0.33 mmol) were dissolved in 1,4-dioxane (1 mL) and water (0.2 mL), replacement with nitrogen was carried out, Pd(dppf)$Cl_2$ (8 mg, 0.01 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 48 mg of a title compound, a yield being 77%.

Third step: preparation of 6-(4-atninopiperid-1-yl)-2-(4-cyano-3-fluorophenyl)-3-(3-fluoro-4-methoxyphenyl)isonicotinamide (2)

The compound 2b (48 mg, 0.09 mmol) was dissolved in ethyl acetate (1 mL), then a hydrochloric acid-ethyl acetate solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 31 mg of a title compound, a yield being 79%.

LC-MS (ESI) m/z $(M+H)^+$: 464.2
$^1$H NMR (400 MHz, DMSO-$d_6$) δ 7.80-7.72 (m, 2H), 7.45-7.40 (m, 1H), 7.31 (dd, $J$ = 10.8, 1.2 Hz, 1H), 7.14 (dd, $J$ = 8.0, 1.2 Hz, 1H), 7.02 (t, $J$ = 8.4 Hz, 1H), 6.95 (dd, $J$ = 12.4, 2.4 Hz, 1H), 6.88 (s, 1H), 6.88-6.72 (m, 1H), 4.32-4.23 (m, 2H), 3.80 (s, 3H), 3.02-2.92 (m, 2H), 2.86-2.77 (m, 1H), 1.85-1.73 (m, 2H), 1.26-1.13 (m, 2H).

Example 3 Preparation of 4-(4-(4-atninopiperid-1-yl)-7-(3-fluoro-4-methoxyphenyl)-3-oxo-2,3-dihydro-1H-pyrrole[3,4-c]pyrid-6-y 1)-2-fluorobenzonitrile (3)

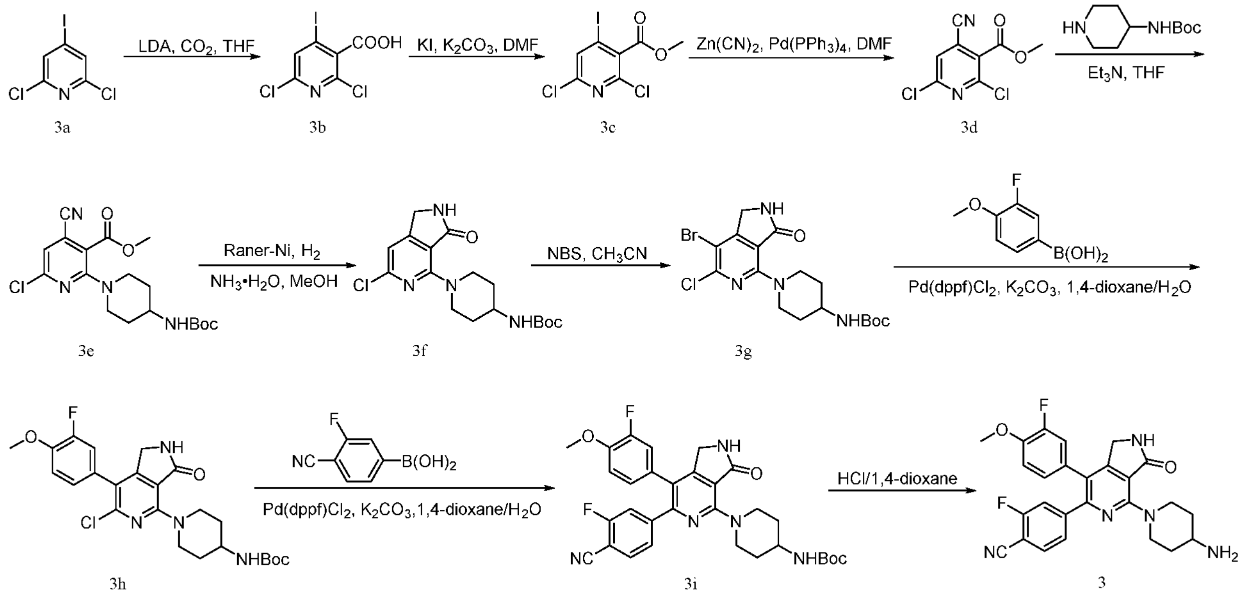

3a 3b 3c 3d 3e 3f 3g 3h 3i 3

First step: preparation of 2,6-dichloro-4-iodonicotinic acid (3b)

A compound 3a (10.0 g, 36.51 mmol) was dissolved in dry tetrahydrofuran (110 mL), replacement with nitrogen was carried out, a system was placed at -50°C, LDA (20 mL, 40.00 mmol, 2 mol/L in THF/Heptane/Ethylbenzene) was slowly added dropwise into the system, and after dropwise adding was finished, the system was maintained at -50°C and continued to be stirred to react for 2 hours. A reaction liquid was quickly poured into a measuring cup containing smashed dry ice and stirred, the reaction liquid slowly restored to the room temperature, the reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was directly used for the next step of reaction without further purification.

Second step: preparation of 2,6-dichloro-4-methyl iodonicotinate (3c)

The compound 3b (12.0 g, 37.75 mmol) was dissolved in *N,N*-dimethylformamide (130 mL), then $K_2CO_3$ (7.9 g, 57.16 mmol) and iodomethane (8.1 g, 57.04 mmol) were added in sequence, and a reaction system was stirred at the room temperature to react for 1 hour. A reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 8.3 g of a title compound, a total yield in the two steps being 68%.

Third step: preparation of 2,6-dichloro-4-methyl cyanonicotinate (3d)

The compound 3c (4.3 g, 12.95 mmol) and $Zn(CN)_2$ (4.56 g, 38.85 mmol) were dissolved in N,N-dimethylformamide (40 mL), replacement with nitrogen was carried out, $Pd(PPh_3)_4$ (750 mg, 0.65 mmol) was added, replacement with nitrogen was carried out again, and a reaction system was placed at 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 1.0 g of a title compound, a yield being 33%.

Fourth step: preparation of 2-(4-((tert-butoxycarbonyl)amino)piperid-1-yl)-6-chloro-4-methyl cyanonicotinate (3e)

The compound 3d (1.0 g, 4.33 mmol), 4-tert-butoxycarbonyl aminopiperidine (1.3 g, 6.49 mmol) and triethylamine (900 mg, 8.89 mmol) were dissolved in tetrahydrofuran (10 mL). A system was heated to 50°C and stirred to react for 3 hours. A reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 647 mg of a title compound, a yield being 38%.

Fifth step: preparation of tert-butyl (1-(6-chloro-3-oxo-2,3-dihydro-1*H*pyrrole[3,4-*c*]pyrid-4-yl)piperid-4-yl)carbamate (3f)

The compound 3e (647 mg, 1.64 mmol) was added into a reaction flask and dissolved in methanol (10 mL), then ammonium hydroxide (10 drops) and Raney-Ni (267 mg) were added in sequence, replacement with nitrogen was carried out, and a reaction system was placed at the room temperature under a hydrogen atmosphere to react overnight. A reaction liquid was filtered, a filtrate was subjected to vacuum concentration, and a crude product was purified using a thin layer silica gel plate to obtain 218 mg of a title compound, a yield being 36%.

Sixth step: preparation of tert-butyl (1-(7-bromo-6-chloro-3-oxy-2,3-dihydro-1*H*pyrrole[3,4-c]pyrid-4-yl)piperid-4-yl)carbamate (3g)

The compound 3f (175 mg, 0.48 mmol) was dissolved in acetonitrile (4 mL), NBS (128 mg, 0.72 mmol) was slowly added into a system under an ice bath condition, and a reaction system was stirred to react for 1 hour with the ice bath condition maintained. A reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 210 mg of a title compound, a yield being 99%.

Seventh step: preparation of tert-butyl (1-(6-chloro-7-(3-fluoro-4-methoxyphenyl)-3-oxo-2,3-dihydro-1Hpyrrolo[3,4-*c*] pyrid-4-yl)piperid-4-yl)c arbamate (3h)

The compound 3g (120 mg, 0.27 mmol), 3-fluoro-4-methoxyphenylboronic acid (46 mg, 0.27 mmol) and $K_2CO_3$ (112 mg, 0.81 mmol) were dissolved in 1,4-dioxane (2 mL) and water (0.2 mL), replacement with nitrogen was carried out, Pd(dppf)$Cl_2$ (36 mg, 0.05 mmol) was added, replacement with nitrogen was carried out again, and a reaction system was heated to 70°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 33 mg of a title compound, a yield being 25%.

Eighth step: preparation of tert-butyl (1-(6-(4-cyano-3-fluorophenyl)-7-(3-fluoro-4-methoxyphenyl)-3-oxo-2,3-dihydro-1*H*pyrrolo[3,4-*c*]pyrid-4-yl)piperid-4-yl)carbamate (3i)

The compound 3h (33 mg, 0.07 mmol), 3-fluoro-4-cyanophenylboronic acid (16 mg, 0.01 mmol) and $K_2CO_3$ (56 mg, 0.40 mmol) were dissolved in 1,4-dioxane (1 mL) and water (0.1 mL), replacement with nitrogen was carried out, Pd(dppf)$Cl_2$ (22 mg, 0.03 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 34 mg of a title compound, a yield being 88%.

Ninth step: preparation of 4-(4-(4-aminopiperid-1-yl)-7-(3-fluoro-4-methoxyphenyl)-3-oxo-2,3-dihydro-1*H*-pyrrole[3,4-*c*] pyrid-6-y 1)-2-fluorobenzonitrile (3)

The compound 3i (34 mg, 0.06 mmol) was dissolved in 1,4-dioxane (2 mL), then a hydrochloric acid-1,4-dioxane solution (2 mL, 4M in 1,4-dioxane) was added, and a reaction system was stirred at the room temperature to react for 2

hours. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 12 mg of a title compound, a yield being 43%.

LC-MS (ESI) m/z $(M+H)^+$: 476.2

$^1$H NMR (400 MHz, DMSO-$d_6$) δ 8.54 (s, 1H), 7.84-7.78 (m, 1H), 7.45 (dd, $J$ = 10.8, 1.2 Hz, 1H), 7.24 (dd, J= 8.0, 1.6 Hz, 1H), 7.18 (dd, $J$ = 12.0, 2.0 Hz, 1H), 7.12 (t, $J$ = 8.8 Hz, 1H), 6.95-6.90 (m, 1H), 4.49-4.37 (m, 2H), 4.15 (s, 2H), 3.83 (s, 3H), 3.06-2.95 (m, 2H), 2.87-2.76 (m, 1H), 2.03-1.93 (m, 1H), 1.86-1.74 (m, 2H), 1.39-1.33 (m, 1H).

**Example** 4 Preparation of 6-(4-aminopiperid-1-yl)-2-(4-cyano-3-fluorophenyl)-3-(1-methyl-1*H*-indazol-5-yl)isonicotinonitrile (4)

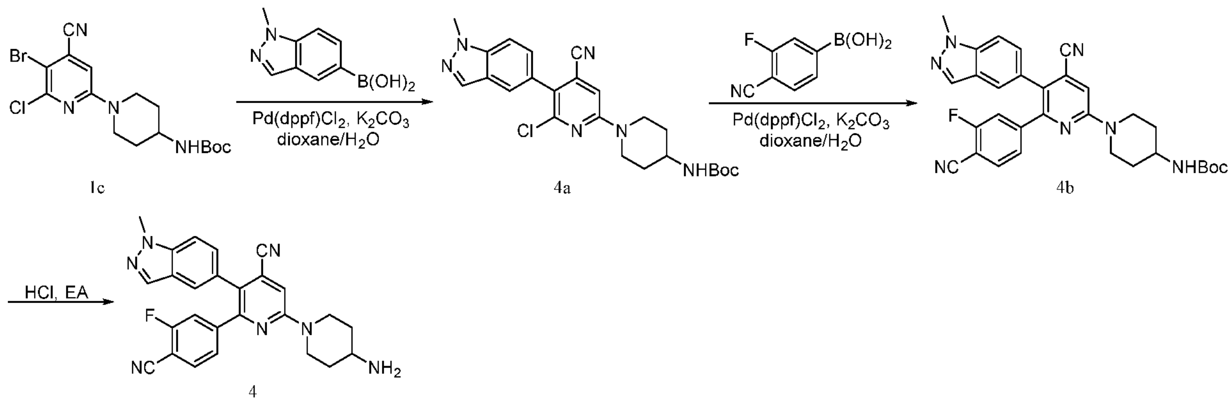


First step: preparation of tert-butyl (1-(6-chloro-4-cyano-5-(1-methyl-1*H*-indazol-5-yl)pyrid-2-yl)piperid-4-yl)carbamate (4a)

A compound 1c (130 mg, 0.31 mmol), 1-methylindazole-5-boric acid (56 mg, 0.32 mmol) and $K_2CO_3$ (64 mg, 0.46 mmol) were dissolved in 1,4-dioxane (3 mL) and water (0.6 mL), replacement with nitrogen was carried out, Pd(dppf)$Cl_2$ (22 mg, 0.03 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 120 mg of a title compound, a yield being 82%.

Second step: preparation of tert-butyl (1-(4-cyano-6-(4-cyano-3-fluorophenyl)-5-(1-methyl-1H-indazol-5-yl)pyrid-2-yl) piperid-4-yl)carbatnate (4b)

The compound 4a (50 mg, 0.11 mmol), 3-fluoro-4-cyanophenylboronic acid (28 mg, 0.17 mmol) and $K_2CO_3$ (46 mg, 0.33 mmol) were added into a reaction flask and dissolved in 1,4-dioxane (1 mL) and water (0.2 mL), replacement with nitrogen was carried out, Pd(dppf)$Cl_2$ (8 mg, 0.01 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 20 mg of a title compound, a yield being 34%.

Third step: preparation of 6-(4-aminopiperid-1-yl)-2-(4-cyano-3-fluorophenyl)-3-(1-methyl-1*H*-indazol-5-yl)isonicotinonitrile (4)

The compound 4b (20 mg, 0.04 mmol) was dissolved in ethyl acetate (0.5 mL), then a hydrochloric acid-ethyl

acetate solution (0.5 mL, 3M) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 15 mg of a title compound, a yield being 92%.

LC-MS (ESI) m/z $(M+H)^+$: 452.2
$^1H$ NMR (400 MHz, DMSO-$d_6$) δ 8.06 (d, $J$ = 0.8 Hz, 1H), 7.75-7.70 (m, 1H), 7.69-7.67 (m, 1H), 7.63-7.59 (m, 1H), 7.57 (s, 1H), 7.38 (dd, $J$ = 10.8, 1.2 Hz, 1H), 7.18-7.12 (m, 2H), 4.38-4.28 (m, 2H), 4.05 (s, 3H), 3.10-3.00 (m, 2H), 2.91-2.81 (m, 1H), 1.85-1.74 (m, 2H), 1.26-1.16 (m, 2H).

**Example** 5 Preparation of 6-(4-amino-3 -hydroxylpiperid-1 -yl)-2-(4-cyano-3 -fhiorophenyl)-3-(3 -fhioro-4-methoxyphenyl)isonicotin onitrile (5)

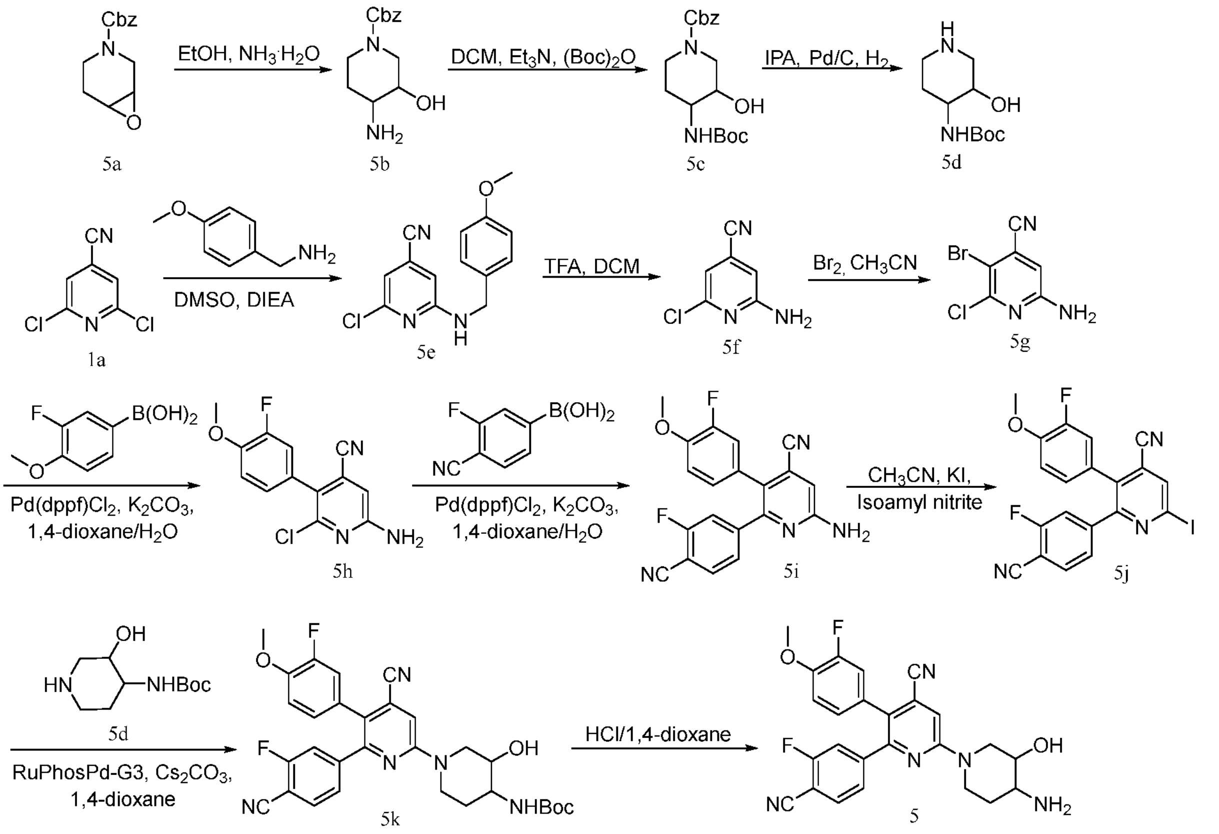


First step: preparation of benzyl 4-amino-3-hydroxylpiperidine-1-carboxylate (5b)

A compound 5a (1.0 g, 4.29 mmol) was dissolved in ethanol (5 mL), then ammonium hydroxide (5 mL) was added, and a reaction system was sealed and then heated to 70°C and stirred to react overnight. The reaction system was cooled to the room temperature, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was directly used for the next step of reaction without purification.

Second step: preparation of benzyl 4-((tert-butoxycarbonyl)amino)-3-hydroxylpiperidine-1-carboxylate (5c)

The compound 5b (700 mg, 2.80 mmol) was dissolved in dichloromethane (10 mL), triethylamine (424 mg, 4.19 mmol) and di-tert-butyl dicarbonate (610 mg, 2.80 mmol) were added into a system in sequence under an ice bath, and a

reaction system was placed at the room temperature and stirred to react overnight. Water was added into the system to quench the reaction, extraction was carried out 3 times with dichloromethane, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 500 mg of a title compound, a total yield in the two steps being 33%.

Third step: preparation of tert-butyl (3-hydroxylpiperid-4-yl)carbamate (5d)

The compound 5c (500 mg, 1.43 mmol) was dissolved in isopropanol (5 mL), then Pd/C (50 mg, 10%) was added, air in a system was replaced with hydrogen, and then the system was stirred at the room temperature under a hydrogen atmosphere to react overnight for 4 hours. A reaction system was filtered and subjected to vacuum concentration, and a crude product was purified using silica gel column chromatography to obtain 200 mg of a title compound, a yield being 65%.

Fourth step: preparation of 2-chloro-6-((4-methoxybenzyl)amino)isonicotinonitrile (5e)

A compound 1a (7.0 g, 40.46 mmol) was dissolved in DMSO (70 mL), then DIEA (5.3 g, 41.09 mmol) and p-methoxybenzylamine (5.7 g, 41.55 mmol) were added in sequence, and a reaction system was placed at 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was directly used for the next step without purification.

Fifth step: preparation of 2-amino-6-chloroisonicotinonitrile (5f)

The compound 5e (9.0 g, 32.88 mmol) was dissolved in dichloromethane (50 mL), then trifluoroacetic acid (50 mL) was added, and a reaction system was placed at the room temperature and stirred to react for 6 hours. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and an obtained crude product was purified using a thin layer silica gel plate to obtain 5.0 g of a title compound, a total yield in the two steps being 81%.

Sixth step: preparation of 6-amino-3-bromo-2-chloroisonicotinonitrile (5g)

The compound 5f (5.0 g, 32.56 mmol) was dissolved in acetonitrile (50 mL), bromine (5.3 g, 33.13 mmol) was slowly added dropwise into a system under an ice bath, and a reaction system was placed at the room temperature and stirred to react for 6 hours. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 4.0 g of a title compound, a yield being 53%.

Seventh step: preparation of 6-amino-2-chloro-3-(3-fluoro-4-methoxyphenyl)isonicotinonitrile (5h)

The compound 5g (2.0 g, 8.60 mmol) was dissolved in 1,4-dioxane (20 mL) and water (4 mL), then 3-fluoro-4-methoxyphenylboronic acid (1.5 g, 8.83 mmol) and $K_2CO_3$ (3.6 g, 26.05 mmol) were added in sequence, replacement with nitrogen was carried out, Pd(dppf)$Cl_2$ (628 mg, 0.86 mmol) was added, replacement with nitrogen was carried out again, and a reaction system was placed at 80°C and stirred to react for 6 hours. The reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 1.5 g of a title compound, a yield being 63%.

Eighth step: preparation of 6-atnino-2-(4-cyano-3-fluorophenyl)-3-(3-fluoro-4-methoxyphenyl)isonicotinonitrile (5i)

The compound 5h (1.5 g, 5.40 mmol) was dissolved in 1,4-dioxane (15 mL) and water (3 mL), then 3-fluoro-4-cyanophenylboronic acid (2.7 g, 16.37 mmol) and $K_2CO_3$ (2.3 g, 16.64 mmol) were added in sequence, replacement with

nitrogen was carried out, $Pd(dppf)Cl_2$ (395 mg, 0.54 mmol) was added, replacement with nitrogen was carried out again, and a reaction system was placed at 80°C and stirred to react for 9 hours. The reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 1.5 g of a title compound, a yield being 77%.

Ninth step: preparation of 2-(4-cyano-3-fluorophenyl)-3-(3-fluoro-4-methoxyphenyl)-6-iodoisonicotinonitrile (5j)

The compound 5i (1.5 g, 4.14 mmol) was dissolved in acetonitrile (15 mL), then potassium iodide (4.1 g, 24.70 mmol) and isoamyl nitrite (2.9 g, 24.75 mmol) were added in sequence, and a reaction system was placed at 75°C and stirred to react for 36 hours. The reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 700 mg of a title compound, a yield being 36%.

Tenth step: preparation of tert-butyl (1-(4-cyano-6-(4-cyano-3-fluorophenyl)-5-(3-fluoro-4-methoxyphenyl)pyrid-2-yl)-3-hydroxylpiperid-4-y 1)carbamate (5k)

The compound 5j (50 mg, 0.11 mmol) was dissolved in 1,4-dioxane (3 mL), then the compound 5d (48 mg, 0.22 mmol) and $Cs_2CO_3$ (108 mg, 0.33 mmol) were added in sequence, replacement with nitrogen was carried out, RuPhos-Pd-G3 (9 mg, 0.01 mmol) was added, replacement with nitrogen was carried out again, and a reaction system was heated to 80°C and stirred to react for 3 hours. The reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 20 mg of a title compound, a yield being 34%.

Eleventh step: preparation of 6-(4-amino-3 -hydroxylpiperid-1 -yl)-2-(4-cyano-3 -fhiorophenyl)-3-(3 -fhioro-4-methoxyphenyl)isonicotin onitrile (5)

The compound 5k (20 mg, 0.04 mmol) was dissolved in a 1,4-dioxane solution (2 mL), then a hydrochloric acid-1,4-dioxane solution (2 mL, 4M in 1,4-dioxane) was added, and a reaction system was placed at the room temperature and stirred to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 8 mg of a title compound, a yield being 49%.
LC-MS (ESI) m/z $(M+H)^+$: 462.2

**Example** 6 Preparation of 6-(4-(aminomethyl)-4-hydroxylpiperid-1-yl)-2-(4-cyano-3-fluorophenyl)-3-(3-fluoro-4-methoxyphenyl)is onicotinonitrile (6)

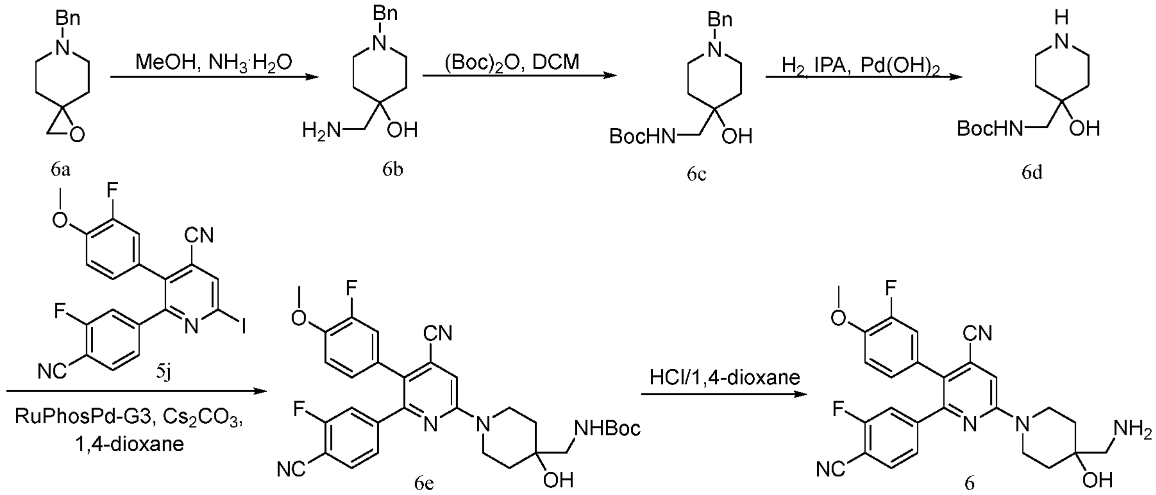


6a 6b 6c 6d 5j 6e 6

First step: preparation of 4-(aminomethyl)-1-benzylpiperidine-4-ol (6b)

A compound 6-1 (500 mg, 2.46 mmol) was dissolved in methanol (2 mL), then ammonium hydroxide (5 mL) was added, and a system was sealed and then placed at the room temperature and stirred to react for 4 hours. A reaction system was cooled to the room temperature, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was directly used for the next step of reaction without purification.

Second step: preparation of tert-butyl ((1-benzyl-4-hydroxylpiperid-4-yl)methyl)carbamate (6c)

The compound 6b (500 mg, 2.27 mmol) was dissolved in dichloromethane (10 mL), di-tert-butyl dicarbonate (495 mg, 2.27 mmol) was added into a system under an ice bath condition, and a reaction system was placed at the room temperature and stirred to react overnight. Water was added into the system to quench the reaction, extraction was carried out 3 times with dichloromethane, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 600 mg of a title compound, a total yield in the two steps being 76%.

Third step: preparation of tert-butyl ((4-hydroxylpiperid-4-yl)methyl)carbamate (6d)

The compound 6c (300 mg, 0.94 mmol) was dissolved in isopropanol (5 mL), then palladium hydroxide (30 mg, 10%) was added at the room temperature, air in a system was replaced with hydrogen, and then the system was placed at the room temperature under a hydrogen atmosphere and stirred overnight. A reaction system was filtered, a filtrate was subjected to vacuum concentration, and a crude product was purified using silica gel column chromatography to obtain 100 mg of a title compound, a yield being 46%.

Fourth step: preparation of tert-butyl ((1-(4-cyano-6-(4-cyano-3-fluorophenyl)-5-(3-fluoro-4-methoxyphenyl)pyrid-2-yl)-4-hydroxylpiperid-4-yl)methyl)carbamate (6e)

The compound 5j (50 mg, 0.11 mmol) was dissolved in 1,4-dioxane (3 mL), then the compound 6d (51 mg, 0.22 mmol) and $Cs_2CO_3$ (108 mg, 0.33 mmol) were added in sequence, replacement with nitrogen was carried out, RuPhos-Pd-G3 (9 mg, 0.01 mmol) was added, replacement with nitrogen was carried out again, and a reaction system was placed at 80°C and stirred to react for 3 hours. The reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 22 mg of a title compound, a yield being 36%.

Fifth step: preparation of 6-(4-(aminomethyl)-4-hydroxylpiperid-1-yl)-2-(4-cyano-3-fluorophenyl)-3-(3-fluoro-4-methoxyphenyl)is onicotinonitrile (6)

The compound 6e (22 mg, 0.04 mmol) was dissolved in a 1,4-dioxane solution (2 mL), then a hydrochloric acid-1,4-dioxane solution (2 mL, 4M in 1,4-dioxane) was added, and a reaction system was placed at the room temperature and stirred to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 10 mg of a title compound, a yield being 55%.

LC-MS (ESI) m/z $(M+H)^+$: 476.2
$^1$H NMR (400 MHz, DMSO-$d_6$) δ 7.86-7.77 (m, 1H), 7.60-7.53 (m, 1H), 7.43-7.36 (m, 1H), 7.23-7.10 (m, 3H), 7.00-6.92 (m, 1H), 4.60 (brs, 1H), 4.21-4.07 (m, 2H), 3.85 (s, 3H), 3.52-3.36 (m, 2H), 2.54 (s, 2H), 1.58-1.39 (m, 4H).

**Example** 7 Preparation of 6-(3-aminoazetidin-1-yl)-2-(4-cyano-3-fluorophenyl)-3-(3-fluoro-4-methoxyphenyl)isonicotinonitrile (7)

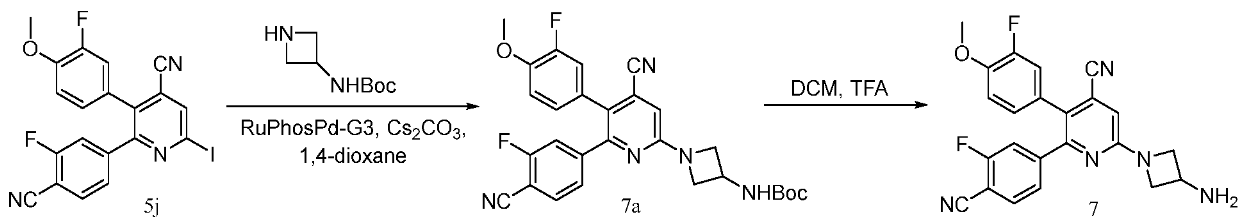


First step: preparation of tert-butyl (1-(4-cyano-6-(4-cyano-3 -fluorophenyl)-5 -(3 -fluoro-4-methoxyphenyl)pyrid-2-yl) azetidin-3 -yl)carbamat e (7a)

A compound 5j (50 mg, 0.11 mmol) was dissolved in 1,4-dioxane (3 mL), then tert-butyl azetidin-3-ylcarbamate (38 mg, 0.22 mmol) and $Cs_2CO_3$ (108 mg, 0.33 mmol) were added in sequence, replacement with nitrogen was carried out, RuPhos-Pd-G3 (9 mg, 0.01 mmol) was added, replacement with nitrogen was carried out again, and a reaction system was placed at 80°C and stirred to react for 3 hours. The reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 22 mg of a title compound, a yield being 40%.

Second step: preparation of 6-(3-atninoazetidin-1-yl)-2-(4-cyano-3-fluorophenyl)-3-(3-fluoro-4-methoxyphenyl)isonicotinonitrile (7)

The compound 7a (22 mg, 0.04 mmol) was dissolved in dichloromethane (2 mL), then trifluoroacetic acid (1 mL) was added, and a reaction system was placed at the room temperature and stirred to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 15 mg of a title compound, a yield being 85%.

LC-MS (ESI) m/z $(M+H)^+$: 418.2
$^1$H NMR (400 MHz, DMSO-$d_6$) δ 7.81 (dd, *J* = 8.0, 6.8 Hz, 1H), 7.37 (dd, *J* = 10.4, 1.2 Hz, 1H), 7.19-7.10 (m, 3H), 7.09 (s, 1H), 6.97-6.91 (m, 1H), 4.27-4.19 (m, 2H), 3.89-3.81 (m, 4H), 3.70-3.63 (m, 2H).

**Example** 8 Preparation of 4-(4-(4-aminopiperid-1-yl)-7-(1-methyl-1*H*-indazol-5-yl)-3-oxo-2,3-dihydro-1*H*-pyrrole[3,4-*c*] pyrid-6-yl )-2-fluorobenzonitrile (8)

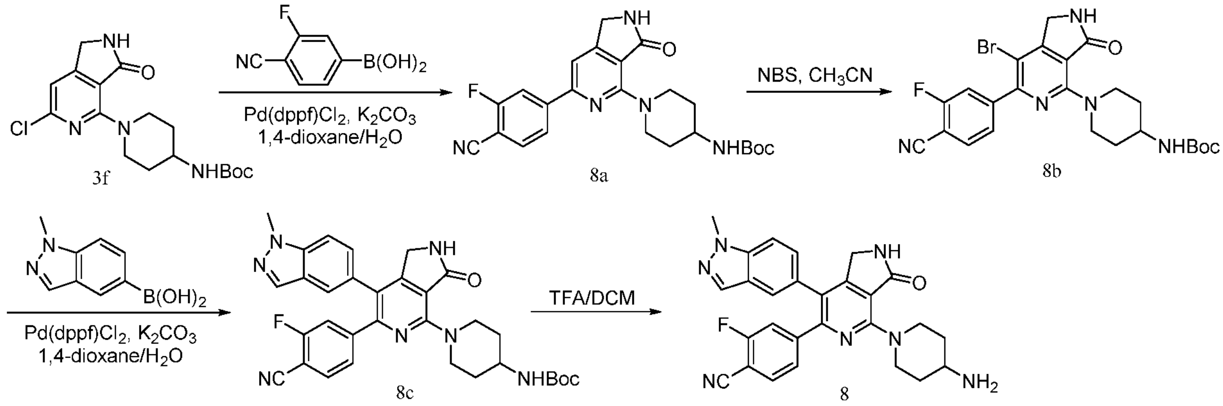

3f 8a 8b 8c 8

First step: preparation of tert-butyl (1-(6-(4-cyano-3-fluorophenyl)-3-oxo-2,3-dihydro-1*H*pyrrole[3,4-*c*]pyrid-4-yl)piperid-4-yl)carbamate (8a)

A compound 3f (287 mg, 0.78 mmol), 3-fluoro-4-cyanophenylboronic acid (193 mg, 1.17 mmol) and $K_2CO_3$ (647 mg, 4.68 mmol) were dissolved in 1,4-dioxane (4 mL) and water (0.4 mL), replacement with nitrogen was carried out, Pd(dppf)$Cl_2$ (29 mg, 0.04 mmol) was added, replacement with nitrogen was carried out again, and a reaction system was placed at 100°C and stirred to react for 2 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 315 mg of a title compound, a yield being 89%.

Second step: preparation of tert-butyl (1-(7-bromo-6-(4-cyano-3-fluorophenyl)-3-oxo-2,3-dihydro-1*H*pyrrolo[3,4-*c*]pyrid-4-yl)piperid-4-yl)carb amate (8b)

The compound 8a (310 mg, 0.69 mmol) was dissolved in acetonitrile (12 mL), NBS (246 mg, 1.38 mmol) was slowly added into a system under an ice bath condition, and a reaction system was stirred to react for 1 hour with the ice bath condition maintained. A reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 364 mg of a title compound, a yield being 100%.

Third step: preparation of tert-butyl (1-(6-(4-cyano-3-fluorophenyl)-7-(1-methyl-1*H*-indazol-5-yl)-3-oxo-2,3-dihydro-1*H*-pyrrole[3,4-*c*]pyrid-4-yl)piperid-4-yl)carbamate (8c)

The compound 8b (70 mg, 0.13 mmol), 1-methylindazole-5-boric acid (35 mg, 0.20 mmol) and $K_2CO_3$ (108 mg, 0.78 mmol) were dissolved in 1,4-dioxane (4 mL) and water (0.4 mL), replacement with nitrogen was carried out, Pd(dppf) $Cl_2$ (15 mg, 0.02 mmol) was added, replacement with nitrogen was carried out again, and a reaction system was placed at 100°C and stirred to react for 1 hour. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 57 mg of a title compound, a yield being 74%.

Fourth step: Preparation of 4-(4-(4-aminopiperid-1-yl)-7-(1-methyl-1*H*-indazol-5-yl)-3-oxo-2,3-dihydro-1*H*-pyrrole [3,4-*c*]pyrid-6-yl )-2-fluorobenzonitrile (8)

The compound 8c (57 mg, 0.10 mmol) was dissolved in dichloromethane (3 mL), then trifluoroacetic acid (3 mL) was added at 0°C, and a reaction system was maintained at 0°C to be stirred to react for 2 hours. The reaction system was

subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 15 mg of a title compound, a yield being 32%.

LC-MS (ESI) m/z $(M+H)^+$: 482.2

$^1$H NMR (400 MHz, DMSO- $d_6$) δ 8.60 (s, 1H), 8.02 (s, 1H), 7.77-7.70 (m, 1H), 7.68 (s, 1H), 7.62 (d, J = 8.4 Hz, 1H), 7.49 (d, J = 10.4 Hz, 1H), 7.20 (d, J = 8.0 Hz, 1H), 7.16 (d, J = 8.4 Hz, 1H), 4.61-4.51 (m, 2H), 4.15 (s, 2H), 4.06 (s, 3H), 3.30-3.17 (m, 1H), 3.07-2.95 (m, 2H), 2.04-1.92 (m, 2H), 1.72-1.58 (m, 2H).

**Example** 9 Preparation of 4-(4-(4-aminopiperid-1-yl)-7-(6-fluoro-1-(2-hydroxyl-2-methylpropyl)-1*H*-indazol-5-yl)-3-oxo-2,3-dihyd ro-1*H*-pyrrole[3,4-*c*]pyrid-6-yl)-2-fluorobenzonitrile (9)

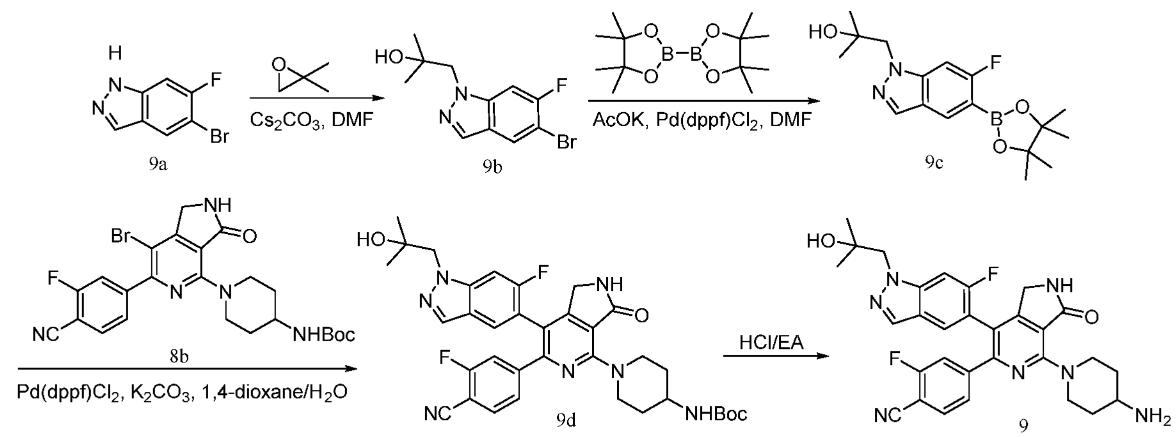


First step: preparation of 1-(5-bromo-6-fluoro-1*H*-indazol-1-yl)-2-methylpropane-2-ol (9b)

A compound 9a (430 mg, 2.00 mmol) was dissolved in *N,N*-dimethylformamide (10 mL), then $Cs_2CO_3$ (2.0 g, 6.14 mmol) and 1,1-dimethyl ethylene oxide (216 mg, 3.00 mmol) were added in sequence, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 488 mg of a title compound, a yield being 85%.

Second step: preparation of 1-(6-fluoro-5-(4,4,5,5-tetramethyl-1,3,2-dioxybenzaldehyde-2-yl)-1*H*-indazol-1-yl)-2-methylpropane-2-o 1 (9c)

The compound 9b (286 mg, 1.00 mmol), bis(pinacolato)diboron (381 mg, 1.50 mmol) and potassium acetate (196 mg, 0.10 mmol) were dissolved in *N,N*-dimethylformamide (5 mL), replacement with nitrogen was carried out, triphenylphosphine (52 mg, 0.20 mmol) and $Pd(AcO)_2$ (23 mg, 0.10 mmol) were added in sequence, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 296 mg of a title compound, a yield being 89%.

Third step: preparation of tert-butyl (1-(6-(4-cyano-3-fluorophenyl)-7-(6-fluoro-1-(2-hydroxyl-2-methylpropyl)-1*H*-indazol-5-yl)-3-oxo-2,3-d ihydro-17/pyrrolo [3,4-c]pyrid-4-yl)piperid-4-yl)carbamate (9d)

A compound 8b (110 mg, 0.21 mmol), the compound 9c (107 mg, 0.32 mmol) and $K_2CO_3$ (175 mg, 1.27 mmol) were dissolved in 1,4-dioxane (2 mL) and water (0.2 mL), replacement with nitrogen was carried out, Pd(dppf)$Cl_2$ (15 mg,

0.02 mmol) was added, replacement with nitrogen was carried out again, and a reaction system was heated to 100°C and stirred to react for 5 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 12 mg of a title compound, a yield being 9%.

Fourth step: preparation of 4-(4-(4-aminopiperid-1-yl)-7-(6-fluoro-1-(2-hydroxyl-2-methylpropyl)-1*H*-indazol-5-yl)-3-oxo-2,3-dihyd ro-1*H*-pyrrole[3,4-*c*]pyrid-6-yl)-2-fluorobenzonitrile (9)

The compound 9d (12 mg, 0.02 mmol) was dissolved in ethyl acetate (1 mL), then a hydrochloric acid-ethyl acetate solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 6 mg of a title compound, a yield being 59%.

LC-MS (ESI) m/z $(M+H)^+$: 558.2
$^1$H NMR (400 MHz, DMSO- $d_6$) δ 8.55 (s, 1H), 8.08 (s, 1H), 7.86 (d, J = 7.2 Hz, 1H), 7.80-7.72 (m, 1H), 7.54 (d, J = 10.8 Hz, 1H), 7.44 (d, J = 10.4 Hz, 1H), 7.27 (d, J = 8.4 Hz, 1H), 4.65 (s, 1H), 4.55-4.42 (m, 2H), 4.40-4.22 (m, 3H), 3.96-3.87 (m, 1H), 3.11-2.98 (m, 2H), 2.87-2.75 (m, 1H), 1.89-1.75 (m, 2H), 1.44-1.32 (m, 2H), 1.16 (s, 3H), 1.10 (s, 3H).

Example 10 Preparation of 6-(4-aminopiperid-1-yl)-2-(4-cyano-3-fluorophenyl)-3-(1-methyl-1*H*-indazol-5-yl)isonicotinamide (10)

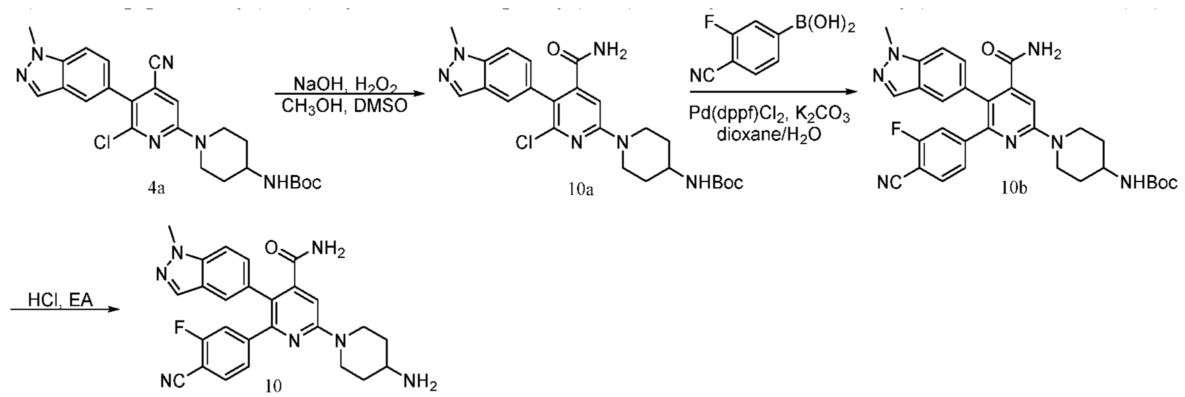


First step: preparation of tert-butyl (1-(4-carbamoyl-6-chloro-5-(1-methyl-1*H*-indazol-5-yl)pyrid-2-yl)piperid-4-yl)carbamate (10a)

A compound 4a (190 mg, 0.41 mmol) was dissolved in a methanol solution (4 mL), then dimethyl sulfoxide (2 mL), NaOH (49 mg, 1.23 mmol) and $H_2O_2$ (232 mg, 2.05 mmol, 30% aqueous solution) were added in sequence, and a reaction system was placed at 50°C and stirred to react for 2 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 126 mg of a title compound, a yield being 64%.

Second step: preparation of tert-butyl (1-(4-carbamoyl-6-(4-cyano-3-fluorophenyl)-5-(1-methyl-1*H*-indazol-5-yl)pyrid-2-yl)piperid-4-yl)carba mate (10b)

The compound 10a (100 mg, 0.21 mmol), 3-fluoro-4-cyanophenylboronic acid (53 mg, 0.32 mmol) and $K_2CO_3$ (87 mg, 0.63 mmol) were dissolved in 1,4-dioxane (4 mL) and water (0.4 mL), replacement with nitrogen was carried out,

$Pd(dppf)Cl_2$ (15 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 95 mg of a title compound, a yield being 81%.

Third step: preparation of 6-(4-aminopiperid-1-yl)-2-(4-cyano-3-fluorophenyl)-3-(1-methyl-1*H*-indazol-5-yl)isonicotinamide (10)

The compound 10b (95 mg, 0.17 mmol) was dissolved in ethyl acetate (2 mL), then a hydrochloric acid-ethyl acetate solution (2 mL, 3M in EA) was added, and a reaction system was placed at the room temperature and stirred to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 31 mg of a title compound, a yield being 40%.

LC-MS (ESI) m/z $(M+H)^+$: 470.2
$^1H$ NMR (400 MHz, DMSO-$d_6$) δ 7.98 (d, *J*= 0.4 Hz, 1H), 7.73-7.64 (m, 2H), 7.51-7.46 (m, 2H), 7.34-7.31 (m, 1H), 7.31 (dd, J= 9.2, 1.2 Hz, 1H), 7.10 (dd, J= 8.0, 1.2 Hz, 1H), 7.10 (dd, J= 8.4, 1.6 Hz, 1H), 6.91 (s, 1H), 4.35-4.25 (m, 2H), 4.02 (s, 3H), 3.03-2.93 (m, 2H), 2.89-2.79 (m, 1H), 1.85-1.74 (m, 2H), 1.29-1.16 (m, 2H).

**Example** 11 Preparation of 2-(4-aminopiperid-1-yl)-6-(4-cyano-3-fluorophenyl)-5-(1-methyl-1*H*-indazol-5-yl)nicotinamide (11)

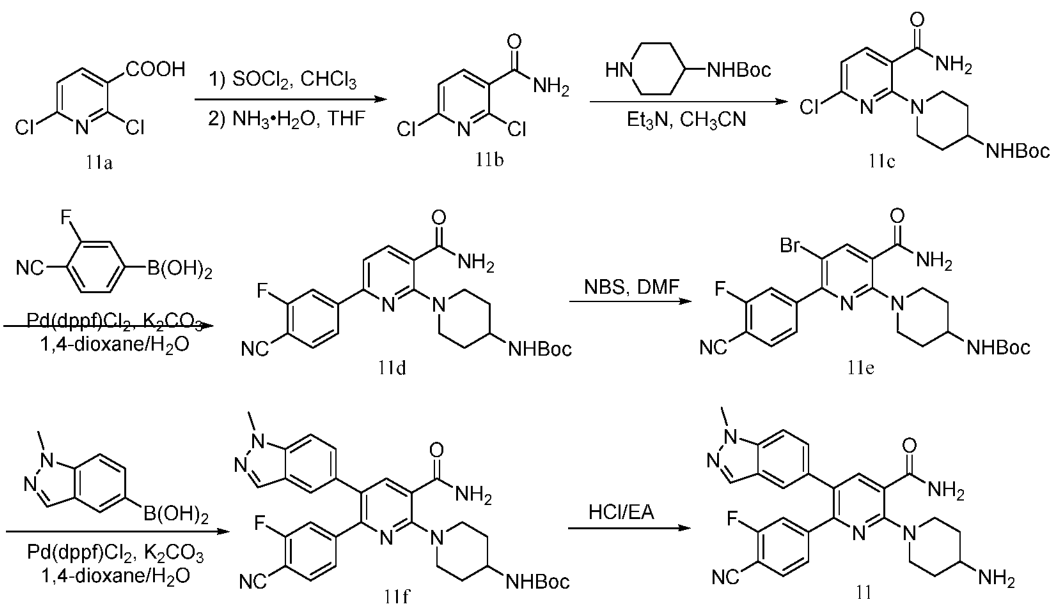


First step: preparation of 2,6-dichloronicotinamide (11b)

A compound 11a (3.0 g, 15.63 mmol) was dissolved in chloroform (30 mL), thionyl chloride (18.6 g, 156.30 mmol) was slowly added into a system under an ice bath, and the system was heated to 70°C and stirred to react for 3 hours. A reaction system was subjected to vacuum concentration and dissolved by tetrahydrofuran (10 mL), then was added dropwise into an ice-bath-cooled solution of ammonium hydroxide (30 mL) in tetrahydrofuran (10 mL), and was stirred to react for 30 minutes. Water was added into the reaction system, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified

using silica gel column chromatography to obtain 2.8 g of a title compound, a yield being 94%.

Second step: preparation of tert-butyl (1-(3-carbamoyl-6-chloropyrid-2-yl)piperid-4-yl)carbamate (11c)

The compound 11b (2.8 g, 14.66 mmol), 4-tert-butoxycarbonyl aminopiperidine (4.9 g, 24.47 mmol) and triethylamine (3.0 g, 29.65 mmol) were dissolved in acetonitrile (30 mL), and a system was heated to 70°C and stirred to react for 3 hours. A reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 4.4 g of a title compound, a yield being 88%.

Third step: preparation of tert-butyl (1-(3-carbatnoyl-6-(4-cyano-3-fluorophenyl)pyrid-2-yl)piperid-4-yl)carbamate (11d)

The compound 11c (400 mg, 1.13 mmol), 3-fluoro-4-cyanophenylboronic acid (280 mg, 1.70 mmol) and $K_2CO_3$ (937 mg, 6.78 mmol) were dissolved in 1,4-dioxane (5 mL) and water (0.5 mL), replacement with nitrogen was carried out, Pd(dppf)$Cl_2$ (41 mg, 0.06 mmol) was added, replacement with nitrogen was carried out again, and a reaction system was heated to 100 °C and stirred to react for 4 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 448 mg of a title compound, a yield being 90%.

Fourth step: preparation of tert-butyl (1-(5-bromo-3-carbamoyl-6-(4-cyano-3-fluorophenyl)pyrid-2-yl)piperid-4-yl)carbamate (11e)

The compound 11d (448 mg, 1.02 mmol) was dissolved in *N,N*-dimethylformamide (5 mL), NBS (200 mg, 1.12 mmol) was slowly added into a system under an ice bath condition, and a reaction system was maintained at 0°C to be stirred to react for 2 hours. A reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 528 mg of a title compound, a yield being 100%.

Fifth step: preparation of tert-butyl (1-(3-carbatnoyl-6-(4-cyano-3-fluorophenyl)-5-(1-methyl-1H-indazol-5-yl)pyrid-2-yl) piperid-4-yl)carba mate (11f)

The compound 11e (518 mg, 1.00 mmol), 1-methylindazole-5-boric acid (263 mg, 1.49 mmol) and $K_2CO_3$ (829 mg, 6.00 mmol) were dissolved in 1,4-dioxane (6 mL) and water (0.6 mL), replacement with nitrogen was carried out, Pd(dppf)$Cl_2$ (37 mg, 0.05 mmol) was added, replacement with nitrogen was carried out again, and a reaction system was heated to 100°C and stirred to react for 3 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a silica gel column to obtain 423 mg of a title compound, a yield being 74%.

Sixth step: preparation of 2-(4-aminopiperid-1-yl)-6-(4-cyano-3-fluorophenyl)-5-(1-methyl-1*H*-indazol-5-yl)nicotinamide (11)

The compound 11f (30 mg, 0.05 mmol) was dissolved in ethyl acetate (1 mL), then a hydrochloric acid-ethyl acetate solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 6 mg of a title compound, a yield being 24%.

LC-MS (ESI) m/z $(M+H)^+$: 470.2

$^1H$ NMR (400 MHz, DMSO-$d_6$) $\delta$ 8.02 (s, 1H), 8.00-7.95 (m, 1H), 7.79 (s, 1H), 7.79-7.73 (m, 1H), 7.68-7.62 (m, 2H),

7.58-7.54 (m, 1H), 7.50-7.45 (m, 1H), 7.22 (dd, *J* = 8.4, 1.6 Hz, 1H), 7.22 (dd, *J* = 8.8, 1.6 Hz, 1H), 4.04 (s, 3H), 3.91-3.80 (m, 2H), 3.02-2.90 (m, 2H), 2.84-2.73 (m, 1H), 1.86-1.74 (m, 2H), 1.43-1.32 (m, 2H).

Example 12 Preparation of 2-(4-atninopiperid-1-yl)-6-(4-cyano-3-fluorophenyl)-5-(1-methyl-1H-indazol-5-yl)nicotinonitrile (12)

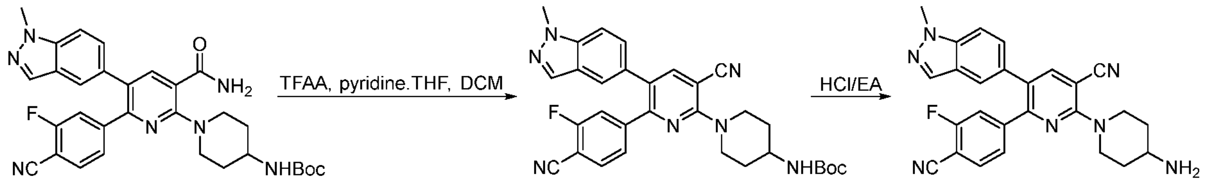


11f 12a 12

First step: preparation of tert-butyl (1-(3-cyano-6-(4-cyano-3-fluorophenyl)-5-(1-methyl-1*H*-indazol-5-yl)pyrid-2-yl)piperid-4-yl)carbamate (12a)

A compound 11f (200 mg, 0.35 mmol) was dissolved in tetrahydrofuran (6 mL), replacement with nitrogen was carried out, pyridine (194 mg, 2.45 mmol) and a solution of trifluoroacetic anhydride (147 mg, 0.70 mmol) in dichloromethane (1 mL) were added in sequence under an ice bath, and a reaction system slowly restored to the room temperature and was stirred to react overnight. A reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 10 mg of a title compound, a yield being 5%.

Second step: preparation of 2-(4-aminopiperid-1-yl)-6-(4-cyano-3-fluorophenyl)-5-(1-methyl-1*H*-indazol-5-yl)nicotinonitrile (12)

The compound 12a (10 mg, 0.02 mmol) was dissolved in ethyl acetate (1 mL), then a hydrochloric acid-ethyl acetate solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 1 mg of a title compound, a yield being 12%.
LC-MS (ESI) m/z $(M+H)^+$: 452.2

**Example** 13 Preparation of 6-(4-amino-3-fluoropiperid-1-yl)-2-(4-cyano-3-fluorophenyl)-3-(3-fluoro-4-methoxyphenyl) isonicotinoni trile (13)

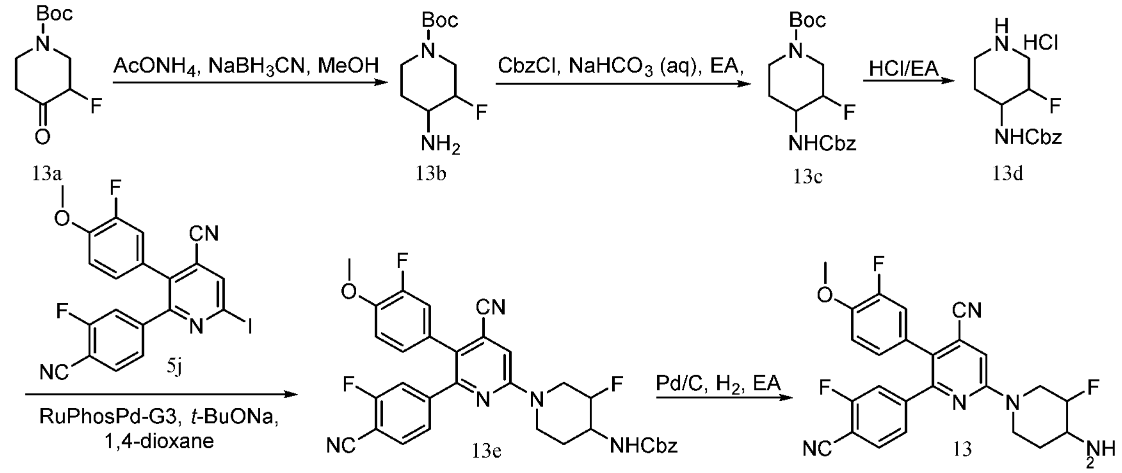


13a 13b 13c 13d 5j 13e 13

First step: preparation of tert-butyl 4-amino-3-fluoropiperidine-1-carboxylate (13b)

A compound 13-1 (1.0 g, 4.60 mmol) was dissolved in methanol (10 mL), ammonium acetate (2.5 g, 32.43 mmol) and sodium cyanoborohydride (375 mg, 5.97 mmol) were added in sequence, and a reaction system was placed at the room temperature and stirred to react overnight. Water was added into the reaction system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 500 mg of a title compound, a yield being 50%.

Second step: preparation of tert-butyl 4-(((benzyloxy)carbonyl)amino)-3-fluoropiperidine-1-carboxylate (13c)

The compound 13b (500 mg, 2.29 mmol) was dissolved in ethyl acetate (5 mL), then a saturated sodium bicarbonate solution (10 mL) was added, benzyl chloroformate (391 mg, 2.29 mmol) was added into a system under an ice bath condition, and a reaction system was placed at the room temperature and stirred to react overnight. A reaction liquid was extracted 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 420 mg of a title compound, a yield being 52%.

Third step: preparation of (3-fluoropiperid-4-yl)benzyl carbamate hydrochloride (13d)

The compound 13c (200 mg, 0.57 mmol) was dissolved in ethyl acetate (10 mL), then a hydrochloric acid-ethyl acetate solution (4 mL, 3M in EA) was added, and a reaction system was stirred at the room temperature to react for 1 hour. The reaction system was subjected to vacuum concentration, and a crude product was directly used for the next step without purification.

Fourth step: preparation of benzyl (1-(4-cyano-6-(4-cyano-3-fluorophenyl)-5-(3-fluoro-4-methoxyphenyl)pyrid-2-yl)-3-fluoropiperid-4-yl)c arbamate (13e)

A compound 5j (50 mg, 0.11 mmol) was dissolved in 1,4-dioxane (3 mL), then the compound 13d (64 mg, 0.22 mmol) and t-BuONa (53 mg, 0.55 mmol) were added in sequence, replacement with nitrogen was carried out, RuPhos-Pd-G3 (9 mg, 0.01 mmol) was added, replacement with nitrogen was carried out again, and a reaction system was placed at 80°C and stirred to react for 3 hours. The reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 21 mg of a title compound, a yield being 33%.

Fifth step: preparation of 6-(4-amino-3-fluoropiperid-1-yl)-2-(4-cyano-3-fluorophenyl)-3-(3-fluoro-4-methoxyphenyl)isonicotinoni trile (13)

The compound 13e (21 mg, 0.04 mmol) was dissolved in ethyl acetate (2 mL), then Pd/C (2 mg, 10%) was added at the room temperature, air in a system was replaced with hydrogen, and then the system was heated to 35°C under a hydrogen atmosphere and stirred to react for 4 hours. A reaction system was filtered and subjected to vacuum concentration, and a crude product was purified using a thin layer silica gel plate to obtain 10 mg of a title compound, a yield being 61%.

LC-MS (ESI) m/z $(M+H)^+$: 464.2
$^1H$ NMR (400 MHz, DMSO-$d_6$) δ 7.95-7.78 (m, 1H), 7.64 (s, 1H), 7.41 (dd, *J* = 10.8, 1.6 Hz, 1H), 7.23-7.11 (m, 3H), 7.00-6.94 (m, 1H), 4.22-4.15 (m, 2H), 4.10-4.00 (m, 1H), 3.85 (s, 3H), 3.45-3.33 (m, 2H), 3.03-2.92 (m, 1H), 1.93-1.82 (m, 1H), 1.41-1.30 (m, 1H).

**Example 14** Preparation of 6-(4-aminocyclohexyl)-2-(4-cyano-3-fluorophenyl)-3-(3-fluoro-4-methoxyphenyl)isonicotinonitrile (14)

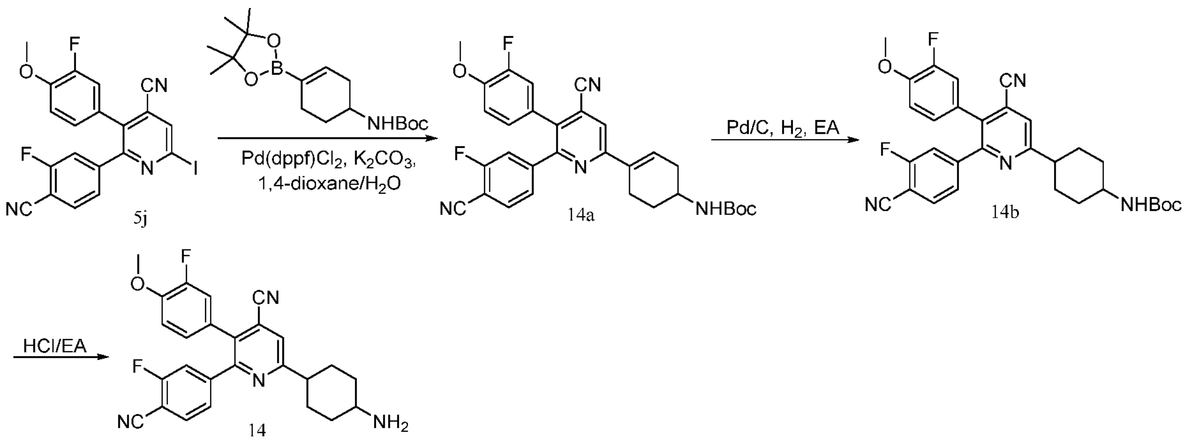


First step: preparation of tert-butyl (4-(4-cyano-6-(4-cyano-3-fluorophenyl)-5-(3-fluoro-4-methoxyphenyl)pyrid-2-yl)cyclohexyl-3-alken-1-y 1)carbamate (14a)

A compound 5j (80 mg, 0.17 mmol) was dissolved in 1,4-dioxane (3 mL) and water (0.5 mL), tert-butyl (4-(4,4,5,5-tetramethyl-1,3,2-dioxaborolane-2-yl)cyclohexyl-3-alken-1-yl)carbamate (165 mg, 0.51 mmol) and $K_2CO_3$ (70 mg, 0.51 mmol) were added in sequence, replacement with nitrogen was carried out, Pd(dppf)$Cl_2$ (15 mg, 0.02 mmol) was added, replacement with nitrogen was carried out again, and a system was heated to 80°C and stirred to react for 2 hours. A reaction system was cooled to the room temperature, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 79 mg of a title compound, a yield being 86%.

Second step: preparation of tert-butyl (4-(4-cyano-6-(4-cyano-3-fluorophenyl)-5-(3-fluoro-4-methoxyphenyl)pyrid-2-yl) cyclohexyl)carbamate (14b)

The compound 14a (40 mg, 0.07 mmol) was dissolved in ethyl acetate (2 mL), then Pd/C (4 mg, 10%) was added at the room temperature, air in a system was replaced with hydrogen, and then the system was stirred at the room temperature under a hydrogen atmosphere to react overnight. A reaction system was filtered, a filtrate was subjected to vacuum concentration, and a crude product was purified using a thin layer silica gel plate to obtain 23 mg of a title compound, a yield being 57%.

Third step: preparation of 6-(4-aminocyclohexyl)-2-(4-cyano-3-fluorophenyl)-3-(3-fluoro-4-methoxyphenyl)isonicotinonitrile (14)

The compound 14b (23 mg, 0.04 mmol) was dissolved in ethyl acetate (5 mL), then a hydrochloric acid-ethyl acetate solution (4 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 11 mg of a title compound, a yield being 59%.

LC-MS (ESI) m/z $(M+H)^+$: 445.2
$^1$H NMR (400 MHz, DMSO-$d_6$) δ 8.08 (s, 0.48H), 8.01 (s, 0.47H), 7.89-7.82 (m, 1H), 7.48-7.41 (m, 1H), 7.55-7.28 (m, 1H), 7.25-7.16 (m, 2H), 7.11-7.05 (m, 1H), 3.88-3.84 (m, 3H), 3.16-3.10 (m, 0.5H), 2.95-2.86 (m, 0.5H), 2.86-2.68 (m, 1H), 2.15-1.89 (m, 3H), 1.75-1.60 (m, 4H), 1.33-1.21 (m, 1H).

**Example 15** Preparation of 2-(4-cyano-3-fluorophenyl)-6-(1,4-diazacyclohept-1-yl)-3-(3-fluoro-4-methoxyphenyl)isonicotinonitrile (15)

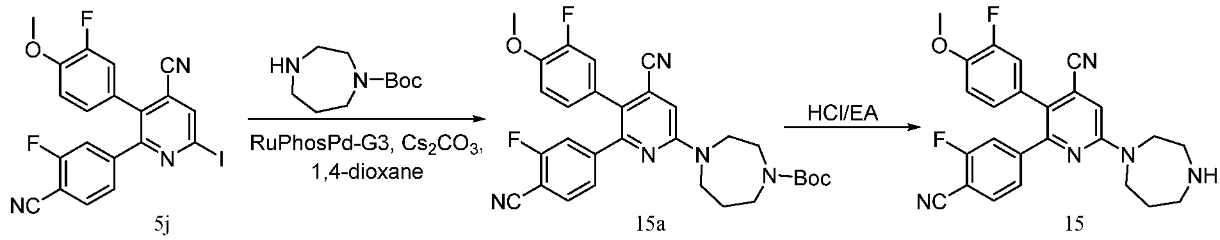


First step: preparation of tert-butyl 4-(4-cyano-6-(4-cyano-3-fluorophenyl)-5-(3-fluoro-4-methoxyphenyl)pyrid-2-yl)-1,4-diazacycloheptane-1-carboxylate (15a)

A compound 5j (50 mg, 0.11 mmol) was dissolved in 1,4-dioxane (3 mL), tert-butyl 1,4-diazacycloheptane-1-carboxylate (44 mg, 0.22 mmol) and $Cs_2CO_3$ (108 mg, 0.33 mmol) were added in sequence, replacement with nitrogen was carried out, RuPhos-Pd-G3 (9 mg, 0.01 mmol) was added, replacement with nitrogen was carried out again, and a system was heated to 80°C and stirred to react for 3 hours. A reaction system was cooled to the room temperature, vacuum concentration was carried out to remove a solvent, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 24 mg of a title compound, a yield being 42%.

Second step: preparation of 2-(4-cyano-3-fluorophenyl)-6-(1,4-diazacyclohept-1-yl)-3-(3-fluoro-4-methoxyphenyl)isonicotinonitrile (15)

The compound 15a (24 mg, 0.04 mmol) was dissolved in ethyl acetate (5 mL), then a hydrochloric acid-ethyl acetate solution (4 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 10 mg of a title compound, a yield being 51%.

LC-MS (ESI) m/z $(M+H)^+$: 446.2
$^1$H NMR (400 MHz, DMSO- $d_6$) δ 7.85-7.79 (m, 1H), 7.42-7.34 (m, 2H), 7.23-7.11 (m, 3H), 7.00-6.93 (m, 1H), 3.85 (s, 3H), 3.80-3.68 (m, 4H), 2.93-2.86 (m, 2H), 2.76-2.69 (m, 2H), 1.84-1.73 (m, 2H).

**Example 16** Preparation of 6-(4-atninopiperid-1-yl)-2-(4-cyano-3-fluorophenyl)-3-(6-fluoro-1-(2-hydroxyl-2-methylpropyl)-1H-inda zol-5-yl)isonicotinonitrile (16)

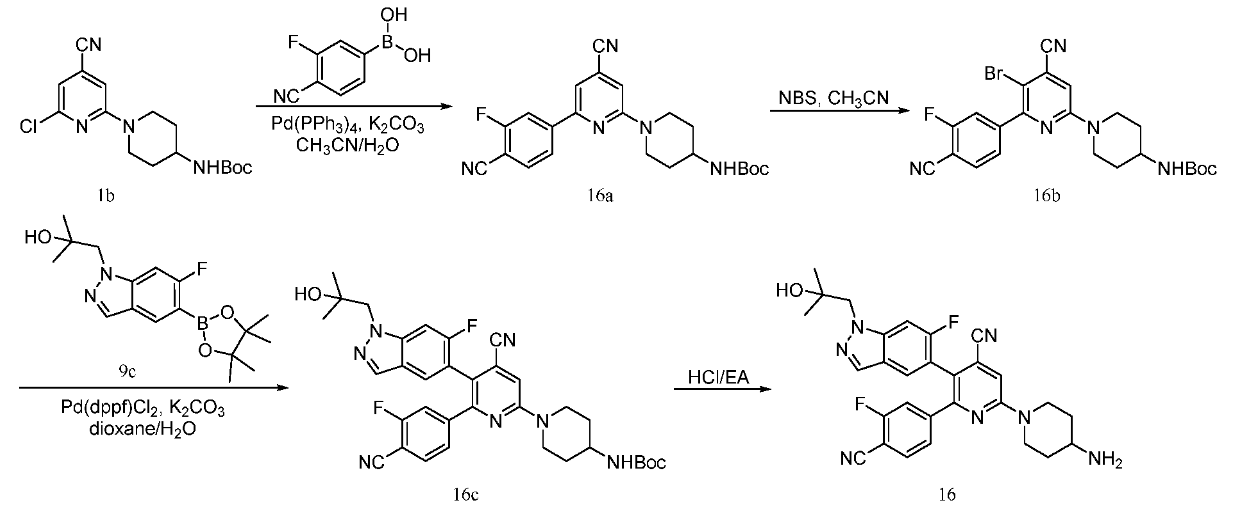


1b 16a 16b 9c 16c 16

First step: preparation of tert-butyl (1-(4-cyano-6-(4-cyano-3-fluorophenyl)pyrid-2-yl)piperid-4-yl)carbamate (16a)

A compound 1b (1.7 g, 5.05 mmol), 3-fluoro-4-cyanophenylboronic acid (1.2 g, 7.28 mmol) and $K_2CO_3$ (2.1 g, 15.20 mmol) were dissolved in acetonitrile (20 mL) and water (10 mL), replacement with nitrogen was carried out, $Pd(PPh_3)_4$ (289 mg, 0.25 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 1.8 g of a title compound, a yield being 85%.

Second step: preparation of tert-butyl (1-(5-bromo-4-cyano-6-(4-cyano-3-fluorophenyl)pyrid-2-yl)piperid-4-yl)carbatnate (16b)

The compound 16a (421 mg, 1.00 mmol) was dissolved in acetonitrile (15 mL), NBS (182 mg, 1.02 mmol) was slowly added into a system under an ice bath, and a reaction system was placed at the room temperature and stirred to react for 3 hours. The reaction system was subjected to vacuum concentration, and a crude product was purified using silica gel column chromatography to obtain 450 mg of a title compound, a yield being 90%.

Third step: preparation of tert-butyl (1-(4-cyano-6-(4-cyano-3-fluorophenyl)-5-(6-fluoro-1-(2-hydroxyl-2-methylpropyl)-1*H*-indazol-5-yl)pyri d-2-yl)piperid-4-yl)carbamate (16c)

The compound 16b (100 mg, 0.20 mmol), a compound 9c (100 mg, 0.30 mmol) and $K_2CO_3$ (83 mg, 0.60 mmol) were dissolved in 1,4-dioxane (2 mL) and water (0.2 mL), replacement with nitrogen was carried out, $Pd(dppf)Cl_2$ (15 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 30 mg of a title compound, a yield being 24%.

Fourth step: preparation of 6-(4-aminopiperid-1-yl)-2-(4-cyano-3-fluorophenyl)-3-(6-fluoro-1-(2-hydroxyl-2-methylpropyl)-1*H*-inda zol-5-yl)isonicotinonitrile (16)

The compound 16c (30 mg, 0.05 mmol) was dissolved in ethyl acetate (1 mL), then a hydrochloric acid-ethyl acetate solution (1 mL, 3M in EA) was added, and a reaction system was stirred at the room temperature to react for 1 hour.

The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 24 mg of a title compound, a yield being 95%.

LC-MS (ESI) m/z $(M+H)^+$: 528.2

$^1H$ NMR (400 MHz, DMSO-$d_6$) δ 8.11 (s, 1H), 7.80-7.72 (m, 2H), 7.61 (s, 1H), 7.57 (d, *J* = 10.8 Hz, 1H), 7.40-7.34 (m, 1H), 7.18 (dd, J= 8.0, 1.2 Hz, 1H), 4.68 (s, 1H), 4.40-4.24 (m, 4H), 3.12-3.01 (m, 2H), 2.92-2.82 (m, 1H), 1.85-1.75 (m, 2H), 1.27-1.19 (m, 2H), 1.14 (s, 3H), 1.10 (s, 3H).

**Example 17** Preparation of 6-(4-atninopiperid-1-yl)-2-(4-cyano-3-fluorophenyl)-3-(6-fluoro-1-(2-hydroxyl-2-methylpropyl)-1H-inda zol-5-yl)isonicotinamide (17)

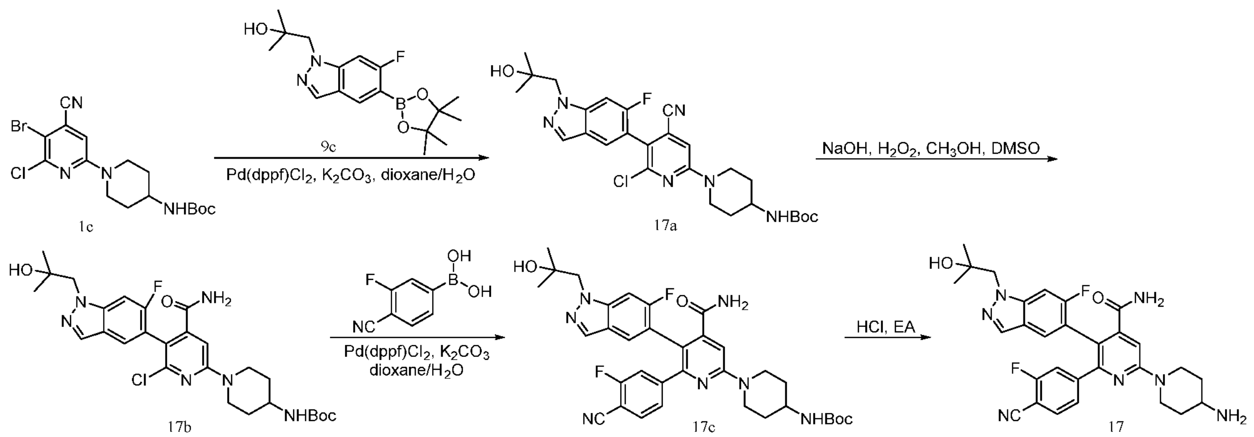


1c, 17a, 17b, 17c, 17

First step: preparation of tert-butyl (1-(6-chloro-4-cyano-5-(6-fluoro-1-(2-hydroxyl-2-methylpropyl)-1*H*-indazol-5-yl)pyrid-2-yl)piperid-4-yl )carbamate (17a)

A compound 1c (229 mg, 0.55 mmol), a compound 9c (368 mg, 1.10 mmol) and $K_2CO_3$ (228 mg, 1.65 mmol) were dissolved in 1,4-dioxane (5 mL) and water (1 mL), replacement with nitrogen was carried out, Pd(dppf)$Cl_2$ (44 mg, 0.06 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 138 mg of a title compound, a yield being 46%.

Second step: preparation of tert-butyl (1-(4-carbatnoyl-6-chloro-5-(6-fluoro-1-(2-hydroxyl-2-methylpropyl)-1H-indazol-5-yl)pyrid-2-yl)piperid -4-yl)carbamate (17b)

The compound 17a (138 mg, 0.25 mmol) was dissolved in a methanol solution (1 mL), then dimethyl sulfoxide (1 mL), NaOH (30 mg, 0.75 mmol) and $H_2O_2$ (170 mg, 1.50 mmol, 30% aqueous solution) were added in sequence, and a reaction system was placed at 50°C and stirred to react for 2 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 126 mg of a title compound, a yield being 88%.

Third step: preparation of tert-butyl (1-(4-carbamoyl-6-(4-cyano-3-fluorophenyl)-5-(6-fluoro-1-(2-hydroxyl-2-methylpropyl)-1*H*-indazol-5-yl )pyrid-2-yl)piperid-4-yl)carbamate (17c)

The compound 17b (126 mg, 0.22 mmol), 3-fluoro-4-cyanophenylboronic acid (54 mg, 0.33 mmol) and $K_2CO_3$

(91 mg, 0.66 mmol) were dissolved in 1,4-dioxane (2 mL) and water (0.4 mL), replacement with nitrogen was carried out, $Pd(dppf)Cl_2$ (15 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 74 mg of a title compound, a yield being 51%.

Fourth step: preparation of 6-(4-aminopiperid-1-yl)-2-(4-cyano-3-fluorophenyl)-3-(6-fluoro-1-(2-hydroxyl-2-methylpropyl)-1*H*-inda zol-5-yl)isonicotinamide (17)

The compound 17c (74 mg, 0.11 mmol) was dissolved in ethyl acetate (1 mL), then a hydrochloric acid-ethyl acetate solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 24 mg of a title compound, a yield being 38%.

LC-MS (ESI) m/z $(M+H)^+$: 546.2
$^1$H NMR (400 MHz, DMSO-$d_6$) $\delta$ 8.03 (d, *J* = 0.8, 1H), 7.85 (s, 1H), 7.25-7.67 (m, 1H), 7.59 (d, *J* = 7.2, 1H), 7.42-7.34 (m, 2H), 7.27 (dd, *J* = 10.4, 1.2 Hz, 1H), 7.16 (dd, *J* = 8.0, 1.6 Hz, 1H), 6.96 (s, 1H), 4.66 (s, 1H), 4.37-4.19 (m, 4H), 3.04-2.94 (m, 2H), 2.88-2.48 (m, 1H), 1.85-1.75 (m, 2H), 1.27-1.19 (m, 2H), 1.13 (s, 3H), 1.07 (s, 3H).

**Example 18** Preparation of 6-(4-atninopiperid-1-yl)-2-(4-cyano-3-fluorophenyl)-3-(1-methyl-2-oxoindol-5-yl)isonicotinonitrile (18)

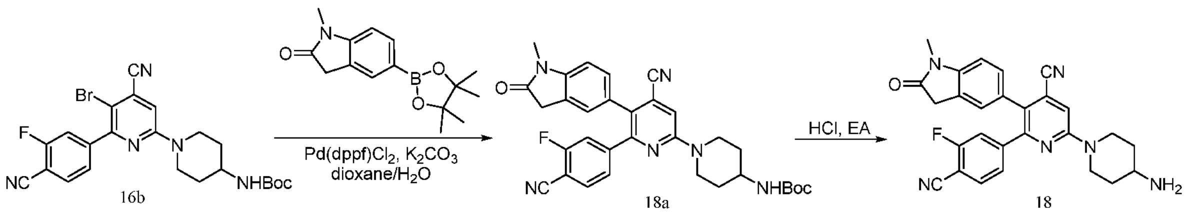


First step: preparation of tert-butyl (1-(4-cyano-6-(4-cyano-3-fluorophenyl)-5-(1-methyl-2-oxoindol-5-yl)pyrid-2-yl)piperid-4-yl)carbamate (18a)

A compound 18b (75 mg, 0.15 mmol), 1-methyl-5-(4,4,5,5-tetramethyl-1,3,2-dioxaborolane-2-yl)indoline-2-one (63 mg, 0.23 mmol) and $K_2CO_3$ (62 mg, 0.46 mmol) were dissolved in 1,4-dioxane (1 mL) and water (0.2 mL), replacement with nitrogen was carried out, $Pd(dppf)Cl_2$ (15 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 36 mg of a title compound, a yield being 42%.

Second step: preparation of 6-(4-atninopiperid-1-yl)-2-(4-cyano-3-fluorophenyl)-3-(1-methyl-2-oxoindol-5-yl)isonicotinonitrile (18)

The compound 18a (36 mg, 0.06 mmol) was dissolved in ethyl acetate (1 mL), then a hydrochloric acid-ethyl acetate solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product

was purified using a thin layer silica gel plate to obtain 18 mg of a title compound, a yield being 61%.

LC-MS (ESI) m/z $(M+H)^+$: 467.2
$^1H$ NMR (400 MHz, DMSO-$d_6$) δ 7.77 (dd, $J$ = 8.0, 7.2 Hz, 1H), 7.53 (s, 1H), 7.39 (dd, $J$ = 10.8, 1.2 Hz, 1H), 7.19-7.12 (m, 2H), 7.09-7.07 (m, 1H), 7.00-6.96 (m, 1H), 4.34-4.26 (m, 2H), 3.51 (s, 2H), 3.12 (s, 3H), 3.08-2.98 (m, 2H), 2.89-2.80 (m, 1H), 1.82-1.74 (m, 2H), 1.26-1.17 (m, 2H).

**Example 19** Preparation of 6-(4-atninopiperid-1-yl)-2-(4-cyano-3-fluorophenyl)-3-(2-fluoro-4-(2-hydroxyl-2-methylpropyl)phenyl)is onicotinonitrile (19)

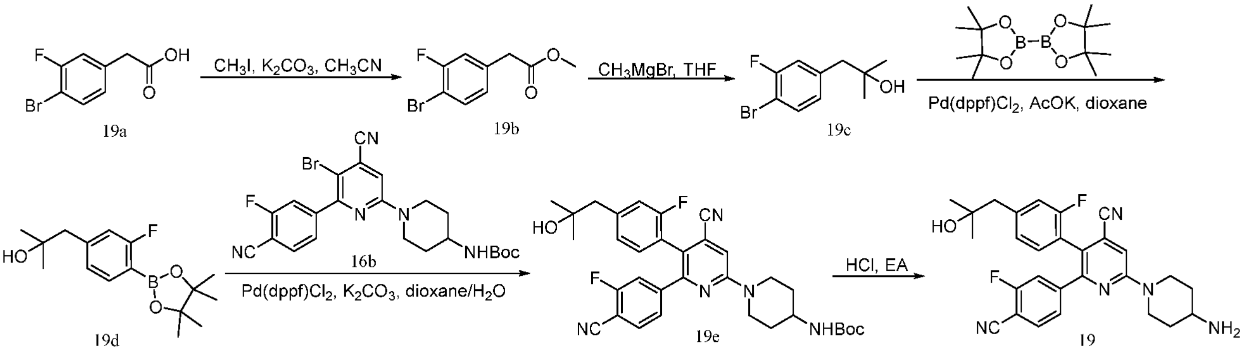


First step: preparation of methyl 2-(4-bromo-3-fluorophenyl)acetate (19b)

A compound 19a (1.0 g, 4.29 mmol) was dissolved in acetonitrile (40 mL), then $K_2CO_3$ (1.8 g, 13.02 mmol) and iodomethane (1.5 g, 10.57 mmol) were added in sequence, and a reaction system was heated to 60°C and stirred to react for 3 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 1.0 g of a title compound, a yield being 94%.

Second step: preparation of 1-(4-bromo-3-fluorophenyl)-2-methylpropane-2-ol (19c)

The compound 19b (815 mg, 3.30 mmol) was dissolved in dry tetrahydrofuran (30 mL), then methyl magnesium bromide (5.5 mL, 3M in THF) was slowly added dropwise under an ice bath, and after dropwise adding was finished, the temperature of a reaction system slowly returned to the room temperature, and the reaction system was stirred to react overnight. The reaction system was cooled to 0°C, an appropriate amount of water was slowly added to quench the reaction, filtering was carried out, a filtrate was extracted 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 366 mg of a title compound, a yield being 45%.

Third step: preparation of 1-(3-fluoro-4-(4,4,5,5-tetramethyl-1,3,2-dioxybenzaldehyde-2-yl)phenyl)-2-methylpropane-2-ol (19d)

The compound 19c (360 mg, 1.46 mmol), bis(pinacolato)diboron (741 mg, 2.92 mmol) and potassium acetate (429 mg, 4.37 mmol) were dissolved in 1,4-dioxane (15 mL), replacement with nitrogen was carried out, Pd(dppf)$Cl_2$ (107 mg, 0.15 mmol) was added, replacement with nitrogen was carried out again, and a reaction system was heated to 100°C and stirred to react for 24 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 177 mg of a title compound, a yield being 41%.

Fourth step: preparation of tert-butyl (1-(4-cyano-6-(4-cyano-3-fluorophenyl)-5-(2-fluoro-4-(2-hydroxyl-2-methylpropyl) phenyl)pyrid-2-yl)pip erid-4-yl)carbamate (19e)

Acompound 16b (100 mg, 0.20 mmol), the compound 19d (118 mg, 0.40 mmol) and $K_2CO_3$ (83 mg, 0.60 mmol) were dissolved in 1,4-dioxane (2 mL) and water (0.2 mL), replacement with nitrogen was carried out, Pd(dppf)$Cl_2$ (15 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 50 mg of a title compound, a yield being 43%.

Fifth step: preparation of 6-(4-atninopiperid-1-yl)-2-(4-cyano-3-fluorophenyl)-3-(2-fluoro-4-(2-hydroxyl-2-methylpropyl) phenyl)is onicotinonitrile (19)

The compound 19e (50 mg, 0.09 mmol) was dissolved in ethyl acetate (1 mL), then a hydrochloric acid-ethyl acetate solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 36 mg of a title compound, which was a yellow solid, a yield being 87%.

LC-MS (ESI) m/z $(M+H)^+$: 488.2
$^1$H NMR (400 MHz, DMSO-$d_6$) $\delta$ 7.82-7.76 (m, 1H), 7.59 (s, 1H), 7.27-7.17 (m, 3H), 7.08 (s, 1H), 7.07-7.04 (m, 1H), 4.56 (s, 1H), 4.37-4.27 (m, 2H), 3.10-3.00 (m, 2H), 2.91-2.80 (m, 1H), 2.68 (s, 2H), 1.85-1.74 (m, 2H), 1.27-1.17 (m, 2H), 1.04 (s, 3H), 1.03 (s, 3H).

Example **20** Preparation of 2-(4-cyano-3-fluorophenyl)-6-(1,4-diazaniline-1-yl)-3-(1-methyl-1*H*-indazol-5-yl)isonicotinonitrile (20)

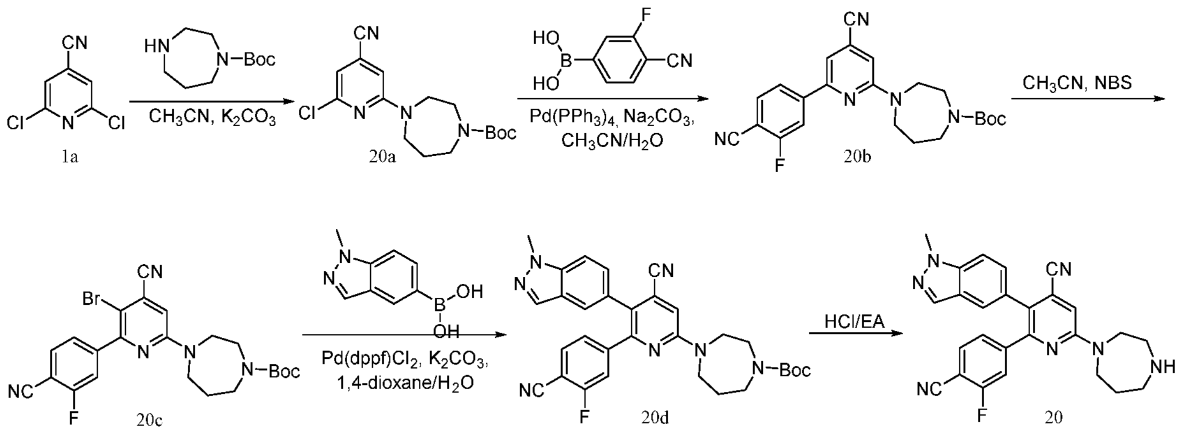


First step: preparation of tert-butyl 4-(6-chloro-4-cyanopyrid-2-yl)-1,4-diazane-1-carboxylate (20a)

A compound 1a (1.5 g, 8.67 mmol), tert-butyl 1,4-diazacycloheptane-1-formate (1.7 g, 8.49 mmol), and $K_2CO_3$ (2.4 g, 17.36 mmol) were dissolved in acetonitrile (20 mL), and this system was heated to 80°C and stirred to react overnight. A reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 2.1 g of a title compound, a yield being 72%.

Second step: preparation of tert-butyl 4-(4-cyano-6-(4-cyano-3-fluorophenyl)pyrid-2-yl)-1,4-diazane-1-carboxylate (20b)

The compound 20a (1.0 g, 2.97 mmol), 3-fluoro-4-cyanophenylboronic acid (735 mg, 4.46 mmol) and $Na_2CO_3$ (630 mg, 5.94 mmol) were dissolved in acetonitrile (10 mL) and water (5 mL), replacement with nitrogen was carried out, $Pd(PPh_3)_4$ (173 mg, 0.15 mmol) was added, replacement with nitrogen was carried out again, and a reaction system was heated to 85°C and stirred to react for 6 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 800 mg of a title compound, a yield being 64%.

Third step: preparation of tert-butyl 4-(5-bromo-4-cyano-6-(4-cyano-3-fluorophenyl)pyrid-2-yl)-1,4-diazane-1-carboxylate (20c)

The compound 20b (500 mg, 1.19 mmol) was dissolved in acetonitrile (5 mL), NBS (211 mg, 1.19 mmol) was slowly added into a system under an ice bath, and a reaction system was placed at the room temperature and stirred to react for 3 hours. The reaction system was subjected to vacuum concentration, and a crude product was purified using silica gel column chromatography to obtain 420 mg of a title compound, a yield being 71%.

Fourth step: preparation of tert-butyl 4-(4-cyano-6-(4-cyano-3-fluorophenyl)-5-(1-methyl-1H-indazol-5-yl)pyrid-2-yl)-1,4-diazane-1-carboxyla te (20d)

The compound 20c (50 mg, 0.10 mmol), 1-methylindazole-5-boric acid (53 mg, 0.30 mmol) and $K_2CO_3$ (41 mg, 0.30 mmol) were dissolved in 1,4-dioxane (2 mL) and water (0.4 mL), replacement with nitrogen was carried out, $Pd(dppf)Cl_2$ (7 mg, 0.01 mmol) was added, replacement with nitrogen was carried out again, and a reaction system was heated to 80°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 30 mg of a title compound, a yield being 54%.

Fifth step: preparation of 2-(4-cyano-3-fluorophenyl)-6-(1,4-diazaniline-1-yl)-3-(1-methyl-1H-indazol-5-yl)isonicotinonitrile (20)

The compound 20d (30 mg, 0.05 mmol) was dissolved in ethyl acetate (1 mL), then a hydrochloric acid-ethyl acetate solution (2 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 20 mg of a title compound, a yield being 82%.

LC-MS (ESI) m/z $(M+H)^+$: 452.2
$^1H$ NMR (400 MHz, DMSO-$d_6$) δ 8.07-8.05 (m, 1H), 7.75-7.70 (m, 1H), 7.69-7.66 (m, 1H), 7.63-7.59 (m, 1H), 7.40-7.35 (m, 2H), 7.18-7.13 (m, 2H), 4.06 (s, 3H), 3.83-3.67 (m, 4H), 2.93-2.87 (m, 2H), 2.76-2.69 (m, 2H), 1.85-1.74 (m, 2H).

**Example 21** Preparation of 2-(4-cyano-3-fluorophenyl)-6-(1,4-diazaniline-1-yl)-(6-fluoro-1-(2-hydroxyl-2-methylpropyl)-1H-indazol -5-yl)isonicotinonitrile (21)

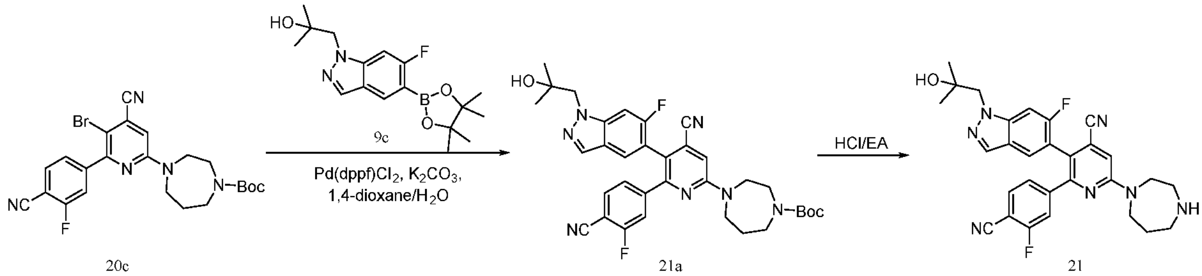


20c 21a 21

First step: preparation of tert-butyl 4-(4-cyano-6-(4-cyano-3-fluorophenyl)-5-(6-fluoro-1-(2-hydroxyl-2-methylpropyl)-1*H*-indazol-5-yl)pyri d-2-yl)-1,4-diazane-1-carboxylate (21a)

A compound 20c (50 mg, 0.10 mmol), a compound 9c (100 mg, 0.30 mmol) and $K_2CO_3$ (41 mg, 0.30 mmol) were dissolved in 1,4-dioxane (2 mL) and water (0.4 mL), replacement with nitrogen was carried out, $Pd(dppf)Cl_2$ (7 mg, 0.01 mmol) was added, replacement with nitrogen was carried out again, and a reaction system was heated to 80°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 10 mg of a title compound, a yield being 16%.

Second step: preparation of 2-(4-cyano-3-fluorophenyl)-6-(1,4-diazaniline-1-yl)-3-(6-fluoro-1-(2-hydroxyl-2-methylpropyl)-1*H*-indaz ol-5-yl)isonicotinonitrile (21)

The compound 21a (10 mg, 0.02 mmol) was dissolved in ethyl acetate (1 mL), then a hydrochloric acid-ethyl acetate solution (2 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 4 mg of a title compound, a yield being 48%.

LC-MS (ESI) m/z $(M+H)^+$: 528.2
1H NMR (400 MHz, DMSO- d6) δ 8.11 (s, 1H), 7.79-7.73 (m, 2H), 7.58 (d, J = 10.4 Hz, 1H), 7.41 (s, 1H), 7.39-7.33 (m, 1H), 7.21-7.16 (m, 1H), 4.67 (s, 1H), 4.32-4.23 (m, 2H), 3.88-3.67 (m, 4H), 2.94-2.85 (m, 2H), 2.76-2.68 (m, 2H), 1.86-1.72 (m, 2H), 1.15 (s, 3H), 1.10 (s, 3H).

**Example 22** Preparation of 2-(4-cyano-3-fluorophenyl)-3-(3-fluoro-4-methoxyphenyl)-6-(2,7-diazaspiro[3.5]non-7-yl) isonicotinonitr ile (22)

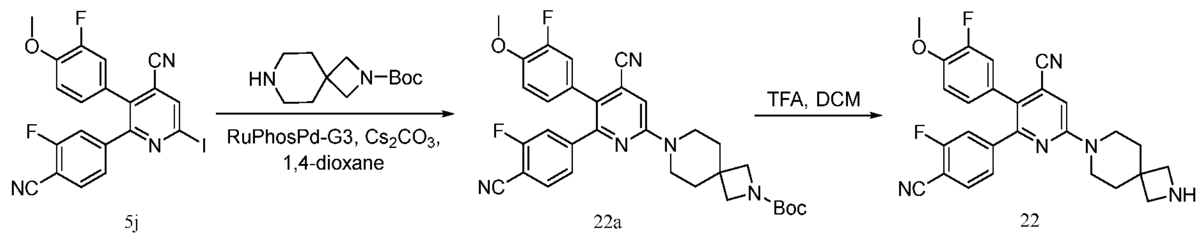


5j 22a 22

First step: preparation of tert-butyl 7-(4-cyano-6-(4-cyano-3-fluorophenyl)-5-(3-fluoro-4-methoxyphenyl)pyrid-2-yl)-2,7-diazaspiro[3.5]non ane-2-carboxylate (22a)

A compound 5j (50 mg, 0.11 mmol) was dissolved in 1,4-dioxane (3 mL), then 2-tert-butoxycarbonyl-2,7-diazaspiro[3.5]nonane (50 mg, 0.22 mmol) and $Cs_2CO_3$ (107 mg, 0.33 mmol) were added in sequence, replacement with nitrogen was carried out, RuPhos-Pd-G3 (9 mg, 0.01 mmol) was added, replacement with nitrogen was carried out again,

and this system was heated to 80°C and stirred to react for 3 hours. A reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 30 mg of a title compound, a yield being 50%.

Second step: preparation of 2-(4-cyano-3-fluorophenyl)-3-(3-fluoro-4-methoxyphenyl)-6-(2,7-diazaspiro[3.5]non-7-yl) isonicotinonitr ile (22)

The compound 22a (30 mg, 0.05 mmol) was dissolved in dichloromethane (2 mL), then trifluoroacetic acid (1 mL) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 15 mg of a title compound, a yield being 61%.

LC-MS (ESI) m/z $(M+H)^+$: 472.2
$^1H$ NMR (400 MHz, DMSO-$d_6$) δ 7.85-7.78 (m, 1H), 7.59 (s, 1H), 7.44-7.36 (m, 1H), 7.22-7.11 (m, 3H), 6.99-6.93 (m, 1H), 3.85 (s, 3H), 3.72-3.56 (m, 8H), 1.85-1.67 (m, 4H).

**Example 23** Preparation of 6-(4-aminocyclohexyl)-2-(4-cyano-3-fluorophenyl)-3-(1-methyl-1*H*-indazol-5-yl)isonicotinonitrile (23-1, 23-2)

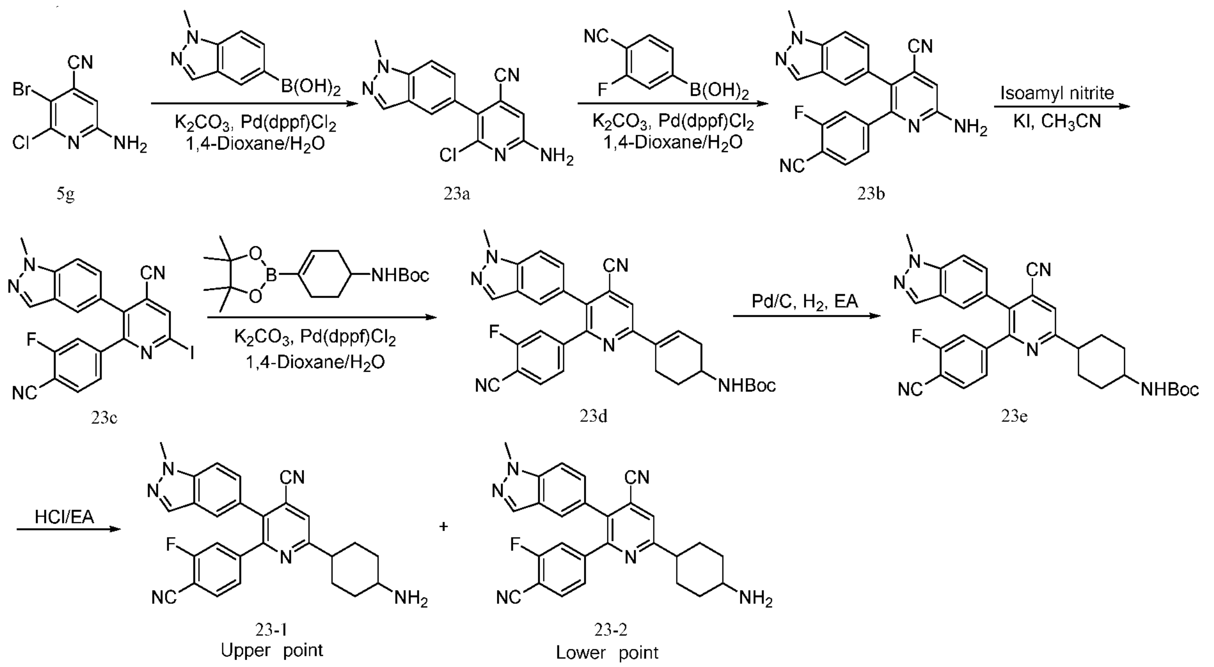


First step: preparation of 6-atnino-2-chloro-3-(1-methyl-1*H*-indazol-5-yl)isonicotinonitrile (23a)

A compound 5g (1.5 g, 6.45 mmol) was dissolved in 1,4-dioxane (20 mL) and water (3 mL), then 1-methylindazole-5-boric acid (1.1 g, 6.25 mmol) and $K_2CO_3$ (1.8 g, 13.02 mmol) were added in sequence, replacement with nitrogen was carried out, $Pd(dppf)Cl_2$ (472 mg, 0.64 mmol) was added, replacement with nitrogen was carried out again, and a reaction system was placed at 80°C and stirred to react for 3 hours. The reaction system was cooled to the room temperature, water was added into the system for dilution, extraction was carried out 3 times with ethyl acetate, an organic phase was combined and then was washed with a saturated saline solution, drying was carried out with anhydrous sodium sulfate

sulfate, filtering was carried out, a filtrate was subjected to vacuum concentration, and a crude product was purified using silica gel column chromatography to obtain 1.1 g of a title compound, a yield being 60%.

Second step: preparation of 6-amino-2-(4-cyano-3-fluorophenyl)-3-(1-methyl-1*H*-indazol-5-yl)isonicotinonitrile (23b)

The compound 23a (1.1 g, 3.88 mmol) was dissolved in 1,4-dioxane (30 mL) and water (3 mL), then 3-fluoro-4-cyanophenylboronic acid (1.9 g, 11.52 mmol) and $K_2CO_3$ (1.6 g, 11.58 mmol) were added in sequence, replacement with nitrogen was carried out, Pd(dppf)$Cl_2$ (278 mg, 0.38 mmol) was added, replacement with nitrogen was carried out again, and a reaction system was placed at 80°C and stirred to react overnight. The reaction system was cooled to the room temperature, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 1.1 g of a title compound, a yield being 77%.

Third step: preparation of 2-(4-cyano-3-fluorophenyl)-6-iodo-3-(1-methyl-1*H*-indazol-5-yl)isonicotinonitrile (23c)

The compound 23b (500 mg, 1.36 mmol) was dissolved in acetonitrile (10 mL), then potassium iodide (451 mg, 2.72 mmol) and isoamyl nitrite (319 mg, 2.72 mmol) were added in sequence, and a reaction system was placed at 80°C and stirred to react overnight. The reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 90 mg of a title compound, a yield being 14%.

Fourth step: preparation of tert-butyl (4-(4-cyano-6-(4-cyano-3-fluorophenyl)-5-(1-methyl-1*H*-indazol-5-yl)pyrid-2-yl) cyclohexyl-3-alken-1-yl )carbamate (23d)

The compound 23c (90 mg, 0.19 mmol) was dissolved in 1,4-dioxane (3 mL) and water (0.6 mL), then tert-butyl (4-(4,4,5,5-tetramethyl-1,3,2-dioxaborolane-2-yl)cyclohexyl-3-alken-1-yl)carbamate (184 mg, 0.57 mmol) and $K_2CO_3$ (79 mg, 0.57 mmol) were added in sequence, replacement with nitrogen was carried out, Pd(dppf)$Cl_2$ (14 mg, 0.02 mmol) was added, replacement with nitrogen was carried out again, and a reaction system was placed at 80°C and stirred to react for 2 hours. The reaction system was cooled to the room temperature, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 80 mg of a title compound, a yield being 78%.

Fifth step: preparation of tert-butyl (4-(4-cyano-6-(4-cyano-3-fluorophenyl)-5-(1-methyl-1*H*-indazol-5-yl)pyrid-2-yl)cyclohexyl)carbamate (23e)

The compound 23d (80 mg, 0.14 mmol) was dissolved in ethyl acetate (3 mL), then Pd/C (8 mg, 10%) was added at the room temperature, air in a system was replaced with hydrogen, and then the system was stirred at the room temperature under a hydrogen atmosphere to react overnight. A reaction system was filtered, a filtrate was subjected to vacuum concentration, and a crude product was purified using a thin layer silica gel plate to obtain 67 mg of a title compound, a yield being 83%.

Sixth step: preparation of 6-(4-aminocyclohexyl)-2-(4-cyano-3-fluorophenyl)-3-(1-methyl-1*H*-indazol-5-yl)isonicotinonitrile (23-1, 23-2)

The compound 23e (67 mg, 0.12 mmol) was dissolved in ethyl acetate (1 mL), then a hydrochloric acid-ethyl acetate solution (3 mL, 3M in EA) was added, and a reaction system was placed at the room temperature and stirred to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate (DCM/MeOH = 10:1) to obtain 10 mg of a title compound 23-1 (upper point: $R_f$ = 0.3), a yield being 18%, and 10 mg of a title compound 23-2 (lower point: $R_f$ = 0.25), a yield being 18%.

Upper point:

LC-MS (ESI) m/z $(M+H)^+$: 451.2
$^1$H NMR (400 MHz, DMSO-$d_6$) δ 8.13-8.08 (m, 2H), 7.83-7.80 (m, 1H), 7.79-7.73 (m, 1H), 7.69-7.64 (m, 1H), 7.49-7.44 (m, 1H), 7.26-7.22 (m, 1H), 7.21-7.16 (m, 1H), 4.07 (s, 3H), 3.24-3.18 (m, 1H), 3.03-2.92 (m, 1H), 2.15-2.02 (m, 2H), 1.80-1.60 (m, 4H), 1.25-1.10 (m, 2H).

Lower point:

LC-MS (ESI) m/z $(M+H)^+$: 451.2
$^1$H NMR (400 MHz, DMSO-$d_6$) δ 8.12-8.10 (m, 1H), 8.03 (s, 1H), 7.82-7.80 (m, 1H), 7.79-7.73 (m, 1H), 7.68-7.64 (m, 1H), 7.46-7.41 (m, 1H), 7.26-7.22 (m, 1H), 7.20-7.16 (m, 1H), 4.07 (s, 3H), 2.88-2.72 (m, 2H), 2.03-1.91 (m, 4H), 1.75-1.62 (m, 2H), 1.36-1.25 (m, 2H).

**Example 24** Preparation of 2-(4-cyano-3-fluorophenyl)-3-(3-fluoro-4-methoxyphenyl)-6-(7-oxo-1,4-diaza-1-yl)isonicotinonitrile (24)

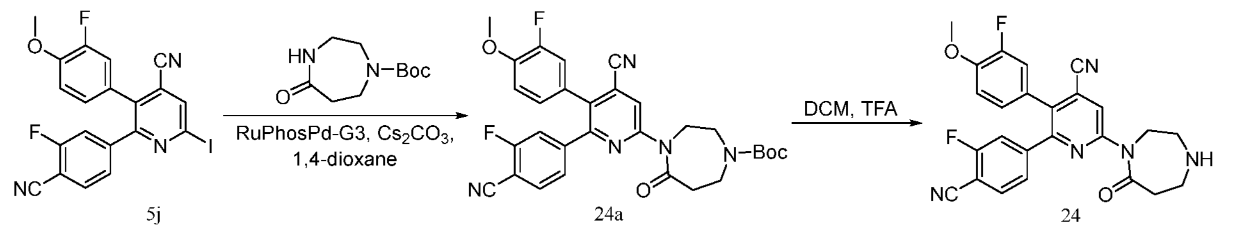


First step: preparation of tert-butyl 4-(4-cyano-6-(4-cyano-3-fluorophenyl)-5-(3-fluoro-4-methoxyphenyl)pyrid-2-yl)-5-oxy-1,4-diazane-1-ca rboxylate (24a)

A compound 5j (50 mg, 0.11 mmol) was dissolved in 1,4-dioxane (3 mL), then 1-tert-butoxycarbonyl-1,4-diaza-5-cycloheptanone (47 mg, 0.22 mmol) and $Cs_2CO_3$ (107 mg, 0.33 mmol) were added in sequence, replacement with nitrogen was carried out, RuPhos-Pd-G3 (9 mg, 0.01 mmol) was added, replacement with nitrogen was carried out again, and this system was heated to 80°C and stirred to react for 3 hours. A reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 40 mg of a title compound, a yield being 68%.

Second step: preparation of 2-(4-cyano-3-fluorophenyl)-3-(3-fluoro-4-methoxyphenyl)-6-(7-oxo-1,4-diaza-1-yl)isonicotinonitrile (24)

The compound 24a (40 mg, 0.07 mmol) was dissolved in dichloromethane (2 mL), then trifluoroacetic acid (1 mL) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 16 mg of a title compound, a yield being 49%.

LC-MS (ESI) m/z $(M+H)^+$: 460.2
$^1$H NMR (400 MHz, DMSO-$d_6$) δ 8.29 (s, 1H), 7.89-7.82 (m, 1H), 7.49 (dd, $J$ = 10.4, 1.2 Hz, 1H), 7.31 (dd, $J$ = 12.0, 2.0

Hz, 1H), 7.25-7.18 (m, 2H), 7.11-7.05 (m, 1H), 4.29-4.19 (m, 2H), 3.85 (s, 3H), 3.03-2.94 (m, 4H), 2.91-2.84 (m, 2H).

**Example** 25 Preparation of 6-(4-aminopiperid-1-yl)-2-(4-cyano-3-fluorophenyl)-3-(2,2-difluorobenzo[1,3-*d*]dioxacyclo-5-yl)isonicot inonitrile (25)

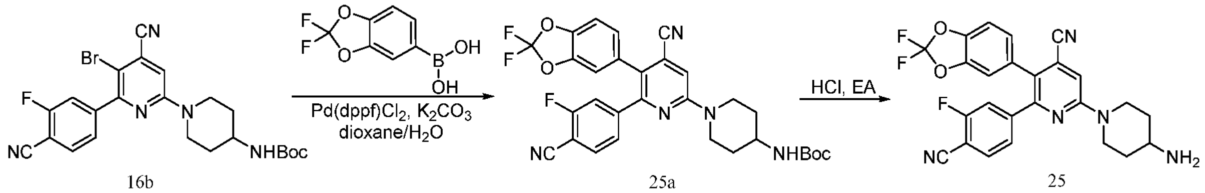


16b 25a 25

First step: preparation of tert-butyl (1-(4-cyano-6-(4-cyano-3 -fluorophenyl)-5 -(2,2-difluorobenzo [1,3 -*d*]dioxacyclo-5-yl)pyrid-2-yl)piperid-4 -yl)carbamate (25a)

A compound 16b (75 mg, 0.15 mmol), (2,2-difluorobenzo[1,3-*d*]dioxol-5-yl)boric acid (46 mg, 0.23 mmol) and $K_2CO_3$ (62 mg, 0.45 mmol) were dissolved in 1,4-dioxane (1.5 mL) and water (0.3 mL), replacement with nitrogen was carried out, $Pd(dppf)Cl_2$ (15 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 53 mg of a title compound, a yield being 61%.

Second step: preparation of 6-(4-aminopiperid-1-yl)-2-(4-cyano-3-fluorophenyl)-3-(2,2-difluorobenzo[1,3-*d*]dioxacyclo-5-yl)isonicot inonitrile (25)

The compound 25a (53 mg, 0.09 mmol) was dissolved in ethyl acetate (1 mL), then a hydrochloric acid-ethyl acetate solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 34 mg of a title compound, a yield being 78%.

LC-MS (ESI) m/z $(M+H)^+$: 478.2
$^1$H NMR (400 MHz, DMSO-$d_6$) δ 7.80 (dd, *J* = 8.0, 6.8 Hz, 1H), 7.57 (s, 1H), 7.44 (d, *J* = 1.6 Hz, 1H), 7.43-7.38 (m, 2H), 7.15 (dd, *J* = 8.0, 1.2 Hz, 1H), 7.04 (dd, *J* = 8.0, 1.2 Hz, 1H), 4.36-4.26 (m, 2H), 3.10-3.00 (m, 2H), 2.90-2.80 (m, 1H), 1.83-1.73 (m, 2H), 1.26-1.14 (m, 2H).

**Example 26** Preparation of 6-(4-cyano-3-fluorophenyl)-2-(1,4-diaza-1-yl)-5-(1-methyl-1H-indazol-5-yl)nicotinatnide (26)

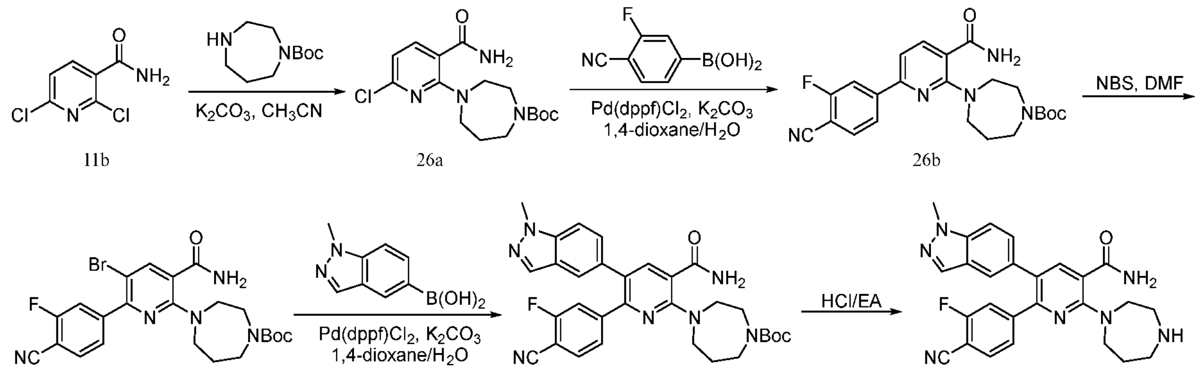


11b 26a 26b

26c 26d 26
First step: preparation of tert-butyl 4-(3-carbamoyl-6-chloropyrid-2-yl)-1,4-diazane-1-carboxylate (26a)

A compound 11b (550 mg, 2.88 mmol), tert-butyl 1,4-diazacycloheptane-1-formate (572 mg, 2.86 mmol), and $K_2CO_3$ (792 mg, 5.73 mmol) were dissolved in acetonitrile (7 mL), and this system was heated to 80°C and stirred to react overnight. A reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 620 mg of a title compound, a yield being 61%.

Second step: preparation of tert-butyl 4-(3-carbamoyl-6-(4-cyano-3-fluorophenyl)pyrid-2-yl)-1,4-diazane-1-carboxylate (26b)

The compound 26a (620 mg, 1.75 mmol), 3-fluoro-4-cyanophenylboronic acid (433 mg, 2.63 mmol) and $K_2CO_3$ (723 mg, 5.23 mmol) were dissolved in 1,4-dioxane (7 mL) and water (0.7 mL), replacement with nitrogen was carried out, Pd(dppf)$Cl_2$ (64 mg, 0.09 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 754 mg of a title compound, a yield being 98%.

Third step: preparation of tert-butyl 4-(5-bromo-3-carbamoyl-6-(4-cyano-3-fluorophenyl)pyrid-2-yl)-1,4-diazane-1-carboxylate (26c)

The compound 26b (754 mg, 1.72 mmol) was dissolved in *N,N*-dimethylformamide (9 mL), NBS (609 mg, 3.42 mmol) was slowly added into a system under an ice bath, and a reaction system was stirred at the room temperature to react for 2 hours. A reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 315 mg of a title compound, a yield being 35%.

Fourth step: preparation of tert-butyl 4-(3-carbamoyl-6-(4-cyano-3-fluorophenyl)-5-(1-methyl-1H-indazol-5-yl)pyrid-2-yl)-1,4-diazane-1-carb oxylate (26d)

The compound 26c (160 mg, 0.31 mmol), 1-methylindazole-5-boric acid (82 mg, 0.47 mmol) and $K_2CO_3$ (128 mg, 0.93 mmol) were dissolved in 1,4-dioxane (3 mL) and water (0.3 mL), replacement with nitrogen was carried out, Pd(dppf) $Cl_2$ (12 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a silica gel column to obtain 145 mg of a title compound, a yield being 82%.

Fifth step: preparation of 6-(4-cyano-3-fluorophenyl)-2-(1,4-diaza-1-yl)-5-(1-methyl-1H-indazol-5-yl)nicotinatnide (26)

The compound 26d (72 mg, 0.13 mmol) was dissolved in ethyl acetate (1 mL), then a hydrochloric acid-ethyl acetate solution (1 mL, 3M in EA) was added, and a reaction system was stirred at the room temperature to react for 2 hours. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 49 mg of a title compound, a yield being 83%.

LC-MS (ESI) m/z $(M+H)^+$: 470.2

$^1$H NMR (400 MHz, DMSO-*d*$_6$) δ 8.02 (s, 1H), 8.02-7.98 (m, 1H), 7.79-7.74 (m, 1H), 7.73-7.69 (m, 1H), 7.68-7.64 (m, 1H), 7.58-7.53 (m, 1H), 7.51-7.41 (m, 2H), 7.26-7.21 (m, 1H), 7.14-7.08 (m, 1H), 4.04 (s, 3H), 3.76-3.66 (m, 2H), 3.56-3.49 (m, 2H), 2.98-2.91 (m, 2H), 2.77-2.70 (m, 2H), 1.91-1.78 (m, 2H).

**Example 27** Preparation of 6-(4-cyano-3-fluorophenyl)-2-(1,4-diazaniline-1-yl)-*N,N*-dimethyl-5-(1-methyl-1*H*-indazol-5-yl)nicotina mide (27)

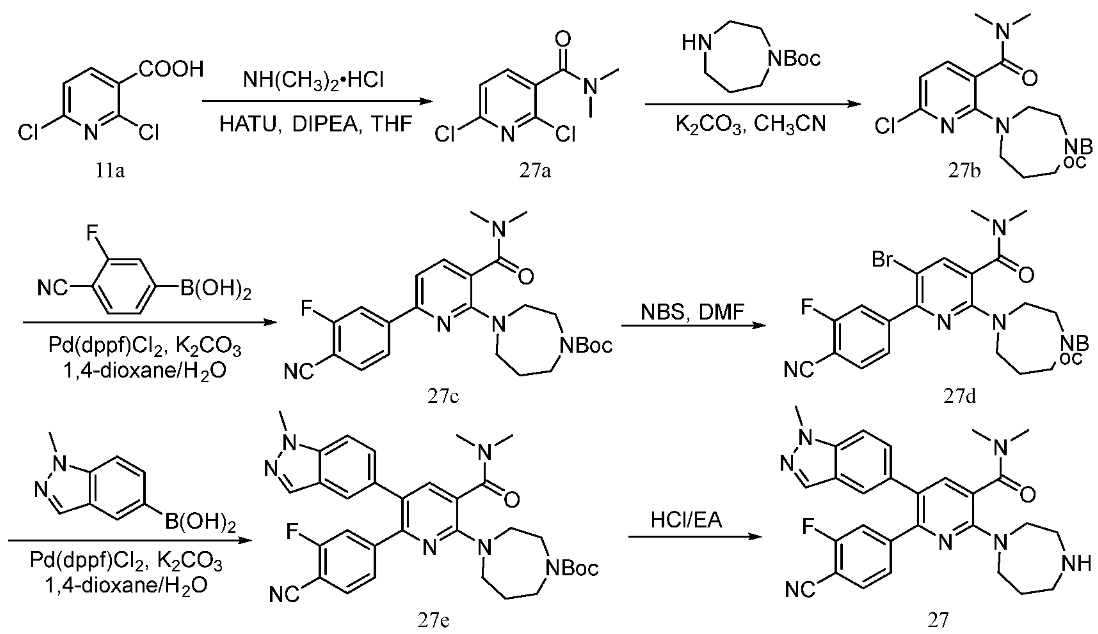


11a 27a 27b 27c 27d 27e 27

First step: preparation of 2,6-dichloro-*N,N*-dimethylnicotinamide (27a)

A compound 11a (500 mg, 2.60 mmol) was dissolved in tetrahydrofuran (5 mL), and then dimethylamine hydrochloride (636 mg, 7.80 mmol), 2-(7-azobenzotriazole)-*N,N,N',N"*-tetramethylurea hexafluorophosphate (1.2 g, 3.16 mmol) and *N,N*-diisopropylethylamine (1.0 g, 7.74 mmol) were added and stirred at the room temperature to react overnight. A reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and 570 mg of a title compound was obtained and was directly used for the next step of reaction without purification.

Second step: preparation of tert-butyl 4-(6-chloro-3-(dimethyl carbamoyl)pyrid-2-yl)-1,4-diazane-1-carboxylate (27b)

The compound 27a (570 mg, 2.60 mmol), tert-butyl 1,4-diazacycloheptane-1-formate (518 mg, 2.59 mmol), and $K_2CO_3$ (708 mg, 5.12 mmol) were dissolved in acetonitrile (7 mL), and this system was heated to 80°C and stirred to react overnight. A reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum

concentration, and a crude product was purified using silica gel column chromatography to obtain 653 mg of a title compound, a total yield in the two steps being 65%.

Third step: preparation of tert-butyl 4-(6-(4-cyano-3-fluorophenyl)-3-(dimethyl carbatnoyl)pyrid-2-yl)-1,4-diazane-1-carboxylate (27c)

The compound 27b (520 mg, 1.36 mmol), 3-fluoro-4-cyanophenylboronic acid (335 mg, 2.03 mmol) and $K_2CO_3$ (559 mg, 4.04 mmol) were dissolved in 1,4-dioxane (7 mL) and water (0.7 mL), replacement with nitrogen was carried out, Pd(dppf)$Cl_2$ (50 mg, 0.07 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 399 mg of a title compound, a yield being 63%.

Fourth step: preparation of tert-butyl 4-(5-bromo-6-(4-cyano-3-fluorophenyl)-3-(dimethyl carbamoyl)pyrid-2-yl)-1,4-diazane-1 -carboxylate (27d)

The compound 27c (389 mg, 0.83 mmol) was dissolved in *N,N*-dimethylformamide (4 mL), NBS (295 mg, 1.66 mmol) was slowly added into a system under an ice bath condition, and a reaction system was stirred at the room temperature to react for 2 hours. A reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 311 mg of a title compound, a yield being 68%.

Fifth step: preparation of tert-butyl 4-(6-(4-cyano-3-fluorophenyl)-3-(dimethyl carbamoyl)-5-(1-methyl-1H-indazol-5-yl) pyrid-2-yl)-1 ,4-diazane-1-carboxylate (27e)

The compound 27d (160 mg, 0.29 mmol), 1-methylindazole-5-boric acid (76 mg, 0.43 mmol) and $K_2CO_3$ (120 mg, 0.87 mmol) were dissolved in 1,4-dioxane (3 mL) and water (0.3 mL), replacement with nitrogen was carried out, Pd(dppf)$Cl_2$ (11 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a silica gel column to obtain 53 mg of a title compound, a yield being 30%.

Sixth step: preparation of 6-(4-cyano-3-fluorophenyl)-2-(1,4-diazaniline-1-yl)-*N,N*-dimethyl-5-(1-methyl-1*H*-indazol-5-yl)nicotina mide (27)

The compound 27e (53 mg, 0.09 mmol) was dissolved in ethyl acetate (1 mL), then a hydrochloric acid-ethyl acetate solution (1 mL, 3M in EA) was added, and a reaction system was stirred at the room temperature to react for 3 hours. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 31 mg of a title compound, a yield being 70%.

LC-MS (ESI) m/z $(M+H)^+$: 498.3
$^1H$ NMR (400 MHz, DMSO-$d_6$) δ 8.01 (s, 1H), 7.79-7.73 (m, 1H), 7.65 (s, 1H), 7.57-7.52 (m, 2H), 7.49-7.43 (m, 1H), 7.26-7.21 (m, 1H), 7.12-7.06 (m, 1H), 4.03 (s, 3H), 3.73-3.40 (m, 4H), 3.00 (s, 3H), 2.95 (s, 3H), 2.94-2.89 (m, 2H), 2.79-2.71 (m, 2H), 1.90-1.74 (m, 2H).

**Example 28** Preparation of 6-(4-cyano-3-fluorophenyl)-*N*-cyclopropyl-2-((1,4-diazaniline-1-yl)-5-(1-methyl-1*H*-indazol-5-yl)nicotin amide (28)

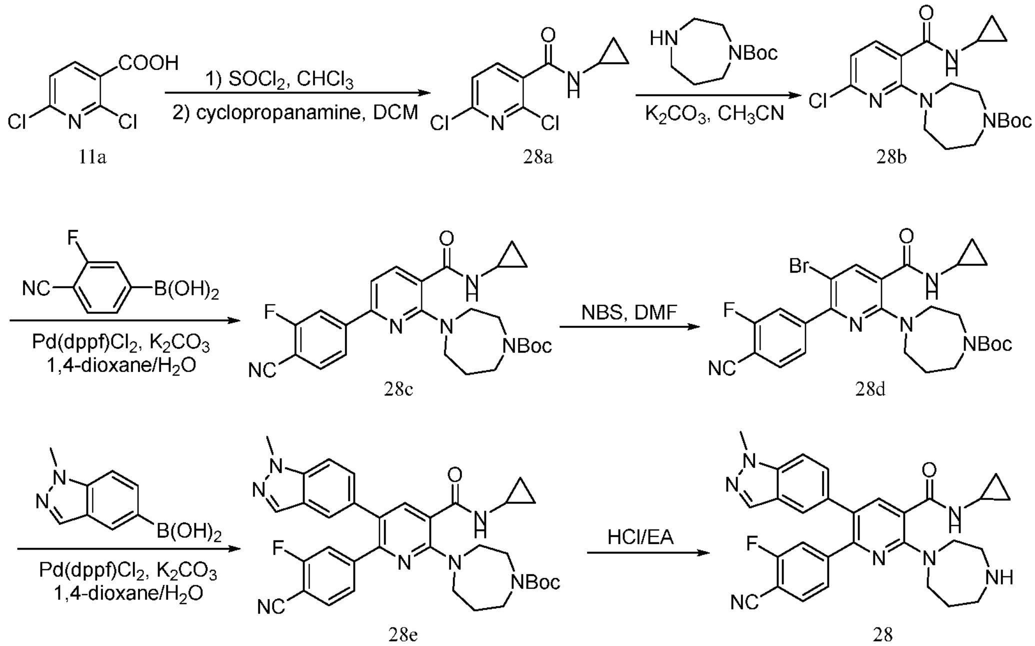


First step: preparation of 2,6-dichloro-*N*-cyclopropyl nicotinamide (28a)

A compound 11a (500 mg, 2.60 mmol) was dissolved in chloroform (10 mL), replacement with nitrogen was carried out, thionyl chloride (3.1 g, 26.00 mmol) was slowly added into a system under an ice bath, replacement with nitrogen was carried out again, and a system was heated to 70°C and stirred to react for 2 hours. A reaction system was subjected to vacuum concentration and dissolved by dichloromethane (1 mL), then was added dropwise into an ice-bath-cooled solution of cyclopropanamine (327 mg, 5.73 mmol) in dichloromethane (1 mL), and was stirred to react for 1 hour. Cold water was added into the reaction system to precipitate a solid, and after filtering, 600 mg of a title compound was obtained and directly used for the next step of reaction without purification.

Second step: preparation of tert-butyl 4-(6-chloro-3-(cyclopropyl carbamoyl)pyrid-2-yl)-1,4-diazane-1-carboxylate (28b)

The compound 28a (600 mg, 2.60 mmol), tert-butyl 1,4-diazacycloheptane-1-formate (520 mg, 2.60 mmol), and $K_2CO_3$ (718 mg, 5.20 mmol) were dissolved in acetonitrile (8 mL), and this system was heated to 80°C and stirred to react overnight. A reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 913 mg of a title compound, a total yield in the two steps being 89%.

Third step: preparation of tert-butyl 4-(6-(4-cyano-3-fluorophenyl)-3-(cyclopropyl carbamoyl)pyrid-2-yl)-1,4-diazane-1 -carboxylate (28c)

The compound 28b (520 mg, 1.32 mmol), 3-fluoro-4-cyanophenylboronic acid (327 mg, 1.98 mmol) and $K_2CO_3$ (547 mg, 3.96 mmol) were dissolved in 1,4-dioxane (5 mL) and water (0.5 mL), replacement with nitrogen was carried out, $Pd(dppf)Cl_2$ (48 mg, 0.07 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 630 mg of a title compound, a yield being 100%.

Fourth step: preparation of tert-butyl 4-(5-bromo-6-(4-cyano-3-fluorophenyl)-3-(cyclopropyl carbamoyl)pyrid-2-yl)-1,4-diazane-1 -carboxylate (28d)

The compound 28c (630 mg, 1.31 mmol) was dissolved in *N,N*-dimethylformamide (8 mL), NBS (480 mg, 2.70 mmol) was slowly added into a system under an ice bath condition, and a reaction system was stirred at the room temperature to react for 2 hours. A reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 315 mg of a title compound, a yield being 43%.

Fifth step: preparation of tert-butyl 4-(6-(4-cyano-3-fluorophenyl)-3-(cyclopropyl carbatnoyl)-5-(1-methyl-1*H*-indazol-5-yl)pyrid-2-yl)-1,4-diazane-1-carboxylate (28e)

The compound 28d (160 mg, 0.29 mmol), 1-methylindazole-5-boric acid (76 mg, 0.43 mmol) and $K_2CO_3$ (120 mg, 0.87 mmol) were dissolved in 1,4-dioxane (3 mL) and water (0.3 mL), replacement with nitrogen was carried out, Pd(dppf)$Cl_2$ (11 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a silica gel column to obtain 52 mg of a title compound, a yield being 30%.

Sixth step: preparation of 6-(4-cyano-3-fluorophenyl)-N-cyclopropyl-2-((1,4-diazaniline-1-yl)-5-(1-methyl-1*H*-indazol-5-yl)nicotin amide (28)

The compound 28e (52 mg, 0.09 mmol) was dissolved in ethyl acetate (1 mL), then a hydrochloric acid-ethyl acetate solution (1 mL, 3M in EA) was added, and a reaction system was stirred at the room temperature to react for 5 hours. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 20 mg of a title compound, a yield being 46%.

LC-MS (ESI) m/z $(M+H)^+$: 510.3
$^1$H NMR (400 MHz, DMSO-$d_6$) δ 8.60-8.57 (m, 1H), 8.01 (s, 1H), 7.79-7.73 (m, 1H), 7.66 (s, 1H), 7.60 (s, 1H), 7.57-7.52 (m, 1H), 7.47-7.41 (m, 1H), 7.25-7.20 (m, 1H), 7.11-7.06 (m, 1H), 4.01 (s, 3H), 3.71-3.64 (m, 2H), 3.54-3.47 (m, 2H), 2.96-2.88 (m, 2H), 2.84-2.76 (m, 1H), 2.75-2.68 (m, 2H), 1.89-1.77 (m, 2H), 0.72-0.65 (m, 2H), 0.54-0.48 (m, 2H).

**Example 29** Preparation of 2-(4-cyano-3-fluorophenyl)-3-(3-fluoro-4-methoxyphenyl)-6-(2,8-diazaspiro[4.5]dec-8-yl) isonicotinonitri le (29)

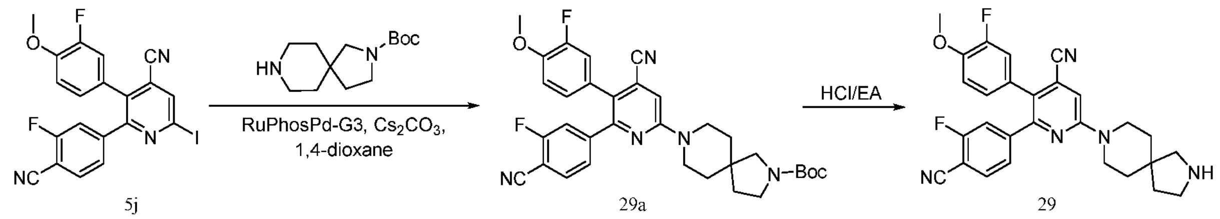

5j 29a 29

First step: preparation of tert-butyl 8-(4-cyano-6-(4-cyano-3-fluorophenyl)-5-(3-fluoro-4-methoxyphenyl)pyrid-2-yl)-2,8-diazaspiro[4.5]deca ne-2-carboxylate (29a)

A compound 5j (50 mg, 0.11 mmol) was dissolved in 1,4-dioxane (3 mL), then tert-butyl 2,8-diazaspiro[4.5] decane-2-carboxylate (53 mg, 0.22 mmol) and $Cs_2CO_3$ (107 mg, 0.33 mmol) were added in sequence, replacement with nitrogen was carried out, RuPhos-Pd-G3 (9 mg, 0.01 mmol) was added, replacement with nitrogen was carried out again, and this system was heated to 80°C and stirred to react for 3 hours. A reaction system was cooled to the room temperature,

a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 32 mg of a title compound, a yield being 52%.

Second step: preparation of 2-(4-cyano-3-fluorophenyl)-3-(3-fluoro-4-methoxyphenyl)-6-(2,8-diazaspiro[4.5]dec-8-yl) isonicotinonitri le (29)

The compound 29a (32 mg, 0.05 mmol) was dissolved in ethyl acetate (1 mL), then a hydrochloric acid-ethyl acetate solution (2 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 16 mg of a title compound, a yield being 60%.

LC-MS (ESI) m/z $(M+H)^+$: 486.3
$^1H$ NMR (400 MHz, DMSO-$d_6$) δ 7.85-7.77 (m, 1H), 7.57 (s, 1H), 7.46-7.36 (m, 1H), 7.24-7.11 (m, 3H), 7.01-6.92 (m, 1H), 3.85 (s, 3H), 3.81-3.57 (m, 4H), 3.50-3.24 (m, 3H), 2.96-2.88 (m, 1H), 1.81-1.72 (m, 1H), 1.65-1.58 (m, 1H), 1.58-1.48 (m, 4H).

Example 30 Preparation of 6-(aza-4-yl)-2-(4-cyano-3-fluorophenyl)-3-(3-fluoro-4-methoxyphenyl)isonicotinonitrile (30)

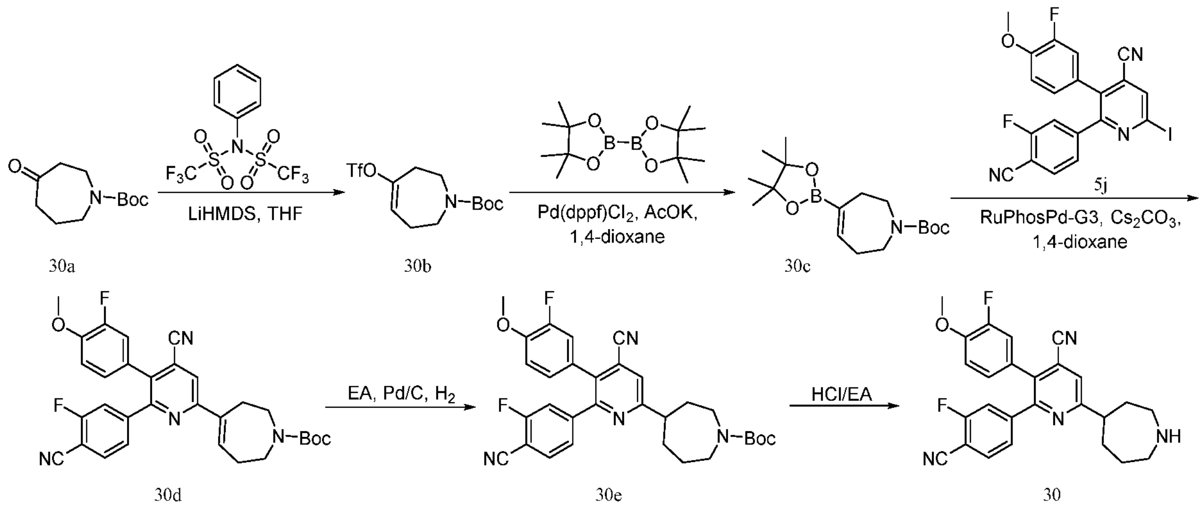


First step: preparation of tert-butyl 4-((((trifluoromethyl)sulfonyl)oxy)-2,3,6,7-tetrahydro-1*H*-aza-1-carboxylate (30b)

A compound 30a (3.0 g, 14.07 mmol) was dissolved in tetrahydrofuran (50 mL), replacement with nitrogen was carried out, the temperature was reduced to -78°C, lithium bis(trimethylsilyl)amide (19 mL, 1M in THF) was added dropwise, this system reacted at -78°C for 1 hour, then N-phenylbis(trifluoromethanesulfon)imide (6.5 g, 18.19 mmol) was added to continue the reaction for 0.5 hour, the system restored to the room temperature to react for 14 hours, then water was added for dilution, extraction was carried out 3 times with ethyl acetate, an organic phase was combined and then washed with a saturated saline solution, drying was carried out with anhydrous sodium sulfate, filtering was carried out, a filtrate was subjected to vacuum concentration, and a crude product was purified using silica gel column chromatography to obtain 2.0 g of a title compound, a yield being 41%.

Second step: preparation of tert-butyl 4-(4,4,5,5-tetramethyl-1,3,2-dioxybenzaldehyde-2-yl)-2,3,6,7-tetrahydro-1H-azacyclo-1-carboxylate (30c)

The compound 30b (500 mg, 1.45 mmol), bis(pinacolato)diboron (1.1 g, 4.33 mmol) and potassium acetate (426

mg, 4.34 mmol) were dissolved in 1,4-dioxane (5 mL), replacement with nitrogen was carried out, Pd(dppf)$Cl_2$ (106 mg, 0.15 mmol) was added, replacement with nitrogen was carried out again, and a reaction system was heated to 90°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 300 mg of a title compound, a yield being 64%.

Third step: preparation of tert-butyl 4-(4-cyano-6-(4-cyano-3-fluorophenyl)-5-(3-fluoro-4-methoxyphenyl)pyrid-2-yl)-2,3,6,7-tetrahydro-1*H*-azapyridine-1-carboxylate (30d)

A compound 5j (50 mg, 0.11 mmol), the compound 30c (177 mg, 0.55 mmol) and $K_2CO_3$ (45 mg, 0.33 mmol) were dissolved in 1,4-dioxane (2 mL) and water (0.4 mL), replacement with nitrogen was carried out, Pd(dppf)$Cl_2$ (7 mg, 0.01 mmol) was added, replacement with nitrogen was carried out again, and a reaction system was heated to 80°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 20 mg of a title compound, a yield being 35%.

Fourth step: preparation of tert-butyl 4-(4-cyano-6-(4-cyano-3-fluorophenyl)-5-(3-fluoro-4-methoxyphenyl)pyrid-2-yl) azacycloheptane-1-carb oxylate (30e)

The compound 30d (20 mg, 0.04 mmol) was dissolved in ethyl acetate (2 mL), then Pd/C (2 mg, 10%) was added at the room temperature, air in a system was replaced with hydrogen, and then the system was stirred at the room temperature under a hydrogen atmosphere to react overnight. A reaction system was filtered, a filtrate was subjected to vacuum concentration, and a crude product was purified using a thin layer silica gel plate to obtain 10 mg of a title compound, a yield being 50%.

Fifth step: preparation of 6-(aza-4-yl)-2-(4-cyano-3-fluorophenyl)-3-(3-fluoro-4-methoxyphenyl)isonicotinonitrile (30)

The compound 30e (10 mg, 0.02 mmol) was dissolved in ethyl acetate (1 mL), then a hydrochloric acid-ethyl acetate solution (2 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 6 mg of a title compound, a yield being 74%.

LC-MS (ESI) m/z $(M+H)^+$: 445.2
$^1$H NMR (400 MHz, DMSO-$d_6$) δ 8.03-7.98 (m, 1H), 7.88-7.82 (m, 1H), 7.46-7.41 (m, 1H), 7.33-7.28 (m, 1H), 7.24-7.16 (m, 2H), 7.10-7.04 (m, 1H), 3.86 (s, 3H), 3.66-3.45 (m, 1H), 3.22-3.10 (m, 1H), 3.08-2.88 (m, 2H), 2.87-2.74 (m, 1H), 2.05-1.58 (m, 6H).

**Example 31** Preparation of 2-(4-cyano-3-fluorophenyl)-3-(3-fluoro-4-methoxyphenyl)-6-(6-hydroxyl-1,4-diaza-1-yl)isonicotinonitril e (31)

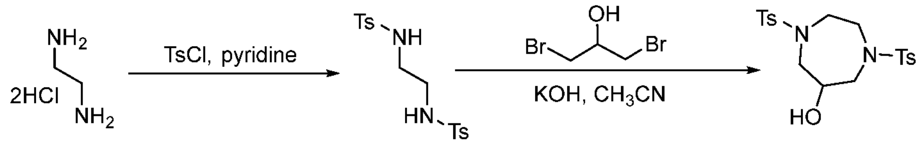


31a 31b 31c

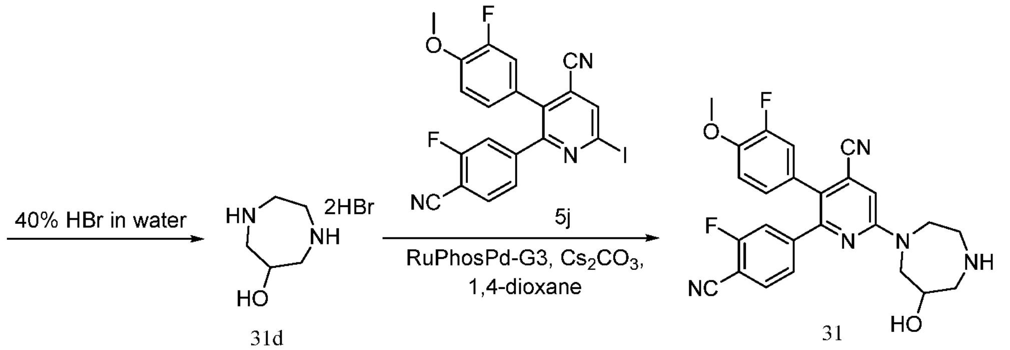


31d 31

First step: preparation of *N,N'*-(ethane-1,2-diyl)bis(4-methylbenzene sulfonamide) (31b)

A compound 31a (10.0 g, 75.18 mmol) was dissolved in pyridine (200 mL), then tosyl chloride (28.0 g, 146.87 mmol) was added, and this system was heated to 100°C and stirred to react for 16 hours. A reaction system was cooled to the room temperature and then poured into water, a solid was precipitated, and filtering and vacuum drying were carried out to obtain 18.0 g of a title compound, a yield being 65%.

Second step: preparation of 1,4-di-p-toluene sulfonyl-1,4-diaza-6-ol (31c)

The compound 31b (5.8 g, 15.74 mmol) was dissolved in acetonitrile (100 mL), potassium hydroxide (2.2 g, 39.21 mmol) was added, a reaction system was heated to 80°C to react for 0.5 hour, the system was cooled to the room temperature, then 1,3-dibromo-2-propanol (4.1 g, 18.82 mmol) was added, and the reaction system was heated to 80°C again to react for 16 hours. The reaction system was cooled to the room temperature and then subjected to vacuum concentration, then water was added for dilution, extraction was carried out 3 times with dichloromethane, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 5.3 g of a title compound, a yield being 79%.

Third step: preparation of 1,4-diaza-6-ol hydrobromide (31d)

The compound 31c (2.0 g, 4.71 mmol) was dissolved in a hydrogen bromide aqueous solution (20 mL, 40%), and a reaction system was heated to 100°C to react for 22 hours. The reaction system was cooled to the room temperature and then subjected to vacuum concentration, and then beating was carried out using methyl tert-butyl ester to obtain 600 mg of a crude product, a yield being 46%.

Fourth step: Preparation of 2-(4-cyano-3 -fluorophenyl)-3 -(3 -fluoro-4-methoxyphenyl)-6-(6-hydroxyl-1,4-diaza-1 -yl) isonicotinonitril e (31)

A compound 5j (50 mg, 0.11 mmol) was dissolved in 1,4-dioxane (3 mL), then the compound 31d (214 mg, 0.77 mmol) and $Cs_2CO_3$ (358 mg, 1.10 mmol) were added in sequence, replacement with nitrogen was carried out, RuPhos-Pd-G3 (9 mg, 0.01 mmol) was added, replacement with nitrogen was carried out again, and this system was heated to 80°C and stirred to react for 3 hours. A reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 5 mg of a title compound, a yield being 10%.
LC-MS (ESI) m/z $(M+H)^+$: 462.2

**Example** 32 Preparation of 2-(4-cyano-3-fluorophenyl)-6-(6-fluoro-1,4-diaza-1-yl)-3-(3-fluoro-4-methoxyphenyl)isonicotinonitrile (32-1, 32-2)

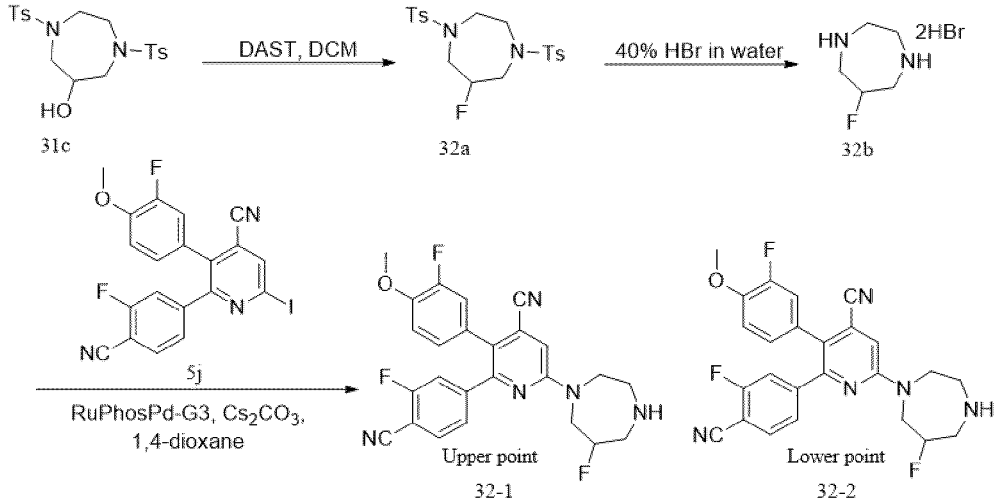


31c 32a 32b 5j 32-1 32-2

First step: preparation of 6-fluoro-1,4-di-p-toluene sulfonyl-1,4-diazane (32a)

A compound 31c (2.5 g, 5.89 mmol) was dissolved in dichloromethane (30 mL), replacement with nitrogen was carried out, a system was cooled to 0°C, then (diethylamino)sulfur trifluoride (1.9 g, 11.79 mmol) was added, and a reaction system was heated to the room temperature to react for 16 hours. Water was then added to quench the reaction, extraction was carried out 3 times with dichloromethane, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 1.6 g of a title compound, a yield being 64%.

Second step: preparation of 6-fluoro-1,4-diazacycloheptane (32b)

The compound 32a (1.6 g, 3.75 mmol) was dissolved in a hydrogen bromide aqueous solution (20 mL, 40%), and a reaction system was heated to 120°C to react for 22 hours. The reaction system was cooled to the room temperature and then subjected to vacuum concentration, and then beating was carried out using methyl tert-butyl ester to obtain 700 mg of a crude product, a yield being 67%.

Third step: preparation of 2-(4-cyano-3-fluorophenyl)-3-(3-fluoro-4-methoxyphenyl)-6-(6-hydroxyl-1,4-diaza-1-yl)isonicotinonitril e (32-1, 32-2)

A compound 5j (50 mg, 0.11 mmol) was dissolved in 1,4-dioxane (3 mL), then the compound 32b (216 mg, 0.77 mmol) and $Cs_2CO_3$ (358 mg, 1.10 mmol) were added in sequence, replacement with nitrogen was carried out, RuPhos-Pd-G3 (9 mg, 0.01 mmol) was added, replacement with nitrogen was carried out again, and this system was heated to 80°C and stirred to react for 3 hours. A reaction system was cooled to the room temperature, a reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain title compounds 32-1 (3 mg, yield: 6%) and 32-2 (4 mg, yield: 8%).

32-1: LC-MS (ESI) m/z $(M+H)^+$: 464.2
$^1H$ NMR (400 MHz, DMSO-$d_6$) $\delta$ 7.85-7.78 (m, 1H), 7.56 (s, 1H), 7.43-7.38 (m, 1H), 7.24-7.12 (m, 3H), 7.00-6.94 (m, 1H), 4.53-4.44 (m, 1H), 4.41-4.28 (m, 2H), 4.26-4.18 (m, 1H), 3.85 (s, 3H), 3.03-2.88 (m, 2H), 2.78-2.65 (m, 2H), 2.03-1.95 (m, 1H).
32-2: LC-MS (ESI) m/z $(M+H)^+$: 464.2
$^1H$ NMR (400 MHz, DMSO-$d_6$) $\delta$ 7.85-7.79 (m, 1H), 7.47 (s, 1H), 7.40 (dd, *J* = 10.8, 1.2 Hz, 1H), 7.23-7.12 (m, 3H), 6.99-6.93 (m, 1H), 4.20-3.91 (m, 2H), 3.85 (s, 3H), 3.80-3.69 (m, 2H), 3.00-2.75 (m, 4H), 2.03-1.94 (m, 1H).

Example 33 Preparation of 6-(4-aminopiperid-1-yl)-2-(4-cyano-3-fluorophenyl)-3-(5-fluoro-3-tolueno[*d*]isoxazol-6-yl) isonicotinonit rile (33)

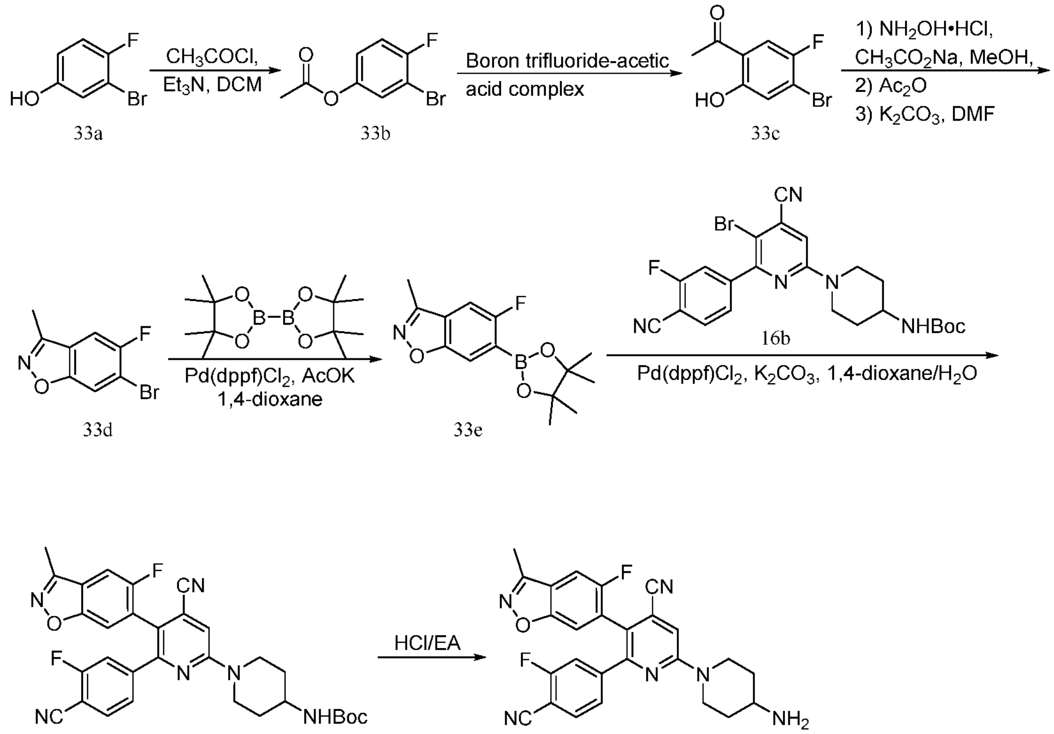


33a 33b 33c 33d 33e 16b 33f 33

First step: preparation of 3-bromo-4-fluorophenylacetate (33b)

A compound 33a (2.0 g, 10.47 mmol) was dissolved in dichloromethane (20 mL), triethylamine (1.6 g, 15.81 mmol) was slowly added into a system under an ice bath, then acetyl chloride (1.2 g, 15.29 mmol) was slowly added, and a reaction system restored to the room temperature and was stirred to react for 1 hour. A reaction liquid was poured into water, extraction was carried out 3 times with dichloromethane, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 2.4 g of a title compound, a yield being 98%.

Second step: preparation of 1-(4-bromo-5-fluoro-2-hydroxylphenyl)ethane-1-one (33c)

The compound 33b (2.4 g, 10.30 mmol) was dissolved in a boron trifluoride-acetic acid solution (26 mL, 33% $BF_3$), replacement with nitrogen was carried out, and a reaction system was heated to 150°C and stirred to react for 8 hours. The reaction system was cooled to the room temperature, a reaction liquid was poured into water to precipitate a solid, and after filtering, 1.6 g of a title compound crude product was obtained and directly used for the next step without purification.

Third step: preparation of 6-bromo-5-fluoro-3-tolueno[d]isoxazole (33d)

The compound 33c (1.6 g, 6.87 mmol) was dissolved in methanol (16 mL), then hydroxylamine hydrochloride (963 mg, 13.86 mmol) and sodium acetate (853 mg, 10.40 mmol) were added, and a reaction system was heated to 60°C and stirred to react for 1 hour. The reaction system was cooled to the room temperature, a reaction liquid was poured into cold water to precipitate a solid, filtering was carried out, and a filter cake was dried to obtain a brown solid. The obtained solid was dissolved in acetic anhydride (6 mL), a reaction system was heated to 60°C and stirred to react for 2 hours, the reaction system was cooled to the room temperature, a reaction liquid was poured into water, extraction was carried out 3 times with ethyl acetate, an organic phase was combined and then was washed with a saturated saline solution, drying was

carried out with anhydrous sodium sulfate, filtering was carried out, and a filtrate was subjected to vacuum concentration to obtain a crude product. The obtained crude product was dissolved in *N,N*-dimethylformamide (10 mL), then $K_2CO_3$ (1.9 g, 13.75 mmol) was added, and a reaction system was stirred at the room temperature to react overnight. A reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 693 mg of a title compound, a total yield in two steps being 29%.

Fourth step: preparation of 5-fluoro-3-methyl-6-(4,4,5,5-tetramethyl-1,3,2-dioxybenzaldehyde-2-yl)benzo[d]isoxazole (33e)

The compound 33d (693 mg, 3.01 mmol), bis(pinacolato)diboron (914 mg, 3.60 mmol) and potassium acetate (588 mg, 5.99 mmol) were dissolved in 1,4-dioxane (7 mL), replacement with nitrogen was carried out, Pd(dppf)$Cl_2$ (66 mg, 0.09 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 757 mg of a title compound, a yield being 91%.

Fifth step: preparation of tert-butyl (1-(4-cyano-6-(4-cyano-3-fluorophenyl)-5-(5-fluoro-3-methylbenzo[d]isoxazol-6-yl) pyrid-2-yl)piperid-4-yl)carbamate (33f)

A compound 16b (150 mg, 0.30 mmol), the compound 33e (166 mg, 0.60 mmol) and $K_2CO_3$ (124 mg, 0.90 mmol) were dissolved in 1,4-dioxane (2 mL) and water (0.2 mL), replacement with nitrogen was carried out, Pd(dppf)$Cl_2$ (15 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 41 mg of a title compound, a yield being 24%.

Sixth step: preparation of 6-(4-aminopiperid-1-yl)-2-(4-cyano-3 -fluorophenyl)-3 -(5-fluoro-3 -tolueno [*d*]isoxazol-6-yl) isonicotinonit rile (33)

The compound 33f (41 mg, 0.07 mmol) was dissolved in ethyl acetate (1 mL), then an ethyl hydrochloride solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 12 mg of a title compound, which was a yellow solid, a yield being 35%.

LC-MS (ESI) m/z $(M+H)^+$: 471.2
$^1$H NMR (400 MHz, DMSO-$d_6$) δ 7.90-7.87 (m, 1H), 7.82-7.74 (m, 2H), 7.66 (s, 1H), 7.47-7.42 (m, 1H), 7.16 (dd, *J* = 8.0, 1.6 Hz, 1H), 4.42-4.26 (m, 2H), 3.14-3.03 (m, 2H), 2.92-2.82(m, 1H), 2.55 (s, 3H), 1.84-1.74 (m, 2H), 1.26-1.16 (m, 2H).

**Example 34** Preparation of 6-(4-atninopiperid-1-yl)-2-(4-cyano-3-fluorophenyl)-3-(3,3-difluoro-1-methyl-2-oxoindol-5-yl)isonicotin onitrile (34)

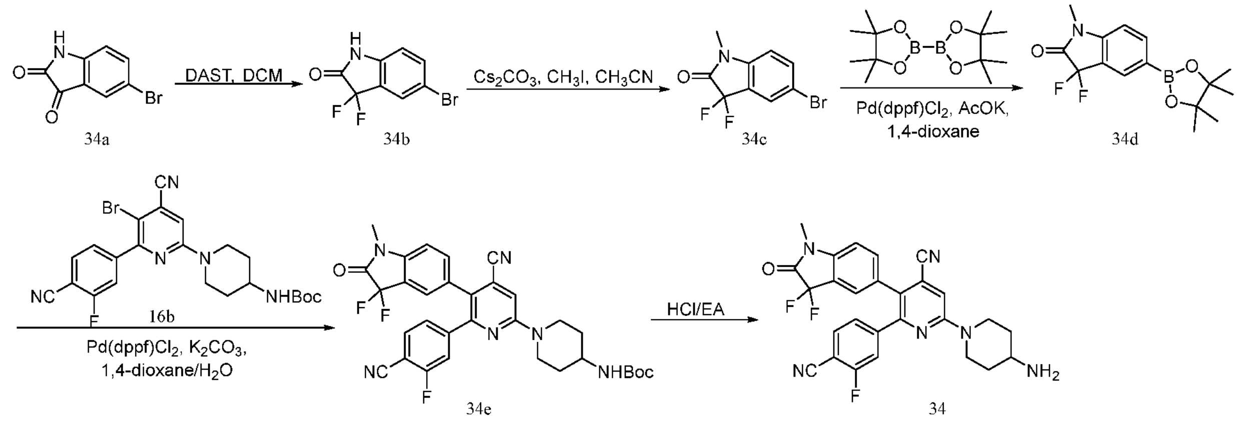

34a 34b 34c 34d 16b 34e 34

First step: preparation of 5-bromo-3,3-difluoroindole-2-one (34b)

A compound 34a (2.0 g, 8.85 mmol) was dissolved in dichloromethane (30 mL), replacement with nitrogen was carried out, a system was cooled to 0°C, then (diethylamino)sulfur trifluoride (4.3 g, 26.68 mmol) was added, and a reaction system was placed at the room temperature to react for 16 hours. Water was then added into the system to quench the reaction, extraction was carried out 3 times with dichloromethane, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 1.8 g of a title compound, a yield being 82%.

Second step: preparation of 5-bromo-3,3-difluoro-1-methylindole-2-one (34c)

The compound 34b (1.0 g, 4.03 mmol) was dissolved in acetonitrile (15 mL), then iodomethane (1.1 g, 7.75 mmol) and $Cs_2CO_3$ (2.0 g, 6.14 mmol) were added, and a reaction system was placed at the room temperature and stirred to react for 16 hours. A reaction liquid was subjected to vacuum concentration, water was added into the system for dilution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 900 mg of a title compound, a yield being 85%.

Third step: preparation of 3,3-difluoro-1-methyl-5-(4,4,5,5-tetramethyl-1,3,2-dioxybenzaldehyde-2-yl)indole-2-one (34d)

The compound 34c (500 mg, 1.91 mmol), bis(pinacolato)diboron (1.5 g, 5.91 mmol) and potassium acetate (562 mg, 5.73 mmol) were dissolved in 1,4-dioxane (5 mL), replacement with nitrogen was carried out, Pd(dppf)$Cl_2$ (139 mg, 0.19 mmol) was added, replacement with nitrogen was carried out again, and a reaction system was heated to 90°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 420 mg of a title compound, a yield being 71%.

Fourth step: preparation of tert-butyl (1-(4-cyano-6-(4-cyano-3-fluorophenyl)-5-(3,3-difluoro-1-methyl-2-oxoindol-5-yl) pyrid-2-yl)piperid-4-y l)carbamate (34e)

A compound 16b (80 mg, 0.16 mmol), the compound 34d (247 mg, 0.80 mmol) and $K_2CO_3$ (66 mg, 0.48 mmol) were dissolved in 1,4-dioxane (2 mL) and water (0.4 mL), replacement with nitrogen was carried out, Pd(dppf)$Cl_2$ (15 mg, 0.02 mmol) was added, replacement with nitrogen was carried out again, and a reaction system was heated to 80°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 30 mg of a title compound, a yield being 31%.

Fifth step: preparation of 6-(4-atninopiperid-1-yl)-2-(4-cyano-3-fluorophenyl)-3-(3,3-difluoro-1-methyl-2-oxoindol-5-yl) isonicotin onitrile (34)

The compound 34e (30 mg, 0.05 mmol) was dissolved in ethyl acetate (1 mL), then a hydrochloric acid-ethyl acetate solution (2 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 22 mg of a title compound, a yield being 88%.

LC-MS (ESI) m/z $(M+H)^+$: 503.2
$^1H$ NMR (400 MHz, DMSO-$d_6$) δ 7.85-7.78 (m, 1H), 7.63-7.54 (m, 2H), 7.51-7.45 (m, 1H), 7.44-7.38 (m, 1H), 7.26-7.20 (m, 1H), 7.19-7.13 (m, 1H), 4.40-4.26 (m, 2H), 3.18 (s, 3H), 3.11-2.98 (m, 2H), 2.95-2.82(m, 1H), 1.86-1.73 (m, 2H), 1.27-1.15 (m, 2H).

**Example 35** Preparation of 6-(4-atninopiperid-1-yl)-2-(4-cyano-3-fluorophenyl)-3-(4-(1,1-difluoro-2-hydroxyl-2-methylpropyl)-2-flu orophenyl)isonicotinonitrile (35)

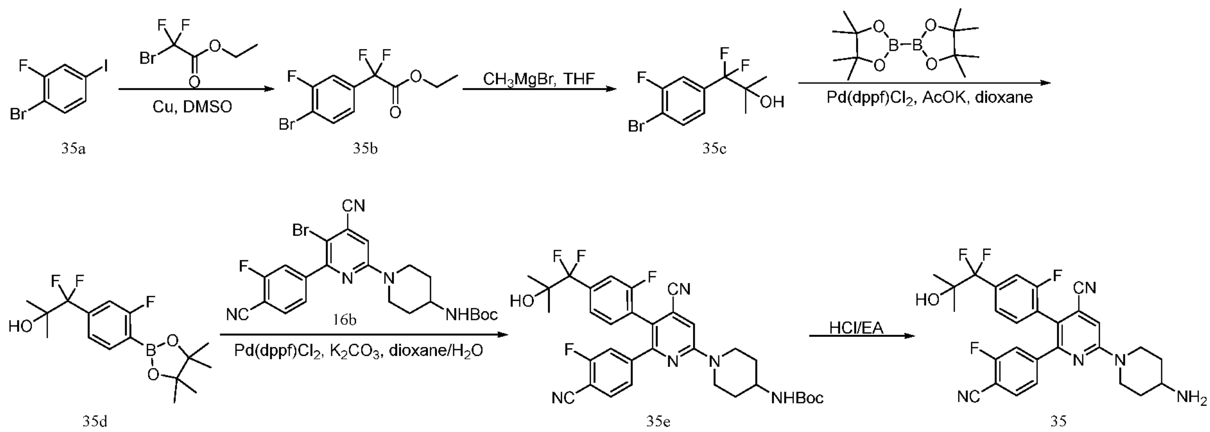

35a 35b 35c 35d 16b 35e 35

First step: preparation of 2-(4-bromo-3-fluorophenyl)-2,2-difluoro ethyl acetate (35b)

A compound 35a (1.3 g, 4.32 mmol) was dissolved in dimethylsulfoxide (20 mL), replacement with nitrogen was carried out, copper powder (707 mg, 11.13 mmol) was added and stirred at the room temperature for 1 hour, then 2-bromo-2,2-difluoro ethyl acetate (2.3 g, 11.33 mmol) was added, and a reaction system was heated to 80°C and stirred to react for 2 hours. The reaction system was cooled to the room temperature, filtering was carried out, a filtrate was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 719 mg of a title compound, a yield being 56%.

Second step: preparation of 1-(4-bromo-3-fluorophenyl)-1,1-difluoro-2-methylpropane-2-ol (35c)

The compound 35b (719 mg, 2.42 mmol) was dissolved in dry tetrahydrofuran (20 mL), then a methyl magnesium bromide reagent (4.1 mL, 3M in THF) was slowly added dropwise under an ice bath, and after dropwise adding was finished, a reaction system slowly restored to the room temperature and was stirred to react overnight. The reaction system was cooled to 0°C, an appropriate amount of water was slowly added to quench the reaction, filtering was carried out, a filtrate was extracted 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 577 mg of a title compound, a yield being 84%.

Third step: preparation of 1,1-difluoro-(3-fluoro-4-(4,4,5,5-tetratnethyl-1,3,2-dioxybenzaldehyde-2-yl)phenyl)-2-methylpropane-2-ol (35d)

The compound 35c (577 mg, 2.04 mmol), bis(pinacolato)diboron (1.0 g, 3.94 mmol) and potassium acetate (600 mg, 6.12 mmol) were dissolved in 1,4-dioxane (20 mL), replacement with nitrogen was carried out, Pd(dppf)$Cl_2$ (146 mg, 0.20 mmol) was added, replacement with nitrogen was carried out again, and a reaction system was heated to 100°C and stirred to react for 24 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 364 mg of a title compound, a yield being 54%.

Fourth step: preparation of tert-butyl (1-(4-cyano-6-(4-cyano-3-fluorophenyl)-5-(4-(1,1-difluoro-2-hydroxyl-2-methylpropyl)-2-fluorophenyl)p yrid-2-yl)piperid-4-yl)carbamate (35e)

A compound 16b (100 mg, 0.20 mmol), the compound 35d (132 mg, 0.40 mmol) and $K_2CO_3$ (83 mg, 0.60 mmol) were dissolved in 1,4-dioxane (2 mL) and water (0.2 mL), replacement with nitrogen was carried out, Pd(dppf)$Cl_2$ (15 mg, 0.02 mmol) was added, replacement with nitrogen was carried out again, and a reaction system was heated to 100°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 57 mg of a title compound, a yield being 46%.

Fifth step: preparation of 6-(4-aminopiperid-1-yl)-2-(4-cyano-3-fluorophenyl)-3-(4-(1,1-difluoro-2-hydroxyl-2-methylpropyl)-2-flu orophenyl)isonicotinonitrile (35)

The compound 35e (57 mg, 0.09 mmol) was dissolved in ethyl acetate (1 mL), then a hydrochloric acid-ethyl acetate solution (1 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 37 mg of a title compound, which was a yellow solid, a yield being 77%.

LC-MS (ESI) m/z $(M+H)^+$: 524.2
$^1H$ NMR (400 MHz, DMSO-$d_6$) δ 7.84-7.78 (m, 1H), 7.64 (s, 1H), 7.50-7.43 (m, 1H), 7.39-7.28 (m, 3H), 7.24-7.19 (m, 1H), 5.49 (s, 1H), 4.40-4.28 (m, 2H), 3.14-3.03 (m, 2H), 2.91-2.82(m, 1H), 1.85-1.75 (m, 2H), 1.28-1.18 (m, 2H), 1.15 (s, 6H).

**Example 36** Preparation of 6-(4-aminopiperid-1-yl)-2-(4-cyano-3-fluorophenyl)-3-(3,6-difluoro-1-(2-hydroxyl-2-methylpropyl)-1H-i ndazol-5-yl)isonicotinonitrile (36)

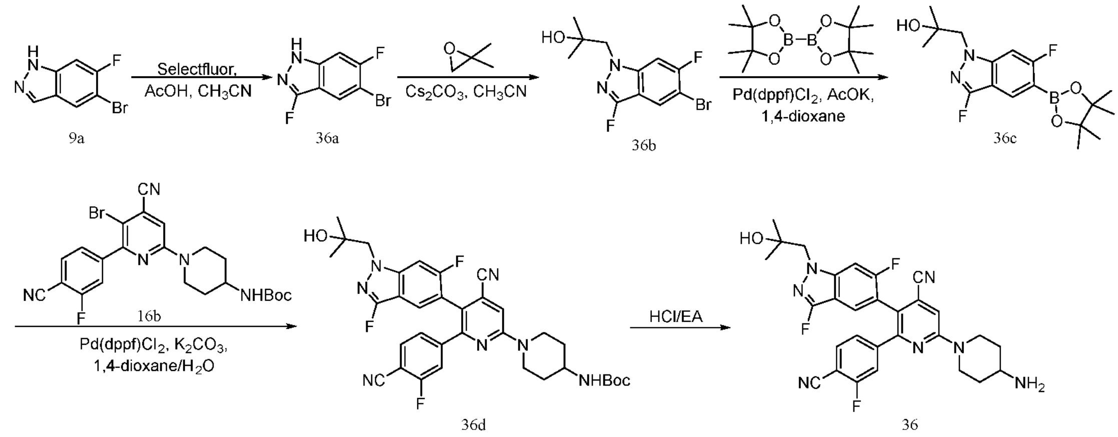


First step: preparation of 5-bromo-3,6-difluoro-1H-indazole (36a)

A compound 9a (500 mg, 2.33 mmol) was dissolved in acetonitrile (7.5 mL), glacial acetic acid (0.75 mL) and 1-chloromethyl-4-fluoro-1,4-diazabicyclo[2.2.2]octane bis(tetrafluoroborate) (1.6 g, 4.66 mmol) were added in sequence, and a reaction system was heated to 80°C and stirred to react for 5 hours. The reaction system was cooled to the room temperature, water was added into the system to quench the reaction, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 300 mg of a title compound, a yield being 55%.

Second step: preparation of 1-(5-bromo-3,6-difhioro-1*H*-indazol-1-yl)-2-methylpropane-2-ol (36b)

The compound 36a (300 mg, 1.29 mmol) was dissolved in acetonitrile (10 mL), then $Cs_2CO_3$ (630 mg, 1.94 mmol) and 1,1-dimethyl ethylene oxide (185 mg, 2.57 mmol) were added in sequence, and a reaction system was heated to 80°C and stirred to react for 16 hours. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 150 mg of a title compound, a yield being 38%.

Third step: preparation of 1-(3,6-difluoro-5-(4,4,5,5-tetramethyl-1,3,2-dioxybenzaldehyde-2-yl)-1*H*-indazol-1-yl)-2-methylpropane-2-ol (36c)

The compound 36b (150 mg, 0.49 mmol), bis(pinacolato)diboron (373 mg, 1.47 mmol) and potassium acetate (144 mg, 1.47 mmol) were dissolved in 1,4-dioxane (5 mL), replacement with nitrogen was carried out, $Pd(dppf)Cl_2$ (37 mg, 0.05 mmol) was added, replacement with nitrogen was carried out again, and a reaction system was heated to 90°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using silica gel column chromatography to obtain 76 mg of a title compound, a yield being 44%.

Fourth step: preparation of tert-butyl (1-(4-cyano-6-(4-cyano-3-fluorophenyl)-5-(3,6-difluoro-1-(2-hydroxyl-2-methylpropyl)-1H indazol-5-yl) pyrid-2-yl)piperid-4-yl)carbamate (36d)

A compound 16b (107 mg, 0.21 mmol), the compound 36c (74 mg, 0.21 mmol) and $K_2CO_3$ (87 mg, 0.63 mmol) were dissolved in 1,4-dioxane (2 mL) and water (0.4 mL), replacement with nitrogen was carried out, $Pd(dppf)Cl_2$ (15 mg, 0.02 mmol) was added, replacement with nitrogen was carried out again, and a reaction system was heated to 80°C and stirred to react overnight. The reaction system was cooled to the room temperature, a reaction liquid was poured into water, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 35 mg of a title compound, a yield being 26%.

Fifth step: preparation of 6-(4-atninopiperid-1-yl)-2-(4-cyano-3-fluorophenyl)-3-(3,6-difluoro-1-(2-hydroxyl-2-methyl-propyl)-1*H-i* ndazol-5-yl)isonicotinonitrile (36)

The compound 36d (35 mg, 0.05 mmol) was dissolved in ethyl acetate (1 mL), then a hydrochloric acid-ethyl acetate solution (2 mL, 3M in EA) was added, and a reaction system was stirred at the room temperature to react for 1 hour. The reaction system was subjected to vacuum concentration and then diluted by adding water, a pH value of the system was adjusted to about 8 using a saturated sodium bicarbonate solution, extraction was carried out 3 times with ethyl acetate, an organic phase was combined and then was washed with a saturated saline solution, drying was carried out with anhydrous sodium sulfate, filtering was carried out, a filtrate was subjected to vacuum concentration, and a crude product was purified using a thin layer silica gel plate to obtain 23 mg of a title compound, a yield being 78%.

LC-MS (ESI) m/z $(M+H)^+$: 546.3
$^1$H NMR (400 MHz, DMSO-$d_6$) δ 7.86-7.81 (m, 1H), 7.80-7.74 (m, 1H), 7.62 (s, 1H),7.60-7.54 (m, 1H), 7.44-7.38 (m, 1H), 7.19 (dd, *J* = 8.0, 1.2 Hz, 1H), 4.67 (brs, 1H), 4.40-4.28 (m, 2H), 4.20-4.07 (m, 2H), 3.13-3.01 (m, 2H), 2.94-2.82(m, 1H), 1.86-1.75 (m, 2H), 1.30-1.18 (m, 2H), 1.14 (s, 3H), 1.10 (s, 3H).

**Biological assessment**

Compounds 1-36 used in following test examples are prepared by synthesis methods of Examples 1-36 respectively.

**Test Example 1: LSD1 activity test**

1. Test objective

In this test, a homogeneous time resolved fluorescence (HTRF) technology was utilized, and the inhibitory activity of compounds on LSD1 was detected by adopting a two-step method. The first step was to incubate LSD1, compounds of different concentrations, and a biotin-labeled methylated polypeptide substrate (H3(1-21)K4 mel-biotin) at the room temperature for fixed time. The second step was to add an antibody (Anti-H3K4 me0-Eu(K)) to a demethylated polypeptide substrate and an XL-665-labeled streptomycin (SA-XL665) detection reagent, and continue to incubate at the room temperature for fixed time. A signal value Intensity (665nM)/Intensity (615nM) was read using EnVision to determine the content of the demethylated polypeptide substrate so as to evaluate the effects of the tested compounds on LSD1 activity, and meanwhile $IC_{50}$ values of the tested compounds on the LSD1 were calculated using an inhibition rate.

2. Test materials

2.1. Compounds to be tested:

Active drug: CC-90011, Chengdu Hyperway Pharmaceuticals, batch number HBW-013-177-77;
Tested drug: compounds 1-36.
2.2. Test reagents and instruments
LSD1, Active Motif;
DMSO, Sigma;
OptiPlate-384, PerkinElmer;
Sonic pipette, Labcyte;
Microplate reader (EnVision), PerkinElmer;
Centrifuge, Eppendorf.
3. Test method
3.1. Compound preparation

Preparation of a 10 mM compound stock solution: compound powder was dissolved in 100% DMSO to prepare the 10 mM compound stock solution respectively.

3.2. Enzyme process

(1) A1× buffer solution was prepared according to an LSD1 enzymatic kit provided by Active Motif (Catalog number: 31432).
(2) Preparation of a compound solution: a final concentration of compound $IC_{50}$ testing started at 500 nM, diluted by 5 folds, with 6 concentrations, and single/multiple well detection was set for each concentration. A corresponding solution with a 1000-fold final concentration was formed by dilution in a 384-well Source plate, and then Echo550 was used for transferring 10 nL to a 384-well reaction plate for being tested. 10 nL of 100% DMSO was transferred into a Min well and a Max well respectively.
(3) A 2x enzyme solution was prepared using a 1x reaction solution.
(4) A 2× substrate mixed solution was prepared using a 1× reaction solution.
(5) 5 μL of a 2× enzyme solution was added into each well, and 5 μL of a 1× reaction solution was added into the Min well, centrifuged at 1000 rpm for 1 min, and incubated at the room temperature for 15 min.
(6) 5 μL of a 2× substrate mixed solution was added into each well of the reaction plate to start the reaction, centrifuging was carried out at 1000 rpm for 1 min, and incubation was carried out at the room temperature for 60 min.
(7) 10 μL of a detection solution was added into each well, centrifuging was carried out at 1000 rpm for 1 min, and incubation was carried out at the room temperature for 60 min.
(8) a signal value Intensity (665nM)/Intensity (615nM) was read using EnVision.

3.3. Data analysis

An inhibition percentage was calculated using a maximum value, a minimum value, and a signal value (maximum value: fluorescence intensity of 5 μL of 2× enzyme solution + 5 μL of 2× substrate mixed solution + 10 nL of 100% DMSO + 10 μL of detection solution; minimum value: fluorescence intensity of 5 μL of 1× reaction solution + 5 μL of 2× substrate mixed solution + 10 nL of 100% DMSO + 10 μL of detection solution; and signal value: fluorescence intensity of 5 μL of 2× enzyme solution + 5 μL of 2× substrate mixed solution + 10 nL of diluted compound to be tested + 10 μL of detection solution), and a calculation formula was as follows:

$$\text{Inhibition percentage (\%)} = \frac{\text{Max value} - \text{signal value}}{\text{Max value} - \text{Min value}} \times 100$$

Using a logarithm of the concentration as an X axis and the percentage inhibition rate as a Y axis, a dose-response curve was fitted using a formula log(inhibitor) vs. response-Variable slope (Four parameters) of analysis software Graphpad Prism 5 to obtain the $IC_{50}$ value of each compound on the enzyme activity.

4. Test result

See Table 1 for inhibitory activities of the tested compounds in the present application and positive controls on the LSD1.

Table 1 Inhibitory activities of tested compounds in the present application and positive controls on LSD1

| No. of compounds | $IC_{50}$ (nM) | No. of compounds | $IC_{50}$ (nM) |
|---|---|---|---|
| 1 | 2.10 | 2 | 4.80 |
| 3 | 5.80 | 4 | 0.47 |
| 8 | 6.50 | 9 | 0.72 |
| 10 | 0.81 | 11 | 1.50 |
| 12 | 4.40 | 14 | 8.9 |
| 15 | 2.00 | 16 | 0.23 |
| 17 | 1.20 | 18 | 1.50 |
| 19 | 3.10 | 20 | 1.40 |
| 21 | 0.79 | 22 | 6.20 |
| 26 | 2.90 | 27 | 3.30 |
| 28 | 2.30 | 29 | 3.40 |

(continued)

| No. of compounds | $IC_{50}$ (nM) | No. of compounds | $IC_{50}$ (nM) |
|---|---|---|---|
| 33 | 0.34 | 34 | 1.50 |
| 35 | 2.20 | 36 | 0.45 |
| CC-90011 | 2.70 | | |

It can be known from the test data of the inhibitory activity of each compound on the LSD1 shown in Table 1 that, the compounds of the present application may have obvious LSD1 inhibitory activity.

**Test Example 2: Kasumi-1 cell proliferation inhibition test**

1. Test principle

Research had confirmed that LSD1 played a crucial role in the occurrence process of various types of cancers, including small cell lung cancer (SCLC) and acute myeloid leukemia (AML). Therefore, a human acute myeloid leukemia (Kasumi-1) cell line was used to determine the ATP content in cells at different drug concentrations using a Celltiter GLO assay kit, the cell activity was reflected by luminescence intensity, and $IC_{50}$ values of different compounds on Kasumi-1 cells were calculated using inhibition rates. The inhibitory effects of the compounds in the present application on the proliferation of Kasumi-1 cells were studied to evaluate the anti-tumor efficacy of the tested compounds at the cellular level.

2. Test materials

2.1. Compounds:

Active drug: CC-90011, Chengdu Hyperway Pharmaceuticals, batch number HBW-013-177-77;
Tested drugs: compounds 4, 16, 18 and 21.

2.2. Test reagents and instruments:

RPMI 1640, Gibico;
Penicillin-streptomycin: Penicillin-Streptomycin, Gibico;
Fetal bovine serum: Fetal Bovine Serum (FBS), Gibico;
Phosphate buffered saline: Phosphate Buffered Saline (PBS), Gibico;
Dimethyl sulfoxide: DMSO, Sigma;
CelltiterGlo assay Kit (CTG), Promega;
White 384-well cell culture plate, PerkinElmer;
Plate oscillator, QILINBE;
Centrifuge, Eppendorf;
$CO_2$ incubator, Thermo Scientific;
Microscope, OLYMMPUS;
Full-automatic cell counter, Gibco;
384-well Source plate, Echo Qualified 384-Well Polypropylene, LABCYTE;
Echo, LABCYTE;
Multifunctional microplate reader (Envision), PerkinElmer.

2.3. Test cell:

Kasumi-1 cells, purchased from ATCC.

3. Test method

3.1. Test steps

Day 1 Cells were seeded into the 384-well cell culture plate and placed in a 37°C 5% $CO_2$ cell incubator to be cultured overnight.

Day 0 The compound to be tested was prepared into a 10 mM mother liquid using DMSO, starting from 1 μM, diluted by 3 folds, with 10 concentration points and double duplicate wells. A diluted compound was added into the 384-well cell culture plate and placed in a 37°C 5% $CO_2$ cell incubator to be cultured for 7 days. The concentration of the DMSO in a cell culture system was 0.1 vol%.

Day 7 Celltiter GLO was added into the 384-well cell culture plate according to a volume ratio of culture system : Celltiter GLO = 1:1, light-shielded oscillation was carried out on a plate oscillator for 3 min, then the cell culture plate was placed at the room temperature for 30 min with light shielded, and the plate was read using a multifunctional microplate reader chemical light emitting module.

3.2. Data analysis

An inhibition rate was calculated by utilizing luminescence intensity of the above DMSO group (with DMSO added), test group (with the tested compound or a positive control solution added) and blank group (not treating cells), and a calculation formula was as follows:

$$\text{Inhibition percentage (\%)} = \frac{\text{DMSO group} - \text{test group}}{\text{DMSO group} - \text{blank group}} \times 100$$

Using a logarithm of the concentration as an X axis and the percentage inhibition rate as a Y axis, a dose-response curve was fitted using a formula log(inhibitor) vs. response-Variable slope (Four parameters) of analysis software Graphpad Prism 8 to obtain the $IC_{50}$ value of each compound on Kasumi-1 proliferation inhibition activity.

4. Test result

See Table 2 for Kasumi-1 cell growth inhibition activities of the tested compounds in the present application and positive controls.

Table 2 Kasumi-1 cell inhibition activities of tested compounds in the present application and positive controls

| No. of compounds | $IC_{50}$ (nM) | No. of compounds | $IC_{50}$ (nM) |
|---|---|---|---|
| 4 | 3.07 | 16 | 0.59 |
| 18 | 8.79 | 21 | 1.03 |
| CC-90011 | 3.18 | | |

It can be known from the test data of the Kasumi-1 cell inhibition activity of each compound shown in Table 2 that, the compounds of the present application may have a high inhibition effect on Kasumi-1 cell growth.

**Test Example 3: NCI-H1417 cell proliferation inhibition test**

1. Test principle

Research had confirmed that LSD1 played a crucial role in the occurrence process of various types of cancers, including small cell lung cancer (SCLC) and acute myeloid leukemia (AML). Therefore, a human small cell lung cancer (NCI-H1417) cell line was used to determine the ATP content in cells at different drug concentrations using a Celltiter GLO assay kit, and the cell activity was reflected by luminescence intensity. $IC_{50}$ values of different compounds on the NCI-H1417 cells were calculated using inhibition rates. The inhibitory effects of the compounds in the present application on the proliferation of the NCI-H1417 cells were studied to evaluate the anti-tumor efficacy of the tested compounds at the cellular level.

2. Test materials

2.1. Compounds:

Active drug: CC-90011, Chengdu Hyperway Pharmaceuticals, batch number HBW-013-177-77;
Tested drugs: compounds 4, 16, 18 and 21.

2.2. Test reagents and instruments:

RPMI 1640, Gibico;
Penicillin-streptomycin: Penicillin-Streptomycin, Gibico;
Fetal bovine serum: Fetal Bovine Serum (FBS), Gibico;
Phosphate buffered saline: Phosphate Buffered Saline (PBS), Gibico;
Dimethyl sulfoxide: DMSO, Sigma;
CelltiterGlo assay Kit (CTG), Promega;
White 384-well cell culture plate, PerkinElmer;
Plate oscillator, QILINBE;
Centrifuge, Eppendorf;
$CO_2$ incubator, Thermo Scientific;
Microscope, OLYMMPUS;
Full-automatic cell counter, Gibco;
384-well Source plate, Echo Qualified 384-Well Polypropylene, LABCYTE;
Echo, LABCYTE;
Multifunctional microplate reader (Envision), PerkinElmer.

2.3. Test cell:

NCI-H1417 cells, purchased from ATCC.
3. Test method

3.1. Test steps

Day 1 Cells were seeded into the 384-well cell culture plate and placed in a 37°C 5% $CO_2$ cell incubator to be cultured overnight.

Day 0 The compound to be tested was prepared into a 10 mM mother liquid using DMSO, starting from 1 μM, diluted by 3 folds, with 10 concentration points and double duplicate wells. A diluted compound was added into the 384-well cell culture plate and placed in a 37°C 5% $CO_2$ cell incubator to be cultured for 7 days. The concentration of the DMSO in a cell culture system was 0.1%.

Day 7 Celltiter GLO was added into the 384-well cell culture plate according to a volume ratio of culture system : Celltiter GLO = 1:1, light-shielded oscillation was carried out on a plate oscillator for 3 min, then the cell culture plate was placed at the room temperature for 30 min with light shielded, and the plate was read using a multifunctional microplate reader chemical light emitting module.

3.2. Data analysis

An inhibition percentage was calculated by utilizing luminescence intensity of the above DMSO group (with DMSO added), test group (with the tested compound and a positive control solution added) and blank group (not treating cells), a calculation formula being

$$\text{Inhibition percentage } (\%) = \frac{\text{DMSO group} - \text{test group}}{\text{DMSO group} - \text{blank group}} \times 100$$

Using a logarithm of the concentration as an X axis and the percentage inhibition rate as a Y axis, a dose-response curve was fitted using a formula log(inhibitor) vs. response-Variable slope (Four parameters) of analysis software Graphpad Prism 8 to obtain the $IC_{50}$ value of each compound on NCI-H1417 proliferation inhibition activity.

4. Test result

See Table 3 for NCI-H1417 cell growth inhibition activities of the tested compounds in the present application and positive controls.

Table 3 NCI-H1417 cell inhibition activities of tested compounds in the present application and positive controls

| No. of compounds | $IC_{50}$ (nM) | No. of compounds | $IC_{50}$ (nM) |
|---|---|---|---|
| 4 | 16.74 | 16 | 2.79 |
| 18 | 33.30 | 21 | 6.51 |
| 36 | 4.00 | CC-90011 | 22.88 |

It can be known from the test data of the NCI-H1417 cell inhibition activity of each compound shown in Table 3 that, the compounds of the present application may have a high inhibition effect on NCI-H1417 cell growth.

**Test Example 4: Study on pharmacokinetics of oral administration of rats**

1. Test principle

With SD rats as tested animals, blood drug concentrations in plasma at different moments after oral administration of the compounds of the present application by the rats in a clarification kit mode were determined using an LC-MS/MS method. Pharmacokinetic parameters of the compounds of the present application in the rats were obtained, and pharmacokinetic characteristics of the compounds were studied.

2. Test materials

2.1. Compounds:

Active drug: CC-90011, Chengdu Hyperway Pharmaceuticals, batch number HBW-013-177-77; Tested drugs: compounds 1, 4, 14-16, 18, 20, 21 and 36.

2.2. Test instruments:

Shimadzu LC-30A AB API4500 tandem mass spectrometer, vacuum blood collection tube, blood taking needle, filter paper, syringe, etc.

2.3. Test animals

SD rats, male, weight 180-220 g, 3 rats per group. The animals were fed in an animal house after being purchased, an adaptation period was at least 3 days, and they were used for tests after passing quarantine.

3. Test method

3.1. Grouping: random grouping was carried out according to Table 4, and after grouping, there was no statistical difference in weight of the SD rats between groups.

Table 4 Test groups and administration regimens

| Group | No. of compounds | Administration route | Administration dosage (mg/kg) | Administration concentration (mg/mL) | Administration volume (mL/kg) |
|---|---|---|---|---|---|
| Positive group | CC-90011 | p.o. | 5.0 | 0.5 | 10.0 |
| Example 1 group | Compound 1 | p.o. | 5.0 | 0.5 | 10.0 |
| Example 4 group | Compound 4 | p.o. | 5.0 | 0.5 | 10.0 |
| Example 14 group | Compound 14 | p.o. | 5.0 | 0.5 | 10.0 |
| Example 15 group | Compound 15 | p.o. | 5.0 | 0.5 | 10.0 |
| Example 16 group | Compound 16 | p.o. | 5.0 | 0.5 | 10.0 |
| Example 18 group | Compound 18 | p.o. | 5.0 | 0.5 | 10.0 |
| Example 20 group | Compound 20 | p.o. | 5.0 | 0.5 | 10.0 |
| Example 21 group | Compound 21 | p.o. | 5.0 | 0.5 | 10.0 |
| Example 36 group | Compound 36 | p.o. | 5.0 | 0.5 | 10.0 |

3.2. Blood specimen collection and determination:

According to Table 4, each group of rats was given the corresponding tested drugs or positive drugs by gavage, before administration, and 15 min, 30 min, 1 h, 2 h, 4 h, 6 h, 8 h, and 24 h after administration, a fixed volume of blood was collected through eye sockets and placed in an EDTA-K2 anticoagulant tube, centrifuging was carried out at 8000 rpm for 1 min to separate plasma into a centrifuge tube, and the plasma was frozen in a -20°C refrigerator.

3.3. Analysis method

The plasma at each time point stored at -20°C was taken out, then acetonitrile was added, after whirling at 1500 revolutions for 2 min, centrifuging was carried out for 15 min (3500 r/min), and a fixed volume of solution supernatant was taken for LC-MS/MS analysis.

4. Pharmacokinetic parameter calculation

Non-compartment model fitting was carried out on pharmacokinetic behaviors of the tested compounds, and DAS3.31 software was adopted to calculate main pharmacokinetic parameters ($T_{1/2}$, $T_{max}$, $C_{max}$, $AUC_{last}$, etc.).

5. Test result

Table 5 Pharmacokinetic parameters of example compounds

| No. of compounds | $C_{max}$ (ng/mL) | $T_{max}$ (h) | $T_{1/2}$(h) | $AUC_{last}$ (ng/mL *h) |
|---|---|---|---|---|
| CC-90011 | 48.93±12.52 | 3.33±2.31 | 1.44±0.52 | 355.51±46.46 |
| Compound 1 | 93.94±19.11 | 6.00±0.00 | 7.64±2.46 | 1299.96±411.26 |
| Compound 4 | 55.00±7.72 | 6.00±0.00 | 5.31±0.24 | 711.92±89.33 |
| Compound 14 | 129.66±7.90 | 4.67±2.31 | 5.97±0.29 | 1468.45±44.61 |
| Compound 15 | 124.52±25.76 | 6.00±0.00 | 5.73±0.80 | 1547.52±251.89 |
| Compound 16 | 69.87±33.48 | 4.00±0.00 | 3.46±0.02 | 560.51±346.63 |
| Compound 18 | 77.18±32.12 | 4.00±0.00 | 2.94±0.11 | 591.26±291.62 |
| Compound 20 | 126.03±23.94 | 4.67±1.15 | 4.66±0.73 | 1410.52±293.29 |
| Compound 21 | 81.36±17.01 | 4.67±1.15 | 2.84±0.59 | 720.92±125.18 |

(continued)

| No. of compounds | $C_{max}$ (ng/mL) | $T_{max}$ (h) | $T_{1/2}$(h) | $AUC_{last}$ (ng/mL *h) |
|---|---|---|---|---|
| Compound 36 | 100.00±54.43 | 4.67±1.15 | 3.57±0.34 | 768.87±360.94 |

It can be seen from the test results in Table 5 that compared to the positive group (CC-90011), the compound 1, compound 4, compound 14-16, compound 18, compound 20-21, and compound 36 administration groups are superior to CC-90011 in terms of plasma exposure and the like, indicating that the pharmacokinetic properties of the compounds prepared in Examples 1, 4, 14, 15, 16, 18, 20, 21 and 36 of the present application are significantly improved compared to CC-90011. Therefore, the compounds of the present application can exhibit good pharmacokinetic properties.

**Test Example 5: Study on pharmacokinetics of oral administration and intravenous administration of rats**

1. Test principle

With SD rats as tested animals, blood drug concentrations of the compounds of the present application in plasma at different moments after oral administration and intravenous administration to the rats were determined using an LC-MS/MS method, pharmacokinetic parameters and bioavailabilities of the compounds of the present application in the rats were obtained, and pharmacokinetic characteristics of the compounds were studied.

2. Test materials

2.1. Compounds:

Active drug: CC-90011, Chengdu Hyperway Pharmaceuticals, batch number HBW-013-177-77;
Tested drug: compound 16;

2.2. Test instruments:

Shimadzu LC-30A AB API4500 tandem mass spectrometer, vacuum blood collection tube, blood taking needle, filter paper, syringe, etc.

2.3. Test animals

SD rats, male, weight 180-220 g, 3 rats per group. The animals were fed in an animal house after being purchased, an adaptation period was at least 3 days, and they were used for tests after passing quarantine.

3. Test method

3.1. Grouping: random grouping was carried out according to Table 6, and after grouping, there was no statistical difference in weight of the SD rats between groups.

3.2. Solvent: p.o.: 0.5% sodium methylcellulose aqueous solution

i.v.: 5% DMSO + 10% Solutol + 85% Saline

Table 6 Test groups and administration regimens

| Group | No. of compounds | Administration route | Administration dosage (mg/kg) | Administration concentration (mg/mL) | Administration volume (mL/kg) |
|---|---|---|---|---|---|
| Positive group | CC-90011 | p.o. | 5.0 | 0.5 | 10.0 |
| | CC-90011 | i.v. | 5.0 | 0.5 | 10.0 |

(continued)

| Group | No. of compounds | Administration route | Administration dosage (mg/kg) | Administration concentration (mg/mL) | Administration volume (mL/kg) |
|---|---|---|---|---|---|
| Example 16 group | Compound 16 | p.o. | 5.0 | 0.5 | 10.0 |
| | Compound 16 | i.v. | 5.0 | 0.5 | 10.0 |

3.2. Blood specimen collection and determination:

According to Table 6, each group of rats was given the corresponding tested drugs or positive drugs by gavage, before administration, and 5 min, 15 min, 30 min, 1 h, 2 h, 4 h, 6 h, 8 h, 10 h and 24 h after administration, a fixed volume of blood was collected through eye sockets and placed in an EDTA-K2 anticoagulant tube, centrifuging was carried out at 8000 rpm for 1 min to separate plasma into a centrifuge tube, and the plasma was frozen in a -20°C refrigerator.

3.3. Analysis method

The plasma at each time point stored at -20°C was taken out, then acetonitrile was added, after whirling at 1500 revolutions for 2 min, centrifuging was carried out for 15 min (3500 r/min), and a fixed volume of solution supernatant was taken for LC-MS/MS analysis.

4. Pharmacokinetic parameter calculation:

Non-compartment model fitting was carried out on pharmacokinetic behaviors of the tested compounds, and DAS3.31 software was adopted to calculate main pharmacokinetic parameters ($T_{1/2}$, $T_{max}$, $C_{max}$, $AUC_{last}$, etc.).

5. Test result:

Table 7 Pharmacokinetic parameters of example compounds

| No. of compounds | Administration method | $C_{max}$ (ng/mL) | $T_{max}$ (h) | $T_{1/2}$ (h) | $AUC_{last}$ (ng/mL*h) | F (%) |
|---|---|---|---|---|---|---|
| CC-90011 | p.o. | 49.20±8.71 | 2.67±1.15 | 4.15±0.70 | 299.05±177.57 | 32.06 |
| | i.v. | 894.55±90.37 | 0.08±0.00 | 3.32±0.72 | 906.36±193.79 | |
| Compound 16 | p.o. | 75.23±20.26 | 4.00±0.00 | 4.73±0.22 | 692.67±247.98 | 46.21 |
| | i.v. | 765.10±180.85 | 0.08±0.00 | 5.61±1.29 | 1491.37±157.26 | |

It can be seen from the test results in Table 7 that, under the same dosage, the compound 16 is superior to CC-90011 in terms of plasma exposure, maximum blood drug concentration, bioavailability and the like of oral administration and intravenous administration, indicating that pharmacokinetic properties of the compound prepared in Example 16 of the present application are obviously improved compared to CC-90011.

**Test Example 6: Study on pharmacokinetics of oral administration of mice**

1. Test principle

With Balb/c mice as tested animals, blood drug concentrations of the compounds of the present application in plasma at different moments after administration of the mice under different solvent conditions were determined using an LC-MS/MS method. Pharmacokinetic parameters of the compounds of the present application in the mice under the different solvent conditions were obtained, and pharmacokinetic characteristics of the compounds were studied.

2. Test materials

2.1. Compounds:

Active drug: CC-90011, Chengdu Hyperway Pharmaceuticals, batch number HBW-013-177-77;
Tested drug: compound 16.

2.2. Test instruments:

Shimadzu LC-30A AB API4500 tandem mass spectrometer, vacuum blood collection tube, blood taking needle, filter paper, syringe, etc.

2.3. Test animals

Balb/c mice, male, weight 18-22 g, 6 mice per group. The animals were fed in an animal house after being purchased, an adaptation period was at least 3 days, and they were used for tests after passing quarantine.

3. Test method

3.1. Grouping: random grouping was carried out according to Table 8, and after grouping, there was no statistical difference in weight of the Balb/c mice between groups.

Table 8 Test groups and administration regimens

| Solvent | Group | No. of compounds | Administration route | Administration dosage (mg/kg) | Administration concentration (mg/mL) | Administration volume (mL/kg) |
|---|---|---|---|---|---|---|
| 5% DMSO + 10% Solutol + 85% Saline | Positive group | CC-90011 | p.o. | 5.0 | 0.5 | 10.0 |
| | Example 16 group | Compound 16 | p.o. | 5.0 | 0.5 | 10.0 |
| 0.5% sodium methylcellulose aqueous solution | Positive group | CC-90011 | p.o. | 5.0 | 0.5 | 10.0 |
| | Example 16 group | Compound 16 | p.o. | 5.0 | 0.5 | 10.0 |

3.2. Blood specimen collection and determination:

According to Table 8, each group of mice was given the corresponding tested drugs or positive drugs by gavage, before administration, and 15 min, 30 min, 1 h, 2 h, 4 h, 6 h, 8 h, 10 h and 24 h after administration, a fixed volume of blood was collected through eye sockets and placed in an EDTA-K2 anticoagulant tube, centrifuging was carried out at 8000 rpm for 1 min to separate plasma into a centrifuge tube, and the plasma was frozen in a -20°C refrigerator.

3.3. Analysis method

The plasma at each time point stored at -20°C was taken out, then acetonitrile was added, after whirling at 1500 revolutions for 2 min, centrifuging was carried out for 15 min (3500 r/min), and a fixed volume of solution supernatant was taken for LC-MS/MS analysis.

4. Pharmacokinetic parameter calculation:

Non-compartment model fitting was carried out on pharmacokinetic behaviors of the tested compounds, and DAS3.31 software was adopted to calculate main pharmacokinetic parameters ($T_{1/2}$, $T_{max}$, Cmax, $AUC_{last}$, etc.).

5. Test result:

Table 9 Pharmacokinetic parameters of example compounds

| Solvent | No. of compounds | $C_{max}$ (ng/mL) | $T_{max}$ (h) | $T_{1/2}$ (h) | $AUC_{last}$ (ng/mL*h) |
|---|---|---|---|---|---|
| 5% DMSO + 10% Solutol + 85% Saline | CC-90011 | 172.68±50.92 | 1.00±0.00 | 2.44±0.34 | 870.87±270.25 |
| | Compound 16 | 611.74±111.04 | 2.67±1.15 | 5.52±0.67 | 2415.34±122.78 |
| 0.5% sodium methylcellulose aqueous solution | CC-90011 | 200.13±19.27 | 3.33±1.15 | 5.01±0.04 | 964.79±120.13 |
| | Compound 16 | 894.54±498.71 | 3.33±1.15 | 3.99±0.59 | 3222.97±1512.16 |

It can be seen from the test results in Table 9 that, compared to the positive group (CC-90011), the compound 16 is superior to CC-90011 in terms of plasma exposure, maximum blood drug concentration and the like under different solvent conditions, indicating that pharmacokinetic properties of the compound prepared in Example 16 of the present application are obviously improved compared to CC-90011, and the compound has pharmacokinetic advantages under the different solvent conditions.

**Test Example 7: Liver microsome stability testing experiment**

1. Test principle

A liver microsome in-vitro warm incubation method used liver microsomes, supplemented by an NADPH regeneration system, to simulate physiological environmental conditions in vitro for metabolic reaction, after a certain period of reaction, LC-MS/MS was used to determine prototype drugs and metabolites in a warm incubation solution, and the content was preliminarily analyzed. Studying the liver microsome stability of drugs in different species was crucial for understanding a change process of the drugs in vivo.

2. Test materials

2.1. Liver microsomes in different species including rats, mice, humans, dogs and monkeys

2.2. Compounds:

Active drug: CC-90011, Chengdu Hyperway Pharmaceuticals, batch number HBW-013-177-77;
Tested drug: compounds 16 and 36.
Tested positive reference compounds: testosterone, diclofenac, and propafenone

2.3. Test instruments:

Shimadzu LC-30A AB API4500 tandem mass spectrometer, electric heating constant temperature shaker, multi-tube vortex oscillator, and pipettes of respective ranges.

3. Test method

According to the requirements of the instructions of a Phase I Metabolic Stability Kit, a liver microsome incubation system was prepared, and according to a sequence, a mixed system of a PBS, liver microsomes, an NADPH regeneration system solution A and an NADPH regeneration system solution B, as well as substances to be tested were added in sequence respectively, and were incubated at 37°C. Each sample was parallelized 3 times, with a sample without an NADPH generation system as a negative control, after 0, 5, 15, 30, 45, and 60 min, equal volumes of pre-cooled acetonitrile were added to terminate the reaction respectively, and the prototype drug content in a warm incubation solution was determined by LC-MS/MS.

4. Test result

Table 10 Liver microsome stability test results of compounds in different species

| | Humans | | | Rats | | |
|---|---|---|---|---|---|---|
| | **$T_{1/2}$** (min) | **CLint (liver)** (mL/min/kg) | **Remaining** (T=60 min) | **$T_{1/2}$** (min) | **CLint (liver)** (mL/min/kg) | **Remaining** (T=60 min) |
| Compound 16 | >145 | <8.6 | 70.8% | >145 | <17.3 | 72.1% |
| Compound 36 | >145 | <8.6 | 81.7% | >145 | <17.3 | 76.1% |
| CC-90011 | 94.4 | 13.2 | 67.2% | 80.0 | 31.2 | 58.6% |
| | Mice | | | Dogs | | |
| | **$T_{1/2}$** (min) | **CLint (liver)** (mL/min/kg) | **Remaining** (T=60 min) | **$T_{1/2}$** (min) | **CLint (liver)** (mL/min/kg) | **Remaining** (T=60 min) |
| Compound 16 | >145 | <38.0 | 77.8% | >145 | <13.8 | 76.4% |
| Compound 36 | >145 | <38.0 | 75.5% | >145 | <13.8 | 76.3% |
| CC-90011 | 130.0 | 42.2 | 72.2% | 126.2 | 15.8 | 76.6% |

| | Monkeys | | |
|---|---|---|---|
| | **$T_{1/2}$** (min) | **CLint (liver)** (mL/min/kg) | **Remaining** (T=60 min) |
| Compound 16 | 97.3 | 19.2 | 62.0% |
| Compound 36 | 110.5 | 16.9 | 72.1% |
| CC-90011 | 75.0 | 25.0 | 56.8% |

The above experiment results indicate that the compounds 16 and 36 have high metabolic stability in liver microsomes of humans, rats, mice, dogs and monkeys. Therefore, the compounds of the present application can exhibit good druggability.

**Test Example 8: In-vitro hERG inhibitory activity determining experiment**

1. Test principle

A rapidly-activated human delay rectifier outward potassium current was mainly mediated by an hERG ion channel and participated in human myocardial cell repolarization. Drug blocking of this current was the main cause of prolonged QT interval syndrome, even acute arrhythmia and sudden death in clinical practice. A whole cell sheet technology was used to detect the blocking effects of compounds on hERG channels on HEK293 cells stably expressing hERG channels, and a median inhibitory concentration $IC_{50}$ of the compound was determined. As part of cardiac safety evaluation, its safety in-vitro screening for cardiac toxicity was preliminarily evaluated.

2. Test materials

2.1. Test cell line:

HEK293 cells stably expressing hEGR ion channels

2.2. Compounds:

Active drug: CC-90011, Chengdu Hyperway Pharmaceuticals, batch number HBW-013-177-77;

Tested drug: compound 16.

2.3. Test instruments:

Patch clamp instrument: PC-505B
Micromanipulator: MP-225
Instrument for drawing electrodes: PC-10 (Narishige, Japan)

3. Test method

Cells were transferred into a perfusion trough, 137 mM NaCl, 4 mM KCl, 1.8 mM $CaCl_2$, 1 mM $MgCl_2$, 10 mM HEPES and 10 mM glucose were adopted to prepare an extracellular fluid, and NaOH was used to adjust to 7.4 (pH) for perfusion. Electrodes were drawn using PC-10 (Narishige, Japan). The whole cell patch clamp was used for recording, and noise was filtered using one fifth of a sampling frequency. The cells were clamped at -80 mV, then depolarized to 40 mV using square waves lasting for 4 seconds, and then hyperpolarized to -40 mV using square waves lasting for 2 seconds to obtain an hERG tail current. This procedure was repeated every 20 seconds. The hERG tail current was a pure hERG current. A maximum current triggered by the second square wave was detected, after the maximum current was stable, the compounds were tested through perfusion, and after reaction was stable, a blocking intensity was calculated.

4. Test result

Table 11 $IC_{50}$ values of compounds on hERG current

| No. of compounds | $IC_{50}$ (μM) |
|---|---|
| Compound 16 | >10 |
| CC-90011 | 6.48 |

The above experiment results indicate that compared to a positive phase (CC-90011), an hERG inhibition $IC_{50}$ value of the compound 16 is greater than 10 μM, indicating that the compound of the present application has the lower cardiac toxicity risk than a positive control.

**Test Example 9: In-vivo efficacy test in Kasumi-1 cell subcutaneous heterograft tumor model**

1. Test method

1.1. Cell culture

RPMI 1640 culture medium containing 20% fetal bovine serum (FBS), 37°C, 5% $CO_2$.

1.2. Compounds:

Solvent: 0.5% methylcellulose aqueous solution;
Tested drug: compounds 16 and 36.

1.3. Experimental process

CB17 SCID mice, female, 6-8 weeks, weight about 18-22 grams, and each mouse was subcutaneously inoculated with 0.1 mL ($1\times10^7$ cells + matrigel) of Kasumi-1 cells on the right side. When an average volume of tumors reached 100-200 cubic millimeters, group administration was performed, see the table below (Table 12) for administration dosages and methods. The tumor volume was measured twice a week, the weights of the mice as well as lengths and widths of tumors were recorded in each time of measurement, and the tumor volume = length × width × width/2 $mm^3$.

When the average tumor volume of a solvent group grew to 3000 cubic millimeters or above, administration was ended, each group of mice was killed and dissected to take out tumor lumps, the tumor weight of each mouse was weighed and the tumor volume was measured, and an average tumor volume difference between a tested compound group and the solvent group was obtained via comparison. The tumor suppression effects of the compounds were evaluated with TGI(%), which could reflect a tumor growth inhibition rate.

Calculation of TGI(%): TGI(%)=[1-(current average tumor volume of compounds - average tumor volume at the beginning of compounds)/(current average tumor volume of solvent group - average tumor volume at the beginning of solvent group)]×100%

The changes in tumor volume and tumor weight of the mice in each group were shown in FIG. 1 and FIG. 2.

2. Test result

Table 12 In-vivo tumor suppression experimental data

| Group | Number of animals | Administration method | Administration dosage (mg/Kg) | Administration days | TGI(%) |
|---|---|---|---|---|---|
| Solvent group | 5 | qd, p.o. | / | 20 | / |
| Compound 16 | 5 | qd, p.o. | 3.5 | 20 | 78.28 |
| Compound 16 | 5 | qd, p.o. | 5 | 20 | 86.13 |
| Compound 36 | 5 | qd, p.o. | 5 | 20 | 84.87 |

3. Test conclusion

The above test results indicate that the compounds 16 and 36 of the present application exhibit good in-vivo efficacy in a human acute myeloid leukemia Kasumi-1 cell subcutaneous heterograft tumor model, with a significant tumor suppression effect (TGI>60%), the tested animals have good tolerance to the compounds 16 and 36, and after administration, the weight of the mice does not decrease (as shown in FIG. 3). Therefore, the compounds of the present application can exhibit good in-vivo tumor suppression effects.

**Test Example 10: In-vivo efficacy test in HL-60 cell subcutaneous heterograft tumor model**

1. Test method

1.1. Cell culture

DMEM culture medium containing 10% fetal bovine serum (FBS), 37°C, 5% $CO_2$.

1.2. Compounds:

Active drug: CC-90011, Chengdu Hyperway Pharmaceuticals, batch number HBW-013-203-33;
Solvent: 0.5% methylcellulose aqueous solution;
Tested drug: compound 16.

1.3. Experimental process

Balb/C nu mice, female, 6-8 weeks, weight about 18-22 grams, and each mouse was subcutaneously inoculated with 0.1 mL ($1\times10^7$ cells + matrigel) of HL-60 cells on the right side. When an average volume of tumors reached 100-200 cubic millimeters, group administration was performed, see the table below (Table 13) for administration dosages and methods. The tumor volume was measured twice a week, the weights of the mice as well as lengths and widths of tumors were recorded in each time of measurement, and the tumor volume = length × width × width/2 $mm^3$. When the average tumor volume of a solvent group grew to 3000 cubic millimeters or above, administration was ended, and an average tumor volume difference between a tested compound group and the solvent group was obtained via comparison. The tumor

suppression effects of the compounds were evaluated with TGI(%), which could reflect a tumor growth inhibition rate.

Calculation of TGI(%): TGI(%)=[1-(current average tumor volume of compounds - average tumor volume at the beginning of compounds)/(current average tumor volume of solvent group - average tumor volume at the beginning of solvent group)] $\times$ 100%

The changes in tumor volume of the mice in each group were shown in FIG. 4.

2. Test result

Table 12 In-vivo tumor suppression experimental data

| Group | Number of animals | Administration method | Administration dosage (mg/Kg) | Administration days | TGI(%) |
|---|---|---|---|---|---|
| Solvent group | 5 | qd, p.o. | / | 10 | / |
| CC-90011 | 5 | qd, p.o. | 10 | 10 | 35.3 |
| Compound 16 | 5 | qd, p.o. | 10 | 10 | 73.2 |

3. Test conclusion

The above test results indicate that, compared to the positive compound CC-90011, the compounds of the present application exhibit good in-vivo efficacy in a human myeloid leukemia HL-60 cell subcutaneous heterograft tumor model, with a significant tumor suppression effect (TGI>60%), the tested animals have good tolerance to the compounds of the present application, and after administration, the weight of the mice does not decrease (as shown in FIG. 5).

It should be understood that the above detailed description and accompanying examples are only illustrative and should not be considered as limiting the scope of the present application, which is solely defined by the accompanying claims and their equivalents. Those skilled in the art can easily see various changes and modifications to the disclosed embodiments. Such changes and modifications may be made without departing from its spirit and scope, including but not limited to those related to the chemical structures, substituents, derivatives, intermediates, synthesis methods, formulations, and/or methods of use of the present application. All publications, patents, and patent applications referred to herein are hereby incorporated for various purposes.

## Claims

**1.** A compound of formula I, and a stereoisomer or a pharmaceutically acceptable salt thereof,

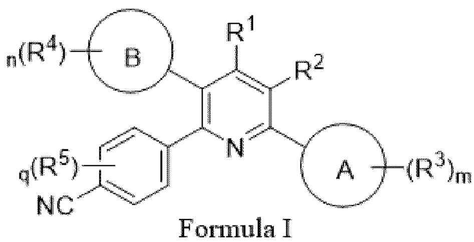

Formula I

wherein a ring A is selected from the group consisting of saturated cycloalkyl, saturated heterocycloalkyl, unsaturated cyclohydrocarbyl and unsaturated heterocyclohydrocarbyl, wherein the cycloalkyl, the heterocycloalkyl, the cyclohydrocarbyl or the heterocyclohydrocarbyl is selected from the group consisting of a monocyclic bridged ring, a dicyclic bridged ring and a dicyclic spiro;

wherein a ring B is selected from the group consisting of unsaturated cyclohydrocarbyl and unsaturated heterocyclohydrocarbyl, wherein the unsaturated cyclohydrocarbyl and the unsaturated heterocyclohydrocarbyl are selected from the group consisting of a monocyclic ring and a dicyclic ring;

wherein $R^1$ and $R^2$ are each independently selected from the group consisting of hydrogen, fluorine, cyano,

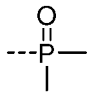, 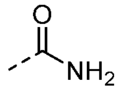 and 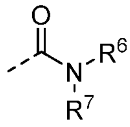

, and $R^1$ and $R^2$ are not hydrogen or fluorine at the same time, and when $R^1$ is hydrogen, $R^2$ is not fluorine, and when $R^1$ is fluorine, $R^2$ is not hydrogen; or $R^1$ and $R^2$ together with carbon atom to which they are connected form a ring structure selected from

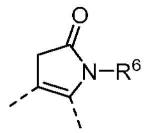 or 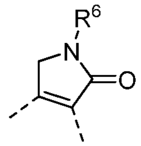;

wherein $R^3$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy;

wherein $R^4$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy;

wherein $R^5$ is selected from the group consisting of halogen, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy;

wherein $R^6$ and $R^7$ are each independently selected from the group consisting of hydrogen, $C_1$-$C_6$ alkyl and $C_3$-$C_8$ cycloalkyl;

wherein m is selected from the group consisting of 0, 1, 2, 3, 4 and 5;

wherein n is selected from the group consisting of 0, 1, 2, 3, 4 and 5; and

wherein q is selected from the group consisting of 0, 1, 2, 3 and 4.

**2.** The compound of formula I, and the stereoisomer or the pharmaceutically acceptable salt thereof according to claim 1, wherein

the ring A is selected from the group consisting of $C_4$-$C_{13}$ cycloalkyl and 3- to 13- membered heterocycloalkyl, and the heterocycloalkyl contains 1-3 heteroatoms selected from the group consisting of a nitrogen atom, an oxygen atom and a sulfur atom;

the ring B is selected from the group consisting of $C_5$-$C_{10}$ cycloalkenyl, 5- to 10- membered heterocycloalkenyl, $C_5$-$C_{10}$ aryl and 5- to 10- membered heteroaryl, and the heterocycloalkenyl or the heteroaryl contain 1-3 heteroatoms selected from the group consisting of a nitrogen atom, an oxygen atom and a sulfur atom;

$R^1$ and $R^2$ are each independently selected from the group consisting of hydrogen, cyano,

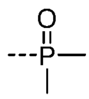,

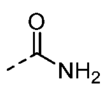 and 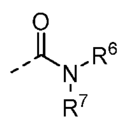

, or $R^1$ and $R^2$ together with carbon atom to which they are connected form a ring structure selected from

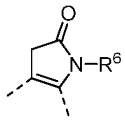 or 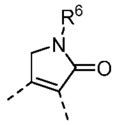 ;

and $R^6$ and $R^7$ are each independently selected from the group consisting of hydrogen, $C_1$-$C_6$ alkyl and $C_3$-$C_6$ cycloalkyl;

m is 0, 1, 2, 3, 4 or 5, and $R^3$ is selected from the group consisting of halogen, oxo, hydroxyl, substituted or unsubstituted amino, $C_1$-$C_6$ alkyl and $C_1$-$C_6$ alkoxy, wherein the $C_1$-$C_6$ alkyl or the $C_1$-$C_6$ alkoxy is unsubstituted or is substituted with one or more substituents selected from the group consisting of halogen, cyano, hydroxyl and amino;

n is selected from the group consisting of 0, 1, 2, 3, 4 and 5, and $R^4$ is selected from the group consisting of halogen, oxo, hydroxyl, $C_1$-$C_6$ alkyl, $C_1$-$C_6$ alkoxy, $C_3$-$C_6$ cycloalkyl and $C_3$-$C_6$ cycloalkyloxy, wherein the $C_1$-$C_6$ alkyl, the $C_1$-$C_6$ alkoxy, the $C_3$-$C_6$ cycloalkyl or the $C_3$-$C_6$ cycloalkyloxy is unsubstituted or is substituted with one or more substituents selected from the group consisting of halogen, hydroxyl and amino; and

q is selected from the group consisting of 0, 1 and 2, and $R^5$ is selected from the group consisting of halogen, hydroxyl, amino, $C_1$-$C_6$ alkyl and $C_1$-$C_6$ alkoxy, wherein the $C_1$-$C_6$ alkyl or the $C_1$-$C_6$ alkoxy is unsubstituted or is substituted with one or more substituents selected from the group consisting of halogen and hydroxyl.

**3.** The compound of formula I, and the stereoisomer or the pharmaceutically acceptable salt thereof according to claim 1 or 2, wherein

the ring A is selected from the group consisting of $C_5$-$C_7$ monocycloalkyl, 3- to 8- membered monoheterocycloalkyl and 8- to 12- membered spiroheterocycloalkyl, and the heterocycloalkyl contains 1-3 heteroatoms selected from the group consisting of a nitrogen atom and an oxygen atom;

the ring B is selected from the group consisting of $C_5$-$C_8$ cycloalkenyl and 8- to 10- membered heterocycloalkenyl, and the heterocycloalkenyl contains 1-3 heteroatoms selected from the group consisting of a nitrogen atom, an oxygen atom and a sulfur atom;

$R^1$ is selected from the group consisting of hydrogen, cyano,

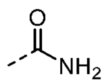 and 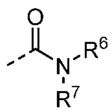 ,

$R^2$ is selected from the group consisting of hydrogen, cyano,

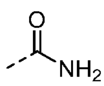 and 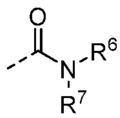 ,

or $R^1$ and $R^2$ together with carbon atom to which they are connected form a ring structure selected from

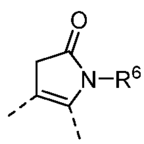

or

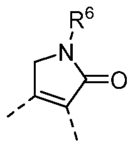 ;

and $R^6$ and $R^7$ are each independently selected from the group consisting of hydrogen, $C_1$-$C_5$ alkyl and $C_3$-$C_5$ cycloalkyl;

m is 0, 1, 2 or 3, and $R^3$ is selected from the group consisting of fluorine, chlorine, bromine, oxo, hydroxyl, amino and $C_1$-$C_6$ alkyl, wherein the $C_1$-$C_6$ alkyl is unsubstituted or is substituted with amino;

n is selected from the group consisting of 0, 1, 2, 3 and 4, and $R^4$ is selected from the group consisting of fluorine, chlorine, bromine, oxo, hydroxyl, $C_1$-$C_6$ alkyl and $C_1$-$C_6$ alkoxy, wherein the $C_1$-$C_6$ alkyl or the $C_1$-$C_6$ alkoxy is unsubstituted or is substituted with one or more substituents selected from the group consisting of halogen and hydroxyl; and

q is 1 or 2, and $R^5$ is halogen.

**4.** The compound of formula I, and the stereoisomer or the pharmaceutically acceptable salt thereof according to claim 1, wherein

the ring A is selected from the group consisting of 5- to 8- membered saturated monocycloalkyl, and 4-to 10-membered saturated monocyclic heterocycloalkyl or spiroheterocycloalkyl, wherein the heterocycloalkyl contains 1-3 nitrogen atoms as heteroatoms;

the ring B is selected from the group consisting of 5- to 8- membered unsaturated monocyclohydrocarbyl, and 5- to 14- membered unsaturated monocyclic heterocyclohydrocarbyl or dicyclic heterocyclohydrocarbyl, wherein the heterocyclohydrocarbyl contains 0-4 nitrogen atoms and 0-2 oxygen atoms as heteroatoms and contains at least one selected from the group consisting of nitrogen and oxygen;

$R^1$ and $R^2$ are each independently selected from the group consisting of hydrogen, fluorine, cyano,

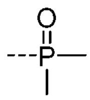 , 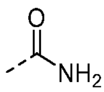 and 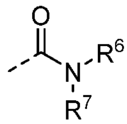 ,

$R^1$ and $R^2$ are not hydrogen or fluorine at the same time, when $R^1$ is hydrogen, $R^2$ is not fluorine, and when $R^1$ is fluorine, $R^2$ is not hydrogen; or $R^1$ and $R^2$ together with carbon atom to which they are connected form a ring structure selected from

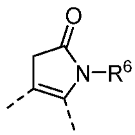

or

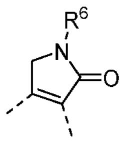 ;

$R^3$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy;

$R^4$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy;

$R^5$ is selected from the group consisting of halogen, hydroxyl, substituted or unsubstituted amino, $C_1$-$C_6$ alkyl, $C_1$-$C_6$ alkoxy, $C_3$-$C_8$ cycloalkyl and $C_3$-$C_8$ wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy;

$R^6$ and $R^7$ are each independently selected from the group consisting of hydrogen, $C_1$-$C_6$ alkyl and $C_3$-$C_8$

cycloalkyl;
m is selected from the group consisting of 0, 1, 2, 3, 4 and 5;
n is selected from the group consisting of 0, 1, 2, 3, 4 and 5; and
q is selected from the group consisting of 0, 1, 2, 3 and 4.

**5.** The compound of formula I, and the stereoisomer or the pharmaceutically acceptable salt thereof according to claim 1 or 4, wherein

the ring A is 4- to 10- membered saturated monocyclic heterocycloalkyl or spiroheterocycloalkyl, wherein the heterocycloalkyl contains 1-3 nitrogen atoms as heteroatoms, preferably 1-2 nitrogen atoms as the heteroatoms;
the ring B is selected from the group consisting of 6-membered unsaturated monocyclohydrocarbyl, and 6- to 10- membered unsaturated dicyclic heterocyclohydrocarbyl, wherein the heterocyclohydrocarbyl contains 0-4 nitrogen atoms and 0-2 oxygen atoms as heteroatoms and contains at least one selected from the group consisting of nitrogen and oxygen; and preferably, the ring B is 6- to 10- membered aryl or heterocycloaryl, wherein the heterocycloaryl contains 1-2 nitrogen atoms and 0-2 oxygen atoms as heteroatoms and contains at least one selected from the group consisting of nitrogen and oxygen;
$R^1$ and $R^2$ are each independently selected from the group consisting of hydrogen, fluorine, cyano,

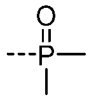, 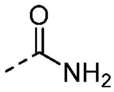 and 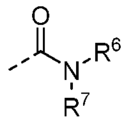,

$R^1$ and $R^2$ are not hydrogen or fluorine at the same time when $R^1$ is hydrogen, $R^2$ is not fluorine, and when $R^1$ is fluorine, $R^2$ is not hydrogen; or $R^1$ and $R^2$ together with carbon atom to which they are connected form a ring structure selected from

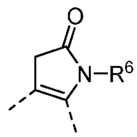

or

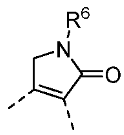;

$R^3$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy; and preferably, $R^3$ is selected from the group consisting of fluorine, chlorine, oxo, hydroxyl, amino and 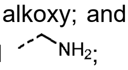;
$R^4$ is selected from the group consisting of halogen, cyano, oxo, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$ cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy; and preferably, $R^4$ is selected from the group consisting of fluorine, chlorine, oxo, methyl, methoxy,

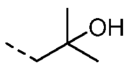 and 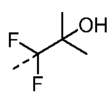;

$R^5$ is selected from the group consisting of halogen, hydroxyl, substituted or unsubstituted amino, $C_1$-$C_6$ alkyl, $C_1$-$C_6$ alkoxy, $C_3$-$C_8$ cycloalkyl and $C_3$-$C_8$ cycloalkyloxy, wherein the $C_1$-$C_6$ alkyl, the $C_1$-$C_6$ alkoxy, the $C_3$-$C_8$

cycloalkyl or the $C_3$-$C_8$ cycloalkyloxy is unsubstituted or is substituted with one or more substituents selected from the group consisting of halogen, cyano, hydroxyl, amino, $C_1$-$C_6$ alkyl and $C_1$-$C_6$ alkoxy; and preferably, $R^5$ is selected from the group consisting of fluorine, chlorine, bromine, methyl, methoxy, trifluoromethyl and trifluoromethoxy;

$R^6$ and $R^7$ are each independently selected from the group consisting of hydrogen, $C_1$-$C_6$ alkyl and $C_3$-$C_8$ cycloalkyl;

m is selected from the group consisting of 0, 1, 2, 3, 4 and 5; and preferably, m is selected from the group consisting of 0, 1, 2 and 3;

n is selected from the group consisting of 0, 1, 2, 3, 4 and 5; and preferably, n is selected from the group consisting of 0, 1, 2, 3 and 4; and

q is selected from the group consisting of 0, 1, 2, 3 or 4; and preferably, q is 1 or 2.

**6.** The compound of formula I, and the stereoisomer or the pharmaceutically acceptable salt thereof according to any one of claims 1 and 4 to 5, having a structure of formula II,

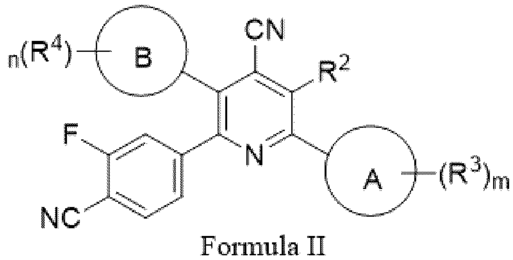

Formula II

wherein,

the ring A is selected from the group consisting of cyclohexyl, piperidyl, piperazinyl, azacycloheptyl and homopiperazinyl; and preferably, the piperidinyl, the piperazinyl, the azacycloheptyl or the homopiperazinyl is connected to a pyridine ring through an N atom;

the ring B is selected from the group consisting of phenyl, pyridyl, indolyl, dihydroindolyl, and indazolyl;

$R^2$ is selected from the group consisting of hydrogen and fluorine;

$R^3$ is selected from the group consisting of fluorine, chlorine, oxo, hydroxyl and amino;

$R^4$ is selected from the group consisting of fluorine, chlorine, oxo, methyl, methoxy,

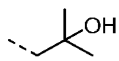

and

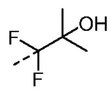

;

m is selected from the group consisting of 0, 1, 2 and 3; and

n is selected from the group consisting of 0, 1, 2, 3 and 4.

**7.** The compound of formula I, and the stereoisomer or the pharmaceutically acceptable salt thereof according to any one of claims 1 to 3, the compound of formula I having a structure of formula II, wherein

the ring A is selected from the group consisting of cyclohexyl, piperidyl, piperazinyl, azacyclobutyl, azacycloheptyl, homopiperazinyl, diazaspirononyl and diazaspriodecyl; and preferably, the piperidinyl, the piperazinyl, the azacyclobutyl, the azacycloheptyl, the homopiperazinyl, the diazaspirononyl or the diazaspriodecyl is connected to a pyridine ring through an N atom;

the ring B is selected from the group consisting of phenyl, pyridyl, indolyl, dihydroindolyl, indazolyl, benzoisoxazolyl, benzoxazolyl and benzodioxolyl;

$R^2$ is selected from the group consisting of hydrogen, cyano,

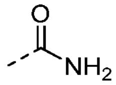 and 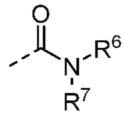 ,

and $R^6$ and $R^1$ are each independently selected from the group consisting of hydrogen, $C_1$-$C_5$ alkyl and $C_3$-$C_5$ cycloalkyl;
$R^3$ is selected from the group consisting of fluorine, chlorine, bromine, oxo, hydroxyl, amino and $C_1$-$C_6$ alkyl, wherein the $C_1$-$C_6$ alkyl is unsubstituted or is substituted with amino;
$R^4$ is selected from the group consisting of fluorine, chlorine, bromine, oxo, $C_1$-$C_6$ alkyl and $C_1$-$C_6$ alkoxy, wherein the $C_1$-$C_6$ alkyl is unsubstituted or is substituted with one or more substituents selected from the group consisting of halogen and hydroxyl;
m is selected from the group consisting of 0, 1, 2 and 3; and
n is selected from the group consisting of 0, 1, 2, 3 and 4.

**8.** The compound of formula I, and the stereoisomer or the pharmaceutically acceptable salt thereof according to any one of claims 1 to 3, wherein

the ring A is selected from the group consisting of piperidyl, tetrahydropyridyl, piperazinyl, azacyclobutyl, azacyclopentyl, azacyclohexyl, azacycloheptyl, homopiperazinyl, cyclohexyl, cyclobutyl, cyclopentyl, cycloheptyl, diazaspirononyl, diazaspirodecyl, oxa-azaspirononyl and oxa-azaspirodecyl;
the ring B is selected from the group consisting of phenyl, pyridyl, dihydropyridyl, pyrazinyl, pyridazinyl, pyrimidinyl, and benzo 5- to 6- membered heterocycloalkenyl containing 1-3 heteroatoms selected from the group consisting of a nitrogen atom and an oxygen atom;
$R^1$ is selected from the group consisting of hydrogen, cyano,

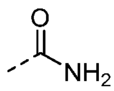 and 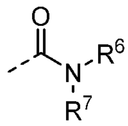 ,

$R^2$ is selected from the group consisting of hydrogen, cyano,

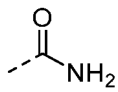 and 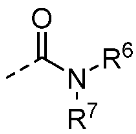 ,

or $R^1$ and $R^2$ together with carbon atom to which they are connected form a ring structure selected from

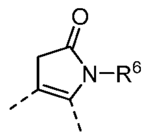

or

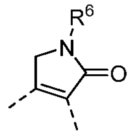

;

and $R^6$ and $R^1$ are each independently selected from the group consisting of hydrogen, $C_1$-$C_4$ alkyl and $C_3$-$C_4$ cycloalkyl;
m is 0, 1 or 2, and $R^3$ is selected from the group consisting of fluorine, chlorine, bromine, oxo, hydroxyl, amino and $C_1$-$C_3$ alkyl, wherein the $C_1$-$C_3$ alkyl is unsubstituted or is substituted with amino;

n is selected from the group consisting of 0, 1, 2, 3 and 4, and $R^4$ is selected from the group consisting of fluorine, chlorine, bromine, oxo, hydroxyl, $C_1$-$C_6$ alkyl and $C_1$-$C_6$ alkoxy, wherein the $C_1$-$C_6$ alkyl or the $C_1$-$C_6$ alkoxy is unsubstituted or is substituted with one or more substituents selected from the group consisting of halogen and hydroxyl; and

q is 1 or 2, and $R^5$ is selected from the group consisting of fluorine, chlorine and bromine.

**9.** The compound of formula I, and the stereoisomer or the pharmaceutically acceptable salt thereof according to claim 8, wherein
the ring B is selected from the group consisting of phenyl, pyridyl, dihydropyridyl, pyrazinyl, pyridazinyl, pyrimidinyl, indolyl, isoindolyl, isoindolinyl, dihydroindolyl, indazolyl, dihydroindazolyl, benzoxazolyl, benzoisoxazolyl, quinolyl, isoquinolyl, quinazolinyl, quinoxalinyl, benzodioxolyl, benzofuryl, benzimidazolyl, and benzodihydrooxazinyl.

**10.** The compound of formula I, and the stereoisomer or the pharmaceutically acceptable salt thereof according to claim 8 or 9, wherein

the ring A is selected from the group consisting of piperidyl, piperazinyl, azacyclobutyl, azacyclopentyl, homopiperazinyl, diazaspirononyl, diazaspirodecyl and oxa-azaspirodecyl;
the ring B is selected from the group consisting of phenyl, pyridyl, indolyl, dihydroindolyl, indazolyl, benzoisoxazolyl, benzoxazolyl, benzodioxolyl and benzodihydrooxazinyl;
$R^1$ and $R^2$ are each independently selected from the group consisting of hydrogen, cyano,

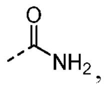,

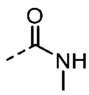, 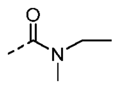, 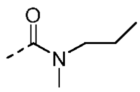, 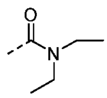, 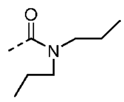, 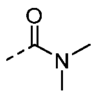, 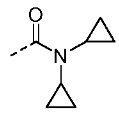 and 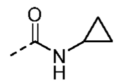;

or $R^1$ and $R^2$ together with carbon atom to which they are connected form a ring structure selected from

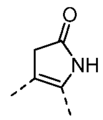

or

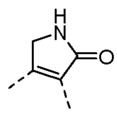;

$R^3$ is selected from the group consisting of fluorine, chlorine, bromine, oxo, hydroxyl, amino and $C_1$-$C_3$ alkyl, wherein the $C_1$-$C_3$ alkyl is unsubstituted or is substituted with amino;
$R^4$ is selected from the group consisting of fluorine, chlorine, bromine, oxo, hydroxyl, $C_1$-$C_6$ alkyl and $C_1$-$C_6$ alkoxy, wherein the $C_1$-$C_6$ alkyl is unsubstituted or is substituted with one or more substituents selected from the group consisting of halogen and hydroxyl;
$R^5$ is selected from the group consisting of fluorine, chlorine and bromine;
m is selected from the group consisting of 0, 1, 2 and 3;
n is selected from the group consisting of 0, 1, 2, 3 and 4; and
q is 1 or 2.

**11.** The compound of formula I, and the stereoisomer or the pharmaceutically acceptable salt thereof according to any one of claims 1 to 10, wherein the compound is selected from the group consisting of:

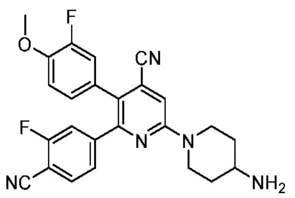 , 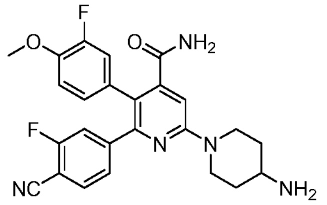 , 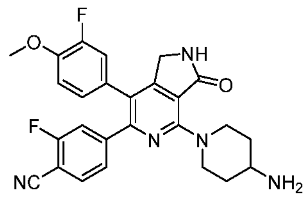 ,

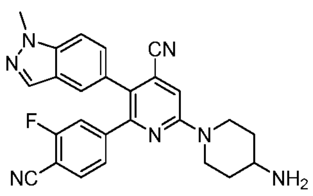 , 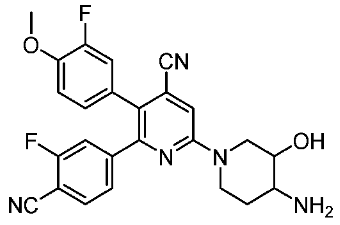 , 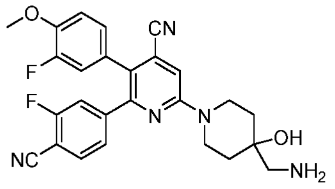 ,

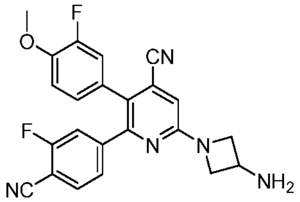 , 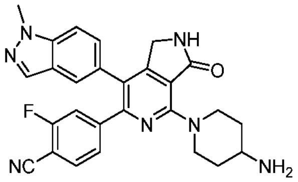 , 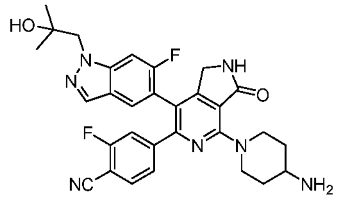 ,

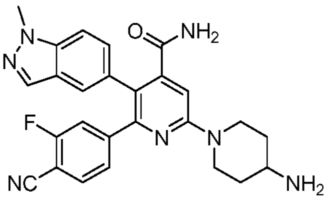 , 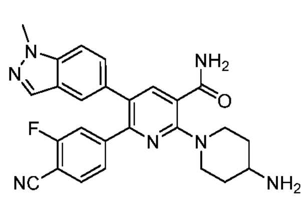 , 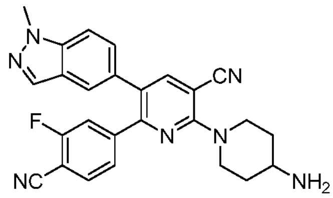 ,

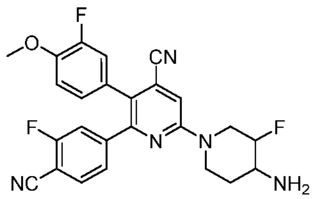 , 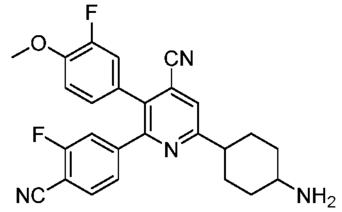 , 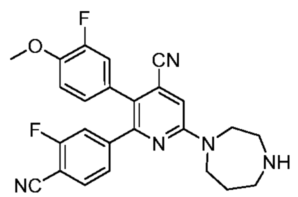 ,

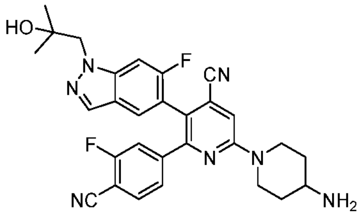 , 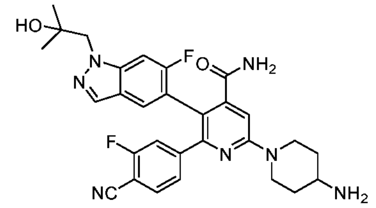 , 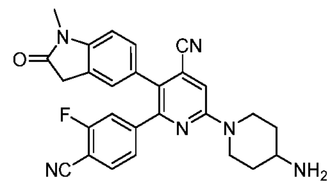 ,

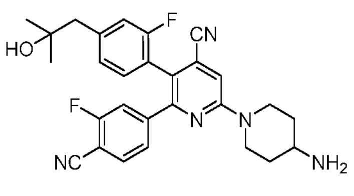 , 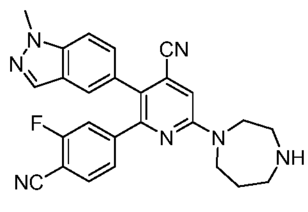 , 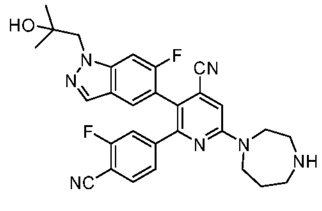 ,

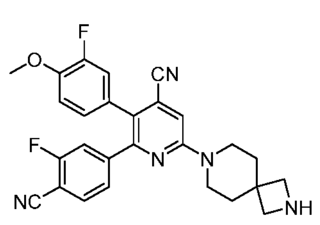,
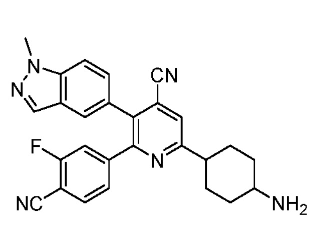,
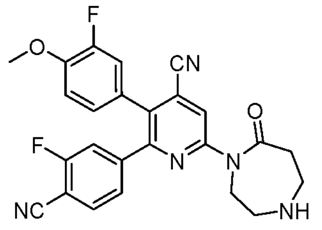,

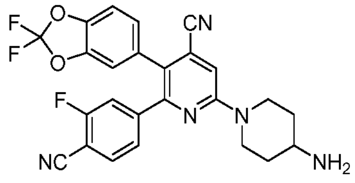,
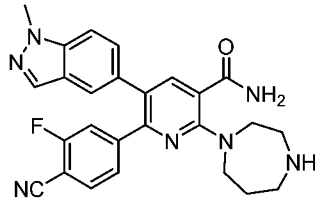,
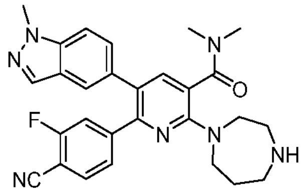,

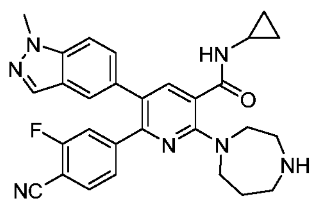,
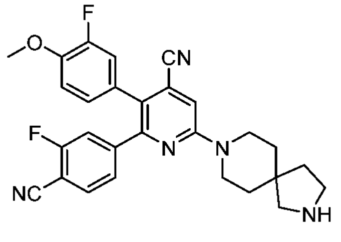,
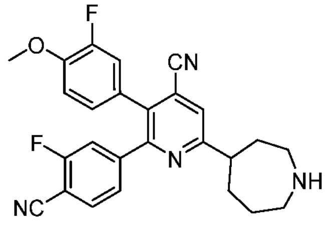,

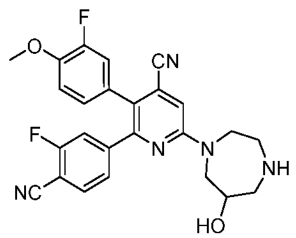,
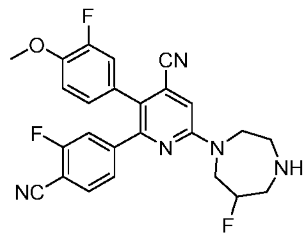,
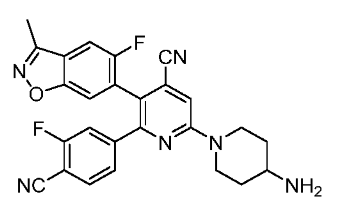,

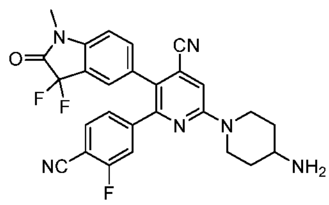,
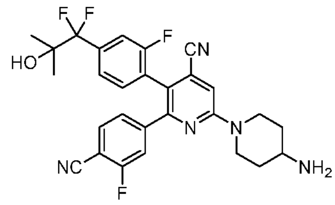,
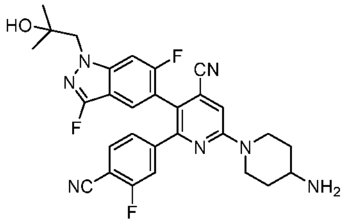,

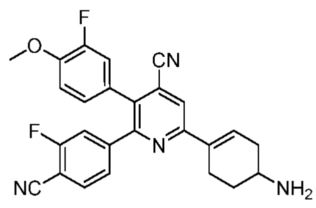,
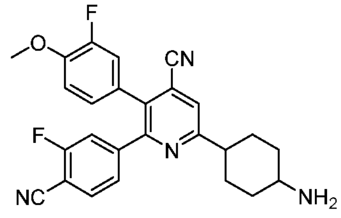,
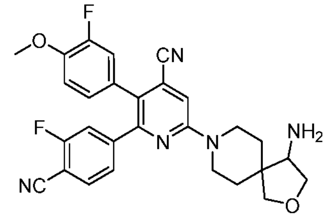,

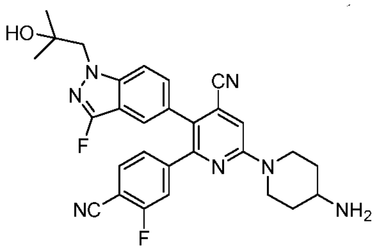,
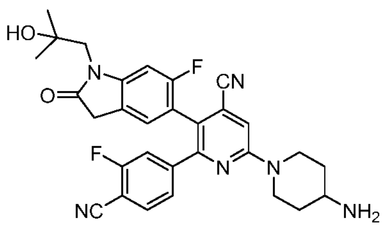,
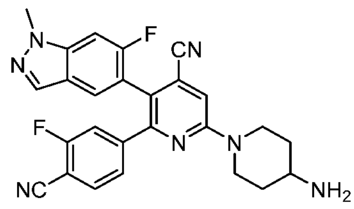,
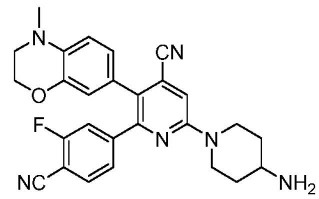,
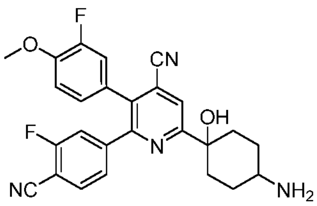,
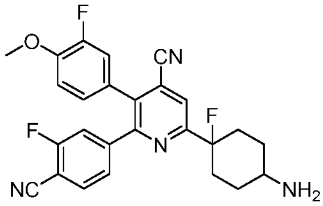,
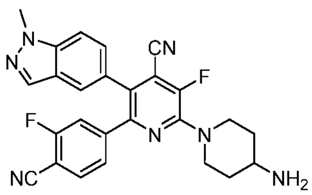,
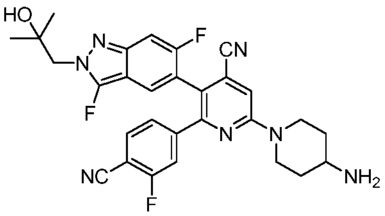,
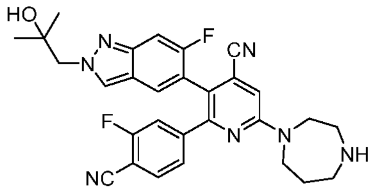
and
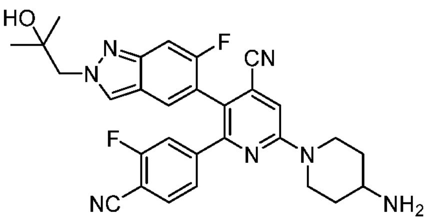

**12.** A pharmaceutical composition, comprising the compound of formula I, the stereoisomer or the pharmaceutically acceptable salt thereof according to any one of claims 1 to 11 and a pharmaceutically acceptable excipient.

**13.** The compound of formula I, the stereoisomer or the pharmaceutically acceptable salt thereof according to any one of claims 1 to 11 or the pharmaceutical composition according to claim 12 for use in a method for preventing or treating a lysine-specific demethylase 1-mediated disease, wherein preferably, the lysine-specific demethylase 1-mediated disease is a tumor or a cancer, and more preferably, the disease is selected from the group consisting of acute myeloid leukemia, chronic myeloid leukemia, acute lymphocytic leukemia, chronic lymphocytic leukemia, small cell lung cancer, non-small cell lung cancer, lymphoma, malignant sarcoma, breast cancer, cervical cancer, colon cancer, lung cancer, oral cancer, brain cancer, stomach cancer, liver cancer, colorectal cancer, pancreatic cancer, skin cancer, prostate cancer, bone cancer, kidney cancer, ovarian cancer, bladder cancer, fallopian tube tumor, peritoneal tumor, melanoma, glioma, neuroblastoma, mastoid malignant tumor, head and neck tumor and myeloma.

**14.** The compound of formula I, the stereoisomer or the pharmaceutically acceptable salt thereof according to any one of claims 1 to 11 or the pharmaceutical composition according to claim 12 for use as an lysine-specific demethylase 1 inhibitor.

**15.** A method for preparing the compound of formula I, the stereoisomer or the pharmaceutically acceptable salt thereof according to any one of claims 1 to 11, comprising:

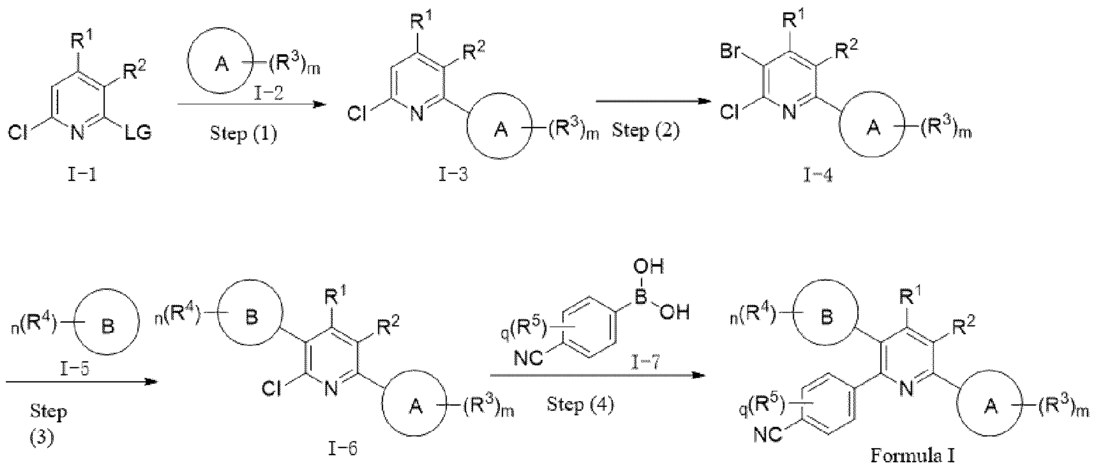


(1) reacting a compound I-1 with a compound I-2 to obtain a compound I-3;
(2) subjecting the compound I-3 to a substitution reaction to obtain a compound I-4;
(3) subjecting the compound I-4 to a coupling reaction with boric acid or borate of a compound I-5 to obtain a compound I-6; and
(4) subjecting the compound I-6 to a coupling reaction with a compound I-7 to obtain the compound of formula I;

or comprising:

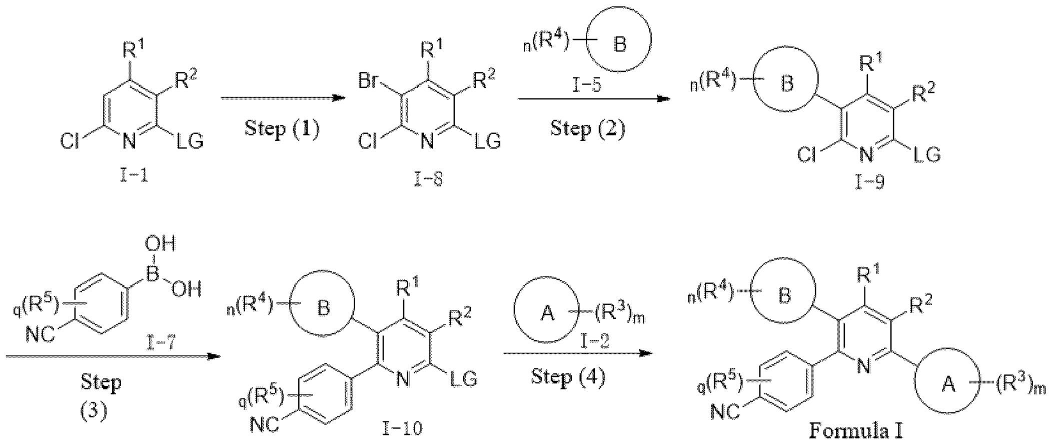


(1) subjecting a compound I-1 to a substitution reaction to obtain a compound I-8;
(2) subjecting the compound I-8 to a coupling reaction with boric acid or borate of a compound I-5 to obtain a compound I-9;
(3) subjecting the compound I-9 to a coupling reaction with a compound I-7 to obtain a compound I-10; and
(4) reacting the compound I-10 with a compound I-2 to obtain the compound of formula I;

or comprising:

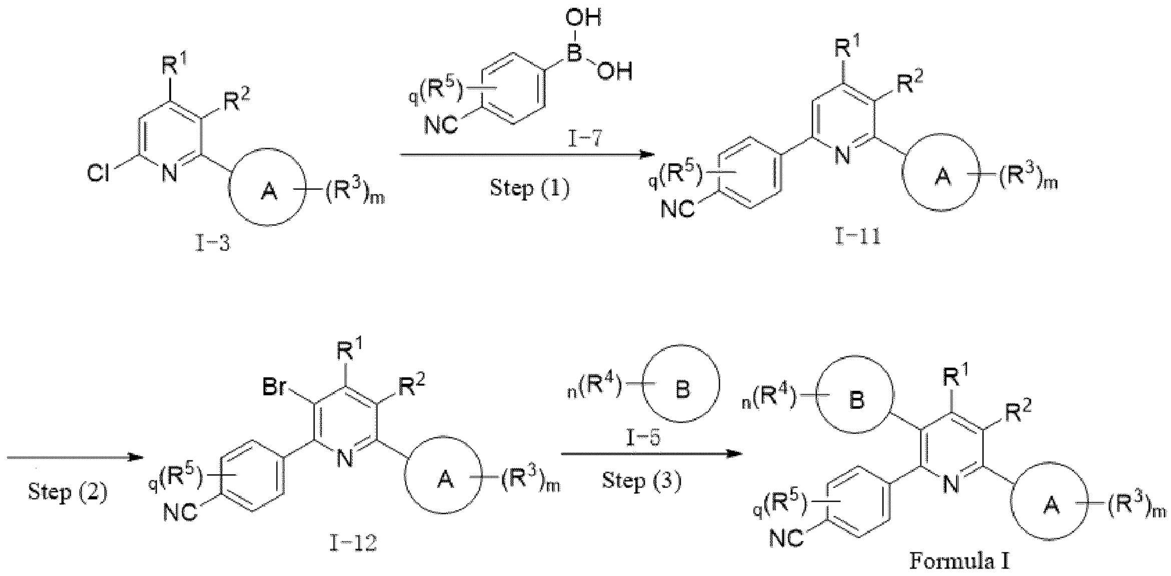


(1) subjecting a compound I-3 to a coupling reaction with a compound I-7 to obtain a compound I-11;
(2) subjecting the compound I-11 to a substitution reaction to obtain a compound I-12; and
(3) subjecting the compound I-12 to a coupling reaction with boric acid or borate of a compound I-5 to obtain the compound of formula I;

wherein LG represents a leaving group, and preferably, the leaving group is selected from the group consisting of a halogen atom, methylsulfonyloxy and p-tosyloxy.

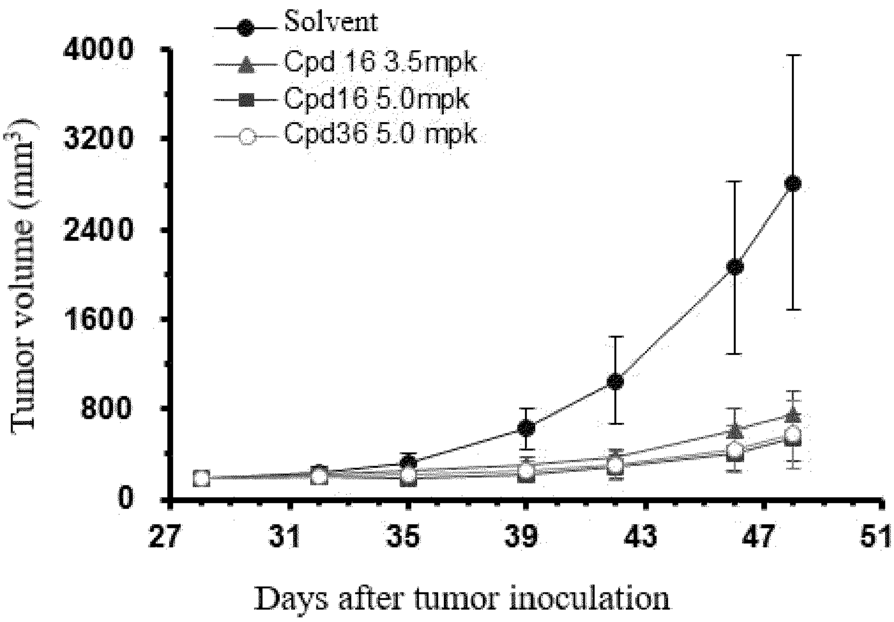
Solvent
Cpd 16 3.5mpk
Cpd16 5.0mpk
Cpd36 5.0 mpk
4000
3200
2400
1600
800
0
Tumor volume (mm3)
27
31
35
39
43
47
51
Days after tumor inoculation

FIG. 1

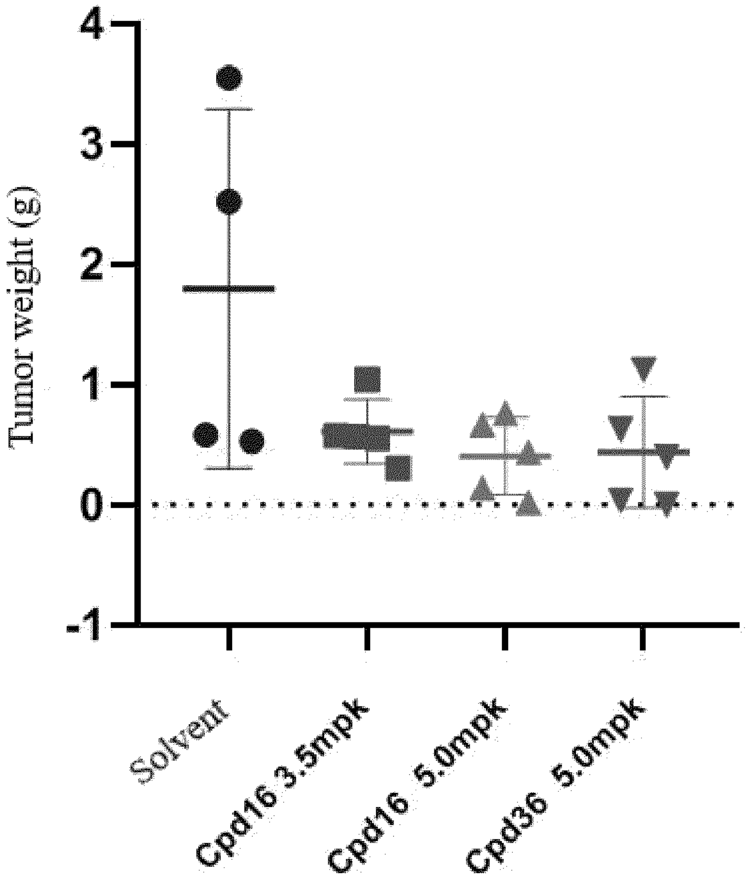
4
3
2
1
0
-1
Tumor weight (g)
Solvent
Cpd16 3.5mpk
Cpd16 5.0mpk
Cpd36 5.0mpk

FIG. 2

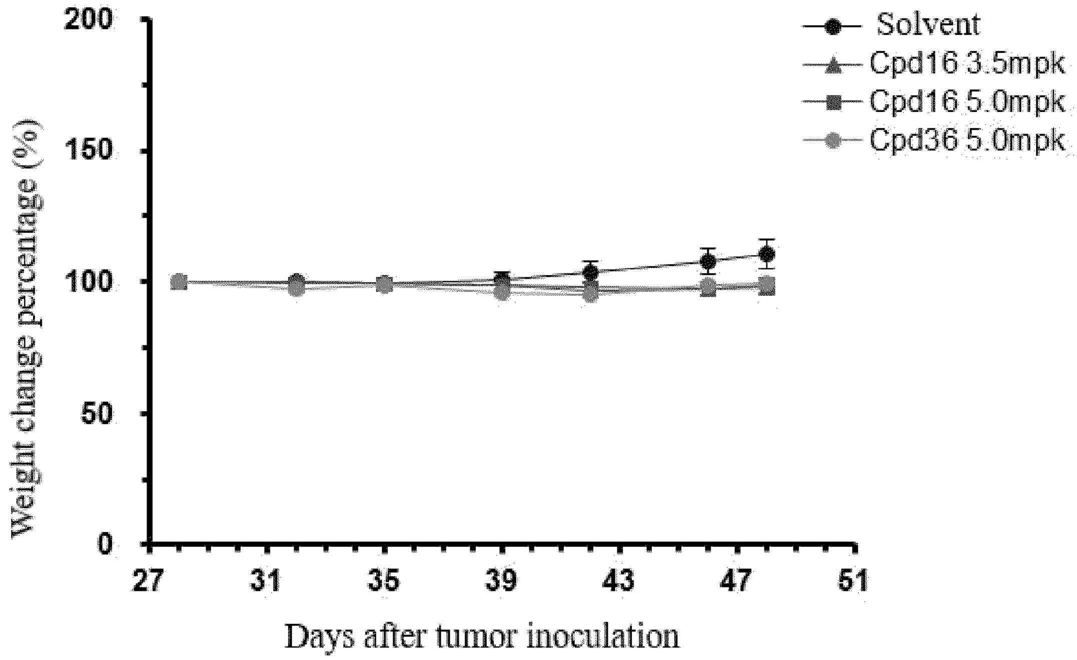
Solvent
Cpd16 3.5mpk
Cpd16 5.0mpk
Cpd36 5.0mpk
200
150
100
50
0
Weight change percentage (%)
27
31
35
39
43
47
51
Days after tumor inoculation

FIG. 3

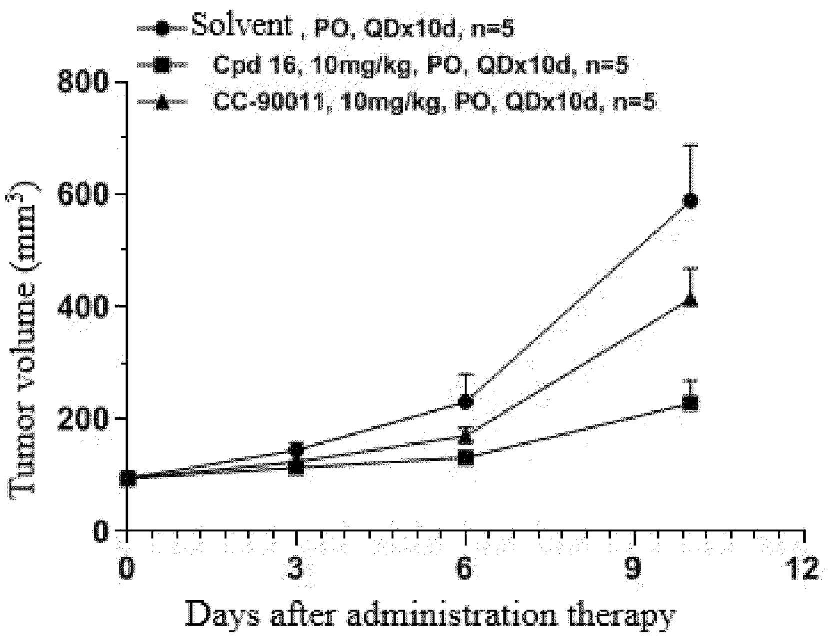
Solvent , PO, QDx10d, n=5
Cpd 16, 10mg/kg, PO, QDx10d, n=5
CC-90011, 10mg/kg, PO, QDx10d, n=5
800
600
400
200
0
Tumor volume (mm3)
0
3
6
9
12
Days after administration therapy

FIG. 4

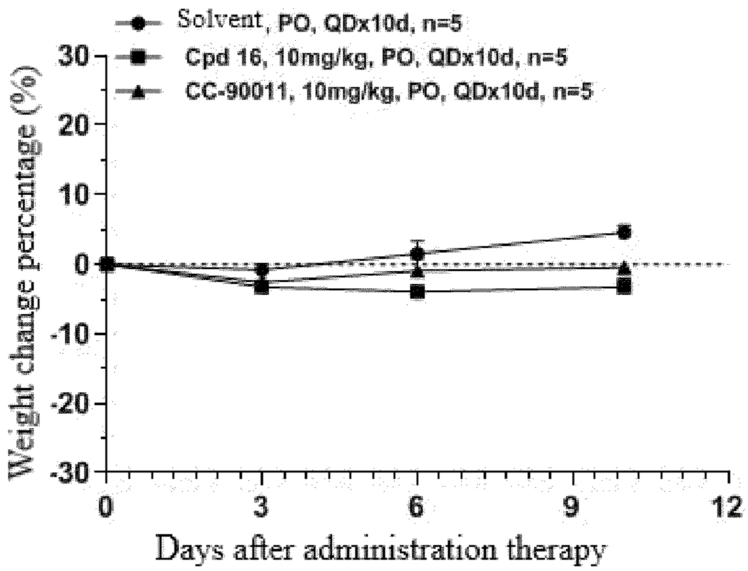
Solvent, PO, QDx10d, n=5
Cpd 16, 10mg/kg, PO, QDx10d, n=5
CC-90011, 10mg/kg, PO, QDx10d, n=5
Weight change percentage (%)
30
20
10
0
-10
-20
-30
0
3
6
9
12
Days after administration therapy

FIG. 5

**INTERNATIONAL SEARCH REPORT**

International application No. **PCT/CN2022/134330**

**A. CLASSIFICATION OF SUBJECT MATTER**

C07D 403/04(2006.01)i; C07D 403/14(2006.01)i; C07D 487/04(2006.01)i; C07D 413/14(2006.01)i; C07D 307/77(2006.01)i; A61K 31/343(2006.01)i; A61K 31/444(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, DWPI, SIPOABS, CNKI, Caplus(STN), Registry(STN): 成都苑东生物, 上海超阳药业, 吡啶, 氰基, 赖氨酸特异性去甲基酶, 苯并异噁唑, 苯并恶唑, 哌嗪, 哌啶, 吲唑, 吡咯, 癌症, 肿瘤, pyridine, cyano, lysine specific demethylase, benzoisoxazole, benzoxazole, piperazine, piperidine, indazole, pyrrole, cancer, tumor, LSD1

**C. DOCUMENTS CONSIDERED TO BE RELEVANT**

| Category* | Citation of document, with indication, where appropriate, of the relevant passages | Relevant to claim No. |
|---|---|---|
| PX | WO 2022199662 A1 (SICHUAN HUIYU PHARMACEUTICAL CO., LTD. et al.) 29 September 2022 (2022-09-29)<br>abstract; description, pages 140, 142, 147, and 149; and claim 93 | 1-15 |
| A | CN 102827073 A (AGIOS PHARMACEUTICALS, INC.) 19 December 2012 (2012-12-19)<br>abstract; and description, pages 13 and 33 | 1-15 |
| A | WO 03082191 A2 (MERCK & CO., INC. et al.) 09 October 2003 (2003-10-09)<br>abstract; and description, pages 155 and 157 | 1-15 |
| A | DOEBELIN Christelle et al. "Fully Regiocontrolled Polyarylation of Pyridine"<br>*The Journal of Organic Chemistry,* Vol. 79, No. 3, 13 January 2014 (2014-01-13),<br>pp. 908-918 | 15 |
| A | SARMA, Rituparna et al. "An approach towards quantitative structure-activity relationship studies for the affinity of diphenyl-pyridines towards CB1 receptor"<br>*Journal of Pharmaceutical Science and Technology,*<br>Vol. 3, No. 1, 31 December 2011 (2011-12-31),<br>pp. 477-493 | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 December 2022** | **18 January 2023** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/134330**

| Patent document cited in search report | Publication date (day/month/year) | Patent family member(s) | Publication date (day/month/year) |
|---|---|---|---|
| WO 2022199662 A1 | 29 September 2022 | None | |
| CN 102827073 A | 19 December 2012 | US 2014213580 A1 | 31 July 2014 |
| | | TW 201317215 A | 01 May 2013 |
| | | WO 2012171337 A1 | 20 December 2012 |
| | | AR 086977 A1 | 05 February 2014 |
| | | JP 2014519518 A | 14 August 2014 |
| | | EP 2721019 A1 | 23 April 2014 |
| | | CA 2839675 A1 | 20 December 2012 |
| | | US 2016229876 A1 | 11 August 2016 |
| | | ES 2694160 T3 | 18 December 2018 |
| | | AU 2012269648 A1 | 16 January 2014 |
| | | MX 321999 B | 14 July 2014 |
| | | MX 2013014922 A | 11 February 2014 |
| WO 03082191 A2 | 09 October 2003 | EP 1492784 A2 | 05 January 2005 |
| | | CA 2479744 A1 | 09 October 2003 |
| | | AU 2003225964 A1 | 13 October 2003 |
| | | US 2005182103 A1 | 18 August 2005 |
| | | JP 2005531520 A | 20 October 2005 |

Form PCT/ISA/210 (patent family annex) (January 2015)